(19) Europäisches Patentamt
European Patent Office
Office européen des brevets

(11) EP 1 402 888 A1

(12) EUROPEAN PATENT APPLICATION

(43) Date of publication:
31.03.2004 Bulletin 2004/14

(21) Application number: 02020987.0

(22) Date of filing: 18.09.2002

(51) Int Cl.[7]: **A61K 31/17**, A61K 31/155, A61K 31/18, A61K 31/135, A61K 31/165

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicant: **Jerini AG**
**10115 Berlin (DE)**

(72) Inventors:
- **Knolle, Jochen**
**10115 Berlin (DE)**
- **Schutkowski, Mike**
**06268 Ziegelroda (DE)**
- **Hummel, Gerd**
**13357 Berlin (DE)**

(74) Representative: **Bohmann, Armin K., Dr.**
**Bohmann & Loosen,**
**Anwaltssozietät,**
**Sonnenstrasse 8**
**80331 München (DE)**

(54) **The use of substituted carbocyclic compounds as rotamases inhibitors**

(57) The present invention is related to the use of a compound as an inhibitor to a rotamase, whereby the compound has the structure:

A-X-Y (I)

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising $-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $-[(CR^aR^b)_n-CO-NR^c-(CR^aR^b)_m]_t-$, $-[(CR^aR^b)_n-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m]_t-$, $-[(CR^aR^b)_n-O-(CR^aR^b)_m]_t-$, $-[(CR^aR^b)_n-SR^c-(CR^aR^b)_m]_t-$;
wherein $X_2$ is selected from the group comprising $-[(CR^aR^b)_n-CO-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-CO-NR^c-(CR^aR^b)_m]_t-$, $-[(CR^aR^b)_n-NR^c-CS-(CR^aR^b)_m]_t-$, $-[(CR^aR^b)_n-CS-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-SO-(CR^aR^b)_m]_t-$, $-[(CR^aR^b)_n-SO_2-(CR^aR^b)_m]_t-$, $-[(CR^aR^b)_n]_t-$;
wherein n and m are independently selected from each other and are any integer between 0 and 10 provided that if n is 0, m is different from 0, and if m is 0, n is different from 0;
wherein t is independently selected from n and/or m and is any integer between 0 and 10;
wherein
$R^a$, $R^b$, $R^c$, $R^d$ and $R^e$ are independently from each other selected from the group comprising H, alkyl, cycloalkyl, aryl, heteroaryl and heterocyclyl;
and wherein Y is selected from the group comprising alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide.

EP 1 402 888 A1

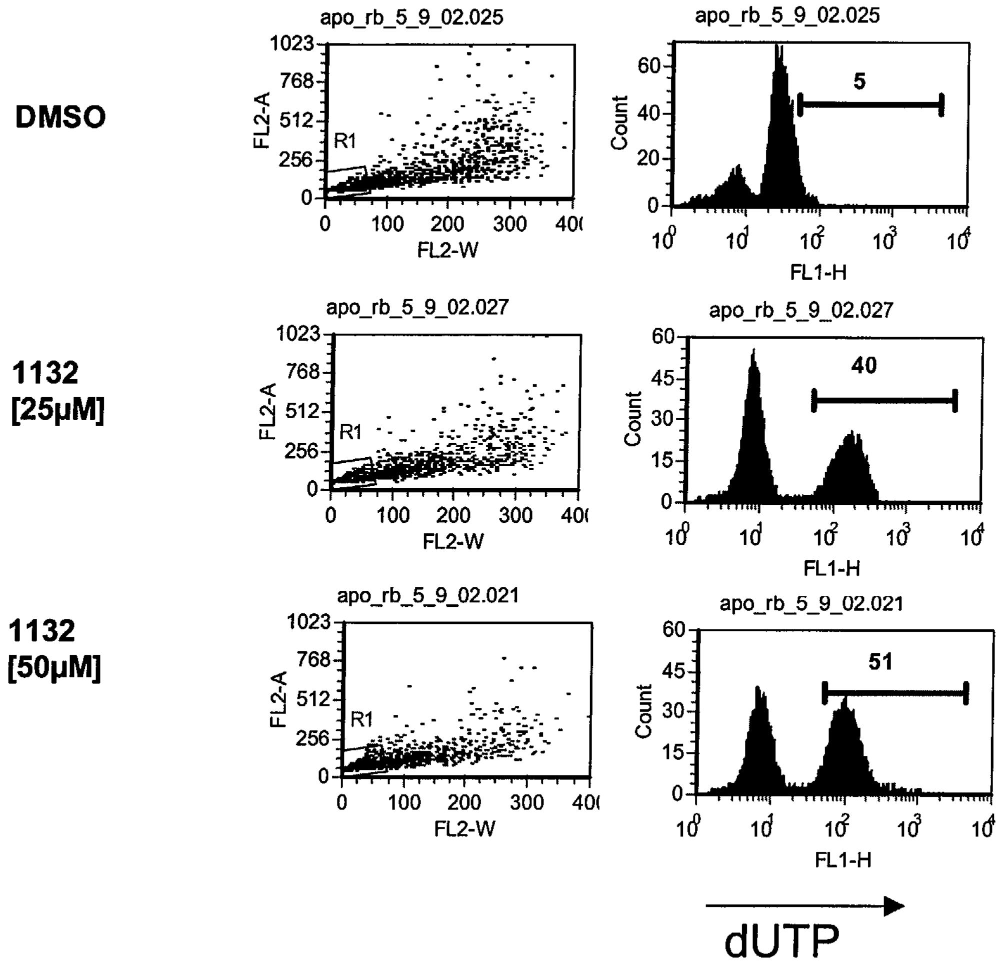
DMSO
1132
[25μM]
1132
[50μM]
apo_rb_5_9_02.025
apo_rb_5_9_02.027
apo_rb_5_9_02.021
FL2-A
FL2-W
R1
Count
FL1-H
5
40
51
dUTP

Fig. 1

## Description

The present invention is related to new compounds and the use of said compounds as an inhibitor to rotamases and for the manufacture of medicaments.

Rotamases, also referred to as peptidyl-prolyl cis-trans isomerases (PPIases) are a family of enzymes important in protein folding, assembly and transport. They act as catalysts to promote isomerization about the peptidyl-prolyl bond, which can have profound effects on protein function.

PPIases are divided into three classes, cyclophilins, FK-506 binding proteins (FKBPs) and the Pin1/parvulin class. While cyclophilins and FKBPs are distinguished by their ability to bind immunosuppressant molecules cyclosporin and FK-506, respectively, the Pin1/parvulin class binds neither of these immunosuppressants and is structurally unrelated to the other two classes. Known members of the Pin1/parvulin class include Pins 1 - 3 (Lu et al., Nature 380: 544-547, 1996), Pin-L (Campbell et al., Genomics 44:157-162, 1997), parvulin (Rahfeld et al., FEBS Letts 352:180-184, 1994), dodo (Maleszka et al., Proc Natl Acad Sci USA 93:447-451, 1996) and Ess1/Pft1 (Hanes et al., Yeast 5:55-72, 1989; and Hani et al., FEBS Letts 365:198-202, 1995).

Recent research suggests that members of the Pin1/parvulin class are essential modulators of the cell cycle, and mitosis in particular. Lu et al., Nature 380:544-547, 1996 reports that depletion of Pin1/Ess1 in yeast or human cells induces mitotic arrest followed by apoptosis, indicating that enzymes in this class serve an essential function in cell division and proliferation.

Accordingly, compounds inhibiting rotamases can serve as agents for the treatment of a variety of disorders which are characterized by an inappropriate cell proliferation including cancer and infectious diseases.

In the prior art a huge number of compounds are described which are active as inhibitors to rotamase. The respective compounds are, among others, peptide derivatives such as amino methylene-peptides which are described in European patent EP 0 610 743, or non-peptidic or non-peptidomimetic molecules.

Given the importance of rotamase there is an ongoing need in the art to provide further compounds which are suitable as inhibitors to rotamases and thus suitable to be used as a medicament for those diseases wherein a rotamase is involved in the pathological mechanism.

Accordingly, the problem underlying the present invention is to provide compounds which inhibit a rotamase. A further problem underlying the present invention is to provide new compounds for the treatment of diseases the pathophysiology of which involves an imbalanced or undesired activity of a rotamase.

In a first aspect the problem underlying the present invention is solved by a compound having the structure

A-X-Y **(I)**

wherein A is selected from the group comprising cycloalkyl, substituted cycloalkyl, heterocyclyl, substituted heterocyclyl, aryl, substituted aryl, heteroaryl and substituted heteroaryl;
X is a spacer and is selected from the group comprising
$-[(CR^aR^b)_n-CO-(CR^aR^b)_m]_t-$, $-[(CR^aR^b)_n-CS-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-NR^c-CS-(CR^aR^b)_m]_t-$, $-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $-[(CR^aR^b)_n-CO-NR^c-(CR^aR^b)_m]_t-$, $-](CR^aR^b)_n-CS-NR^c-(CR^aR^b)_m]_t-$, $-[(CR^aR^b)_n-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m]_t-$, $-[(CR^a-R^b)_n-O-(CR^aR^b)_m]_t-$, $-[(CR^aR^b)_n-SR^c-(CR^aR^b)_m]_t-$, $-[(CR^aR^b)_n-SO-(CR^aR^b)_m]_t-$, $-[(CR^aR^b)_n-SO_2-(CR^aR^b)_m]_t-$, $-[(CR^aR^b)_n]_t-$;
wherein:
$R^a$ is selected from the group comprising H, alkyl, cycloalkyl, aryl, heteroaryl and heterocyclyl;
$R^b$ is selected from the group comprising H, alkyl, cycloalkyl, aryl, heteroaryl and heterocyclyl;
$R^c$ is selected from the group comprising H, alkyl, cycloalkyl, aryl, heteroaryl and heterocyclyl;
$R^d$ is selected from the group comprising H, alkyl, cycloalkyl, aryl, heteroaryl and heterocyclyl;
$R^e$ is selected from the group comprising H, alkyl, cycloalkyl, aryl, heteroaryl and heterocyclyl;
n, m and t are independently selected from each other and are any integer between 0 and 10, i. e. 0, 1, 2, 3, 4 , 5, 6, 7, 8, 9 or 10;
Y is selected from the group comprising H, alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, substituted poly-unsaturated heterocyclyl, aryl, substituted aryl, heteroaryl, and substituted heteroaryl.

In a second aspect which is actually an embodiment of the first aspect of the invention, the problem is solved by a compound which has the structure

A-X-Y **(I)**

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising
$-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^a-R^b)_m]_t-$, $-[(CR^aR^b)_n-NR^c-C(N-R^e)-NR^d-(CR^aR^b)_m]_t-$, $-[(CR^aR^b)_n-CO-NR^c-(CR^aR^b)_m]_t-$, $-[(CR^aR^b)_n-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m]_t-$, $-[(CR^aR^b)_n-O-(CR^aR^b)_m]_t-$, $-[(CR^aR^b)_n-SR^c-(CR^aR^b)_m]_t-$;
$X_2$ is selected from the group comprising
$-[(CR^aR^b)_n-CO-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-CO-NR^c-(CR^aR^b)_m]_t-$, $-[(CR^aR^b)_n-NR^c-CS-(CR^aR^b)_m]_t-$, $-[(CR^aR^b)_n-CS-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-SO-(CR^aR^b)_m]_t-[(CR^aR^b)_nSO_2-(CR^aR^b)_m]_t-$, $-[(CR^aR^b)_n]_t-$;
wherein n and m are independently selected from each other and are any integer between 0 and 10 provided that if n is 0, m is different from 0, and if m is 0, n is different from 0;
wherein t is independently selected from n and/or m and is any integer between 0 and 10;
wherein
$R^a$, $R^6$, $R^c$, $R^d$ and $R^e$ are independently selected from the group comprising H, alkyl, cycloalkyl, aryl, heteroaryl and heterocyclyl;
and wherein Y is selected from the group comprising alkyl, substituted alkyl, cycloalkyl, substituted cycloalkyl, cycloalkenyl, substituted cycloalkenyl, heterocyclyl, substituted heterocyclyl, mono- or poly-unsaturated heterocyclyl or mono- or substituted poly-unsaturated heterocyclyl, aryl, substituted aryl, heteroaryl, and substituted heteroaryl.

As used herein, any integer between, e. g., 0 and 10 means 1, 2, 3, 4, 5, 6, 7, 8, 9 and 10.

In an embodiment of any aspect of the present invention Y is different from a peptide. As used herein peptide means a polymer of at least two amino acids which are linked by an amide bond. Any of the amino acids may be a natural or a non natural amino acid.

In a preferred embodiment of any aspect of the present invention m, n and t are independently selected from each other and are any integer between 0 and 5; more preferably if n is 0, m is different from 0 and if m is 0, n is different from 0.

Also as used herein the term compound(s) according to the present invention means any compound(s) according to any aspect of the present invention. If not indicated to the contrary, any embodiment of the present invention is an embodiment of any aspect of the present invention.

In a preferred embodiment of the inventive compound, A is

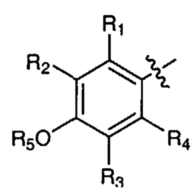

or

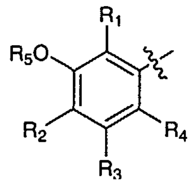

or

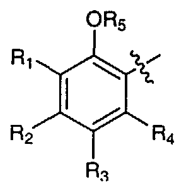

wherein $R_1$, $R_2$, $R_3$ and $R_4$ are each independently selected from the group comprising H, O, S, $NR^e$, halogen, alkyl, cycloalkyl, aryl, heterocyclyl and heteroaryl;
$R_5$ is selected from selected from the group comprising H, alkyl, cycloalkyl, aryl, heterocyclyl and heteroaryl; and
$R^e$ is selected from the group comprising H, alkyl, aryl, alkoxy, aryloxy, alkylamino and arylamino.

In an even more preferred embodiment of the inventive compound $R_1$, $R_2$, $R_3$, $R_4$ and/or $R_5$ have independently from each other one or more groups of the formula $R^f$; whereby $R^f$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, alkanoylamino, aroylamino, alkylthio, arylthio, ureido and amine.

In a further preferred embodiment
the alkylthio group is derivatized, preferably the sulfur atom of the alkylthio group is oxidized to a sulfoxide or sulfone;
the arylthio group is derivatized, preferably the sulfur atom of the arylthio group is oxidized to a sulfoxide or sulfone,
the ureido group is derivatized, preferably the nitrogen atom of the ureido group is independently mono- or di-substituted, more preferably the substitution is selected from the group comprising alkyl, aryl, heterocyclyl, heteroaryl, alkoxycarbonylamino, aryloxycarbonylamino, alkylcarbamoyloxy, arylcarbamoyloxy, alkylsulfonylamino, arylsulfonylamino, alkylaminosulfonyl, and arylaminosulfonyl; and/or
the amino group is derivatized, preferably the nitrogen atom is independently mono- or di-substituted by alkly, aryl, heterocyclyl, heteroalyl, halogen, hydroxy, oxo, carboxy, cyano, nitro, amidino and guanidino.

In a still more preferred embodiment $R^f$ is further substituted by one ore more groups $R^g$, whereby $R^g$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

Particularly preferred compounds according to the present invention are the compounds specified in the following table 1:

| | Structure | chemical name | Formula | MolWeight | MS data |
|---|---|---|---|---|---|
| 1 | 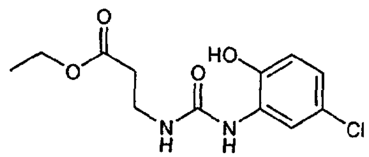 | 3-[3-(5-Chloro-2-hydroxy-phenyl)-ureido]-propionic acid ethyl ester | C12H15ClN2O4 | 286.71 | 286.9 |
| 2 | 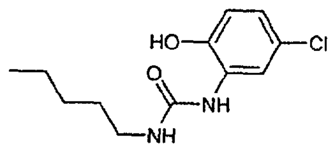 | 1-(5-Chloro-2-hydroxy-phenyl)-3-pentyl-urea | C12H17ClN2O2 | 256.73 | 256.9 |
| 3 | 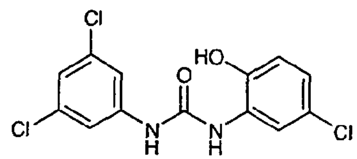 | 1-(5-Chloro-2-hydroxy-phenyl)-3-(3,5-dichloro-phenyl)-urea | C13H9Cl3N2O2 | 331.59 | 331.9 |
| 4 | 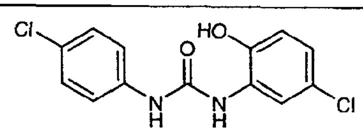 | 1-(5-Chloro-2-hydroxy-phenyl)-3-(4-chloro-phenyl)-urea | C13H10Cl2N2O2 | 297.14 | 297.7 |
| 5 | 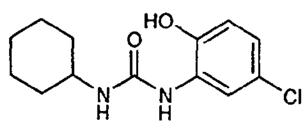 | 1-(5-Chloro-2-hydroxy-phenyl)-3-cyclohexyl-urea | C13H17ClN2O2 | 268.74 | 269.1 |
| 6 | 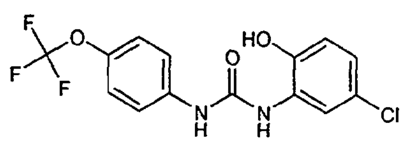 | 1-(5-Chloro-2-hydroxy-phenyl)-3-(4-trifluoromethoxy-phenyl)-urea | C14H10ClF3N2O3 | 346.69 | 346.9 |

| | | | | | |
|---|---|---|---|---|---|
| 7 | 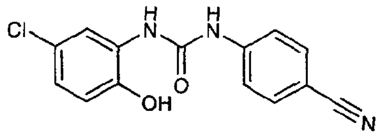 | 1-(5-Chloro-2-hydroxy-phenyl)-3-(4-cyano-phenyl)-urea | C14H10ClN3O2 | 287.70 | 287.9 |
| 8 | 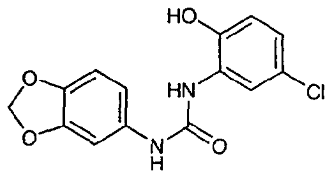 | 1-Benzo[1,3]dioxol-5-yl-3-(5-chloro-2-hydroxy-phenyl)-urea | C14H11ClN2O4 | 306.70 | 306.9 |
| 9 | 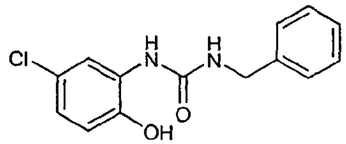 | 1-Benzyl-3-(5-chloro-2-hydroxy-phenyl)-urea | C14H13ClN2O2 | 276.72 | 276.9 |
| 10 | 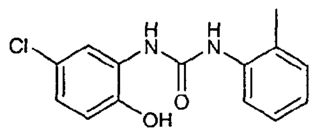 | 1-(5-Chloro-2-hydroxy-phenyl)-3-o-tolyl-urea | C14H13ClN2O2 | 276.72 | 276.9 |
| 11 | 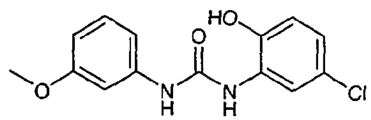 | 1-(5-Chloro-2-hydroxy-phenyl)-3-(3-methoxy-phenyl)-urea | C14H13ClN2O3 | 292.72 | 292.9 |
| 12 | 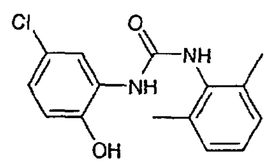 | 1-(5-Chloro-2-hydroxy-phenyl)-3-(2,6-dimethyl-phenyl)-urea | C15H15ClN2O2 | 290.75 | 290.9 |
| 13 | 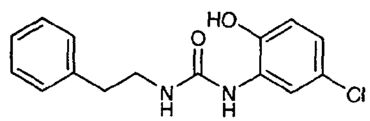 | 1-(5-Chloro-2-hydroxy-phenyl)-3-phenethyl-urea | C15H15ClN2O2 | 290.75 | 291.2 |

| | | | | | |
|---|---|---|---|---|---|
| 14 | 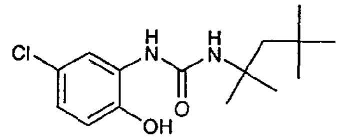 | 1-(5-Chloro-2-hydroxy-phenyl)-3-(1,1,3,3-tetramethyl-butyl)-urea | C15H23ClN2O2 | 298.81 | 299.4 |
| 15 | 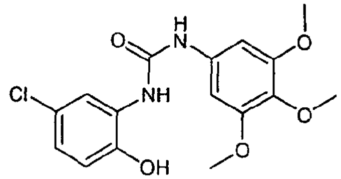 | 1-(5-Chloro-2-hydroxy-phenyl)-3-(3,4,5-trimethoxy-phenyl)-urea | C16H17ClN2O5 | 352.77 | 353.2 |
| 16 | 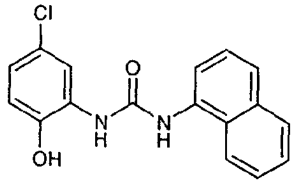 | 1-(5-Chloro-2-hydroxy-phenyl)-3-naphthalen-1-yl-urea | C17H13ClN2O2 | 312.75 | 313.1 |
| 17 | 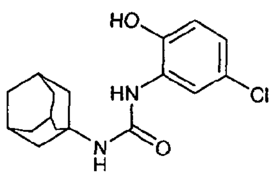 | 1-Adamantan-1-yl-3-(5-chloro-2-hydroxy-phenyl)-urea | C17H21ClN2O2 | 320.82 | 321.4 |
| 18 | 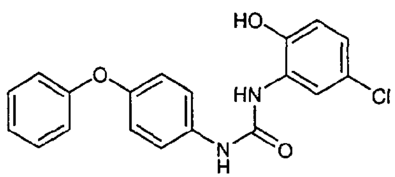 | 1-(5-Chloro-2-hydroxy-phenyl)-3-(4-phenoxy-phenyl)-urea | C19H15ClN2O3 | 354.79 | 354.9 |
| 19 | 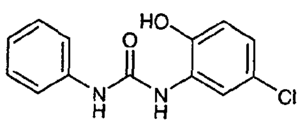 | 1-(5-Chloro-2-hydroxy-phenyl)-3-phenyl-urea | C13H11ClN2O2 | 262.70 | 263.2 |

| | | | | | |
|---|---|---|---|---|---|
| 20 | 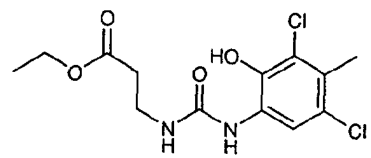 | 3-[3-(3,5-Dichloro-2-hydroxy-4-methyl-phenyl)-ureido]-propionic acid ethyl ester | C13H16Cl2N2O4 | 335.19 | 335.9 |
| 21 | 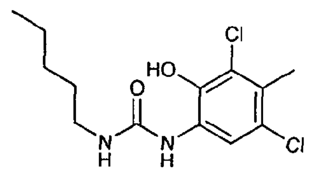 | 1-(3,5-Dichloro-2-hydroxy-4-methyl-phenyl)-3-pentyl-urea | C13H18Cl2N2O2 | 305.20 | 305.9 |
| 22 | 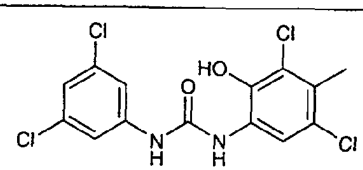 | 1-(3,5-Dichloro-2-hydroxy-4-methyl-phenyl)-3-(3,5-dichloro-phenyl)-urea | C14H10Cl4N2O2 | 380.06 | 380.2 |
| 23 | 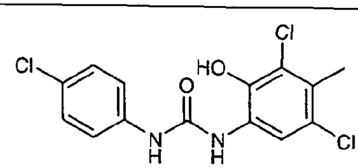 | 1-(4-Chloro-phenyl)-3-(3,5-dichloro-2-hydroxy-4-methyl-phenyl)-urea | C14H11Cl3N2O2 | 345.61 | 345.9 |
| 24 | 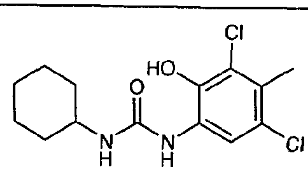 | 1-Cyclohexyl-3-(3,5-dichloro-2-hydroxy-4-methyl-phenyl)-urea | C14H18Cl2N2O2 | 317.21 | 317.8 |
| 25 | 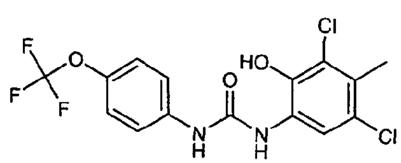 | 1-(3,5-Dichloro-2-hydroxy-4-methyl-phenyl)-3-(4-trifluoromethoxy-phenyl)-urea | C15H11Cl2F3N2O3 | 395.16 | 395.4 |

| | | | | | |
|---|---|---|---|---|---|
| 26 | 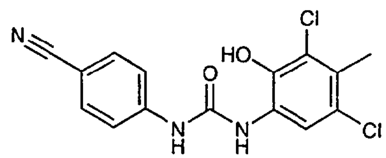 | 1-(4-Cyano-phenyl)-3-(3,5-dichloro-2-hydroxy-4-methyl-phenyl)-urea | C15H11Cl2N3O2 | 336.18 | 336.4 |
| 27 | 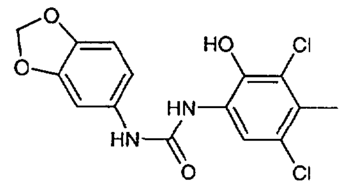 | 1-Benzo[1,3]dioxol-5-yl-3-(3,5-dichloro-2-hydroxy-4-methyl-phenyl)-urea | C15H12Cl2N2O4 | 355.18 | 355.2 |
| 28 | 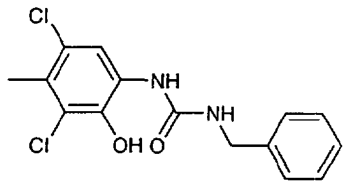 | 1-Benzyl-3-(3,5-dichloro-2-hydroxy-4-methyl-phenyl)-urea | C15H14Cl2N2O2 | 325.19 | 325.5 |
| 29 | 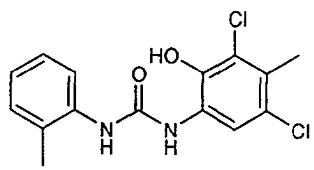 | 1-(3,5-Dichloro-2-hydroxy-4-methyl-phenyl)-3-o-tolyl-urea | C15H14Cl2N2O2 | 325.19 | 325.5 |
| 30 | 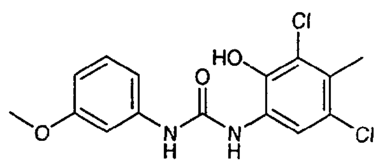 | 1-(3,5-Dichloro-2-hydroxy-4-methyl-phenyl)-3-(3-methoxy-phenyl)-urea | C15H14Cl2N2O3 | 341.19 | 341.4 |
| 31 | 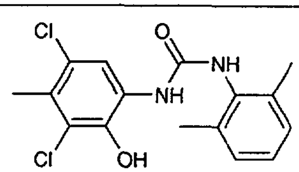 | 1-(3,5-Dichloro-2-hydroxy-4-methyl-phenyl)-3-(2,6-dimethyl-phenyl)-urea | C16H16Cl2N2O2 | 339.22 | 339.4 |

| | | | | | |
|---|---|---|---|---|---|
| 32 | 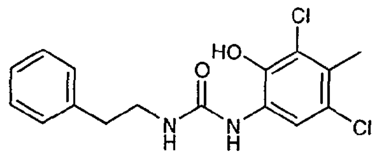 | 1-(3,5-Dichloro-2-hydroxy-4-methyl-phenyl)-3-phenethyl-urea | C16H16Cl2N2O2 | 339.22 | 339.4 |
| 33 | 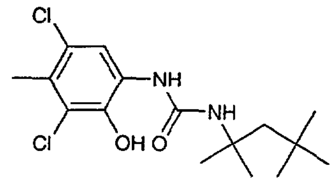 | 1-(3,5-Dichloro-2-hydroxy-4-methyl-phenyl)-3-(1,1,3,3-tetramethyl-butyl)-urea | C16H24Cl2N2O2 | 347.28 | 347.4 |
| 34 | 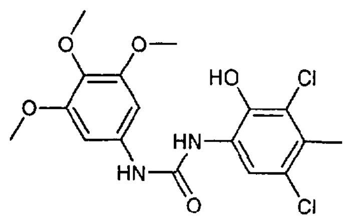 | 1-(3,5-Dichloro-2-hydroxy-4-methyl-phenyl)-3-(3,4,5-trimethoxy-phenyl)-urea | C17H18Cl2N2O5 | 401.25 | 401.3 |
| 35 | 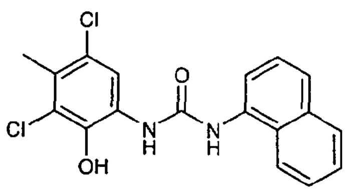 | 1-(3,5-Dichloro-2-hydroxy-4-methyl-phenyl)-3-naphthalen-1-yl-urea | C18H14Cl2N2O2 | 361.23 | 361.3 |
| 36 | 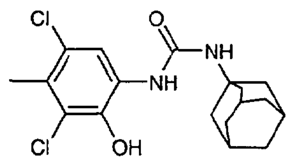 | 1-Adamantan-1-yl-3-(3,5-dichloro-2-hydroxy-4-methyl-phenyl)-urea | C18H22Cl2N2O2 | 369.29 | 369.3 |

| | | | | | |
|---|---|---|---|---|---|
| 37 | 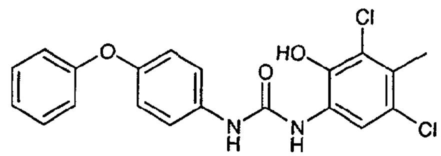 | 1-(3,5-Dichloro-2-hydroxy-4-methyl-phenyl)-3-(4-phenoxy-phenyl)-urea | C20H16Cl2N2O3 | 403.26 | 403.3 |
| 38 | 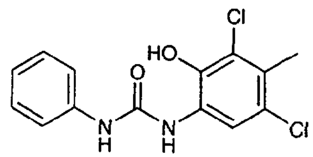 | 1-(3,5-Dichloro-2-hydroxy-4-methyl-phenyl)-3-phenyl-urea | C14H12Cl2N2O2 | 311.17 | 311.3 |
| 39 | 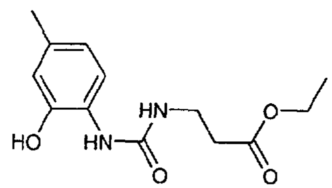 | 3-[3-(2-Hydroxy-4-methyl-phenyl)-ureido]-propionic acid ethyl ester | C13H18N2O4 | 266.30 | 266.5 |
| 40 | 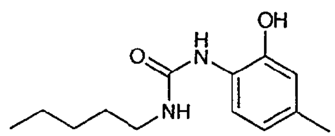 | 1-(2-Hydroxy-4-methyl-phenyl)-3-pentyl-urea | C13H20N2O2 | 236.31 | 236.5 |
| 41 | 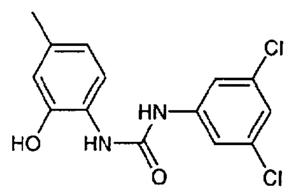 | 1-(3,5-Dichloro-phenyl)-3-(2-hydroxy-4-methyl-phenyl)-urea | C14H12Cl2N2O2 | 311.17 | 311.5 |
| 42 | 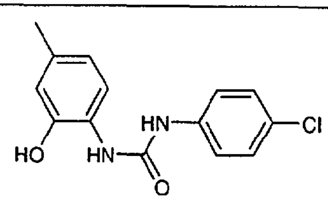 | 1-(4-Chloro-phenyl)-3-(2-hydroxy-4-methyl-phenyl)-urea | C14H13ClN2O2 | 276.72 | 276.8 |

| | | | | | |
|---|---|---|---|---|---|
| 43 | 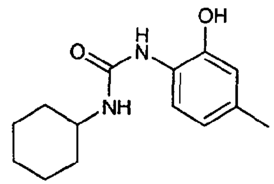 | 1-Cyclohexyl-3-(2-hydroxy-4-methyl-phenyl)-urea | C14H20N2O2 | 248.32 | 248.5 |
| 44 | 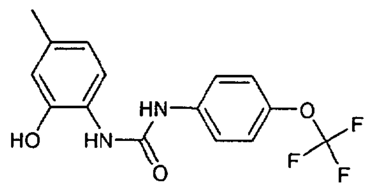 | 1-(2-Hydroxy-4-methyl-phenyl)-3-(4-trifluoromethoxy-phenyl)-urea | C15H13F3N2O3 | 326.27 | 326.6 |
| 45 | 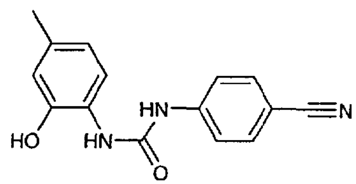 | 1-(4-Cyano-phenyl)-3-(2-hydroxy-4-methyl-phenyl)-urea | C15H13N3O2 | 267.29 | 267.7 |
| 46 | 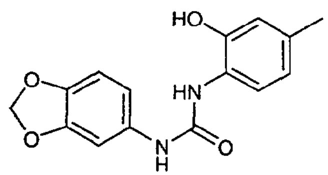 | 1-Benzo[1,3]dioxol-5-yl-3-(2-hydroxy-4-methyl-phenyl)-urea | C15H14N2O4 | 286.29 | 286.7 |
| 47 | 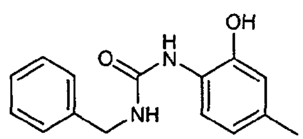 | 1-Benzyl-3-(2-hydroxy-4-methyl-phenyl)-urea | C15H16N2O2 | 256.30 | 256.7 |

| | | | | | |
|---|---|---|---|---|---|
| 48 | 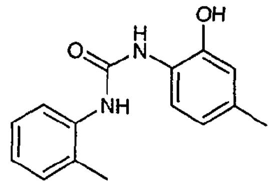 | 1-(2-Hydroxy-4-methyl-phenyl)-3-o-tolyl-urea | C15H16N2O2 | 256.30 | 256.3 |
| 49 | 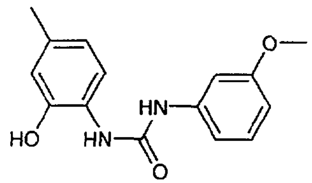 | 1-(2-Hydroxy-4-methyl-phenyl)-3-(3-methoxy-phenyl)-urea | C15H16N2O3 | 272.30 | 272.3 |
| 50 | 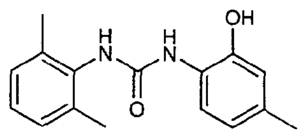 | 1-(2,6-Dimethyl-phenyl)-3-(2-hydroxy-4-methyl-phenyl)-urea | C16H18N2O2 | 270.33 | 270.7 |
| 51 | 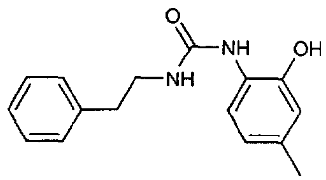 | 1-(2-Hydroxy-4-methyl-phenyl)-3-phenethyl-urea | C16H18N2O2 | 270.33 | 270.7 |
| 52 | 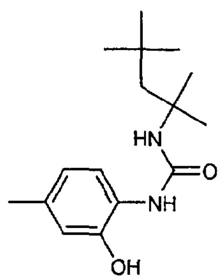 | 1-(2-Hydroxy-4-methyl-phenyl)-3-(1,1,3,3-tetramethyl-butyl)-urea | C16H26N2O2 | 278.39 | 278.5 |

| | | | | | |
|---|---|---|---|---|---|
| 53 | 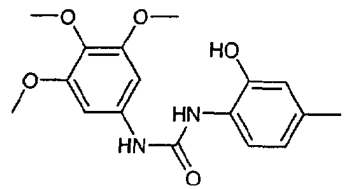 | 1-(2-Hydroxy-4-methyl-phenyl)-3-(3,4,5-trimethoxy-phenyl)-urea | C17H20N2O5 | 332.36 | 332.5 |
| 54 | 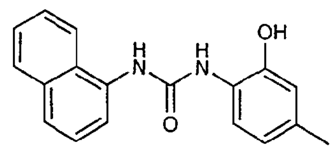 | 1-(2-Hydroxy-4-methyl-phenyl)-3-naphthalen-1-yl-urea | C18H16N2O2 | 292.34 | 292.5 |
| 55 | 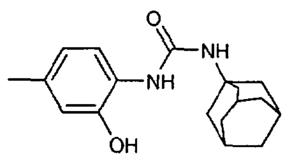 | 1-Adamantan-1-yl-3-(2-hydroxy-4-methyl-phenyl)-urea | C18H24N2O2 | 300.40 | 300.6 |
| 56 | 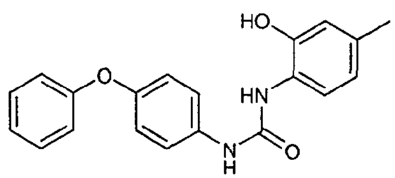 | 1-(2-Hydroxy-4-methyl-phenyl)-3-(4-phenoxy-phenyl)-urea | C20H18N2O3 | 334.37 | 334.6 |
| 57 | 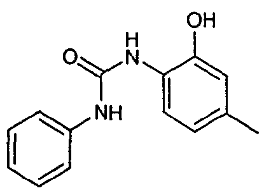 | 1-(2-Hydroxy-4-methyl-phenyl)-3-phenyl-urea | C14H14N2O2 | 242.28 | 242.3 |

| | | | | | |
|---|---|---|---|---|---|
| 58 | 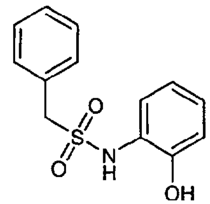 | N-(2-Hydroxy-phenyl)-C-phenyl-methanesulfonamide | C13H13NO3S | 263.31 | 264.0 |
| 59 | 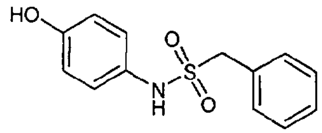 | N-(4-Hydroxy-phenyl)-C-phenyl-methanesulfonamide | C13H13NO3S | 263.31 | 264.0 |
| 60 | 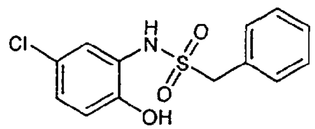 | N-(5-Chloro-2-hydroxy-phenyl)-C-phenyl-methanesulfonamide | C13H12ClNO3S | 297.76 | 298.1 |
| 61 | 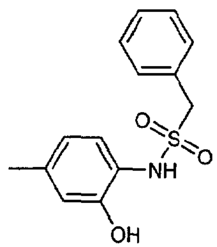 | N-(2-Hydroxy-4-methyl-phenyl)-C-phenyl-methanesulfonamide | C14H15NO3S | 277.34 | 278.1 |
| 62 | 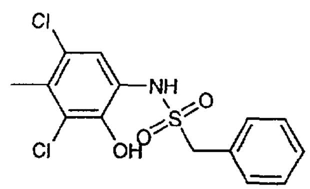 | N-(3,5-Dichloro-2-hydroxy-4-methyl-phenyl)-C-phenyl-methanesulfonamide | C14H13Cl2NO3S | 346.23 | 347.0 |

| | | | | | |
|---|---|---|---|---|---|
| 63 | 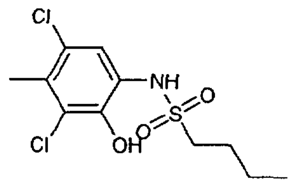 | Butane-1-sulfonic acid (3,5-dichloro-2-hydroxy-4-methyl-phenyl)-amide | C11H15Cl2NO3S | 312.21 | 312.1 |
| 64 | 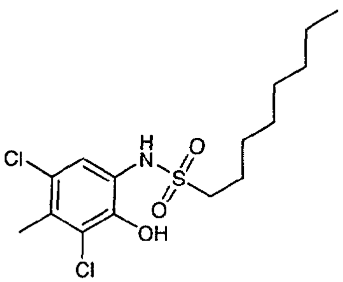 | Octane-1-sulfonic acid (3,5-dichloro-2-hydroxy-4-methyl-phenyl)-amide | C15H23Cl2NO3S | 368.32 | 368.3 |
| 65 | 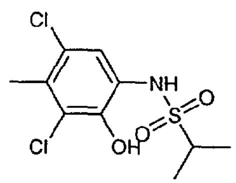 | Propane-2-sulfonic acid (3,5-dichloro-2-hydroxy-4-methyl-phenyl)-amide | C10H13Cl2NO3S | 298.18 | 298.1 |
| 66 | 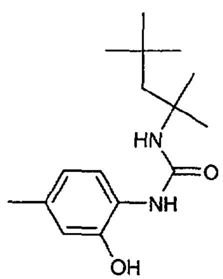 | 1-(2-Hydroxy-4-methyl-phenyl)-3-(1,1,3,3-tetramethyl-butyl)-urea | C16H26N2O2 | 278.39 | 278.5 |

| | | | | | |
|---|---|---|---|---|---|
| 67 | 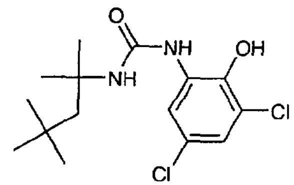 | 1-(3,5-Dichloro-2-hydroxy-phenyl)-3-(1,1,3,3-tetramethyl-butyl)-urea | C15H22Cl2N2O2 | 333.26 | 333.3 |
| 68 | 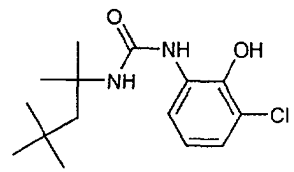 | 1-(3-Chloro-2-hydroxy-phenyl)-3-(1,1,3,3-tetramethyl-butyl)-urea | C15H23ClN2O2 | 298.81 | 298.9 |
| 69 | 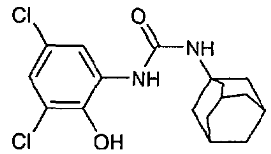 | 1-Adamantan-1-yl-3-(3,5-dichloro-2-hydroxy-phenyl)-urea | C17H20Cl2N2O2 | 355.26 | 355.4 |
| 70 | 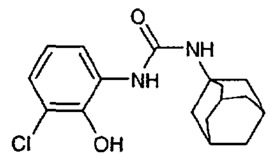 | 1-Adamantan-1-yl-3-(3-chloro-2-hydroxy-phenyl)-urea | C17H21ClN2O2 | 320.82 | 321.0 |
| 71 | 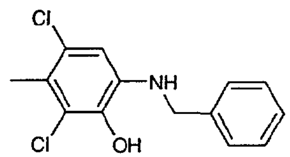 | 6-Benzylamino-2,4-dichloro-3-methyl-phenol | C14H13Cl2NO | 282.17 | 282.2 |
| 72 | 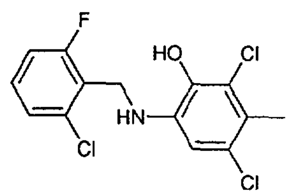 | 2,4-Dichloro-6-(2-chloro-6-fluoro-benzylamino)-3-methyl-phenol | C14H11Cl3FNO | 334.60 | 334.8 |

| | | | | | |
|---|---|---|---|---|---|
| 73 | 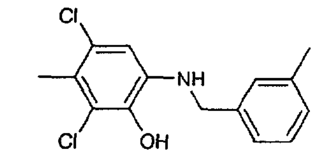 | 2,4-Dichloro-3-methyl-6-(3-methyl-benzylamino)-phenol | C15H15Cl2NO | 296.20 | 296.3 |
| 74 | 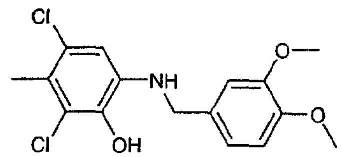 | 2,4-Dichloro-6-(3,4-dimethoxy-benzylamino)-3-methyl-phenol | C16H17Cl2NO3 | 342.22 | 342.2 |
| 75 | 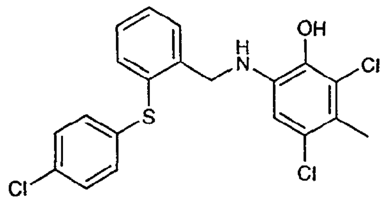 | 2,4-Dichloro-6-[2-(4-chloro-phenylsulfanyl)-benzylamino]-3-methyl-phenol | C20H16Cl3NOS | 424.77 | 424.8 |
| 76 | 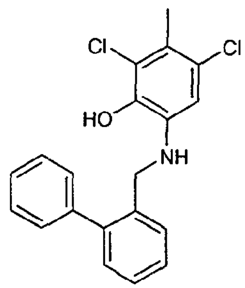 | 6-[(Biphenyl-2-ylmethyl)-amino]-2,4-dichloro-3-methyl-phenol | C20H17Cl2NO | 358.27 | 358.3 |
| 77 | 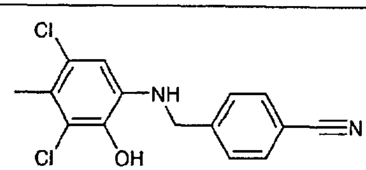 | 4-[(3,5-Dichloro-2-hydroxy-4-methyl-phenylamino)-methyl]-benzonitrile | C15H12Cl2N2O | 307.18 | 307.2 |

| | | | | | |
|---|---|---|---|---|---|
| 78 | 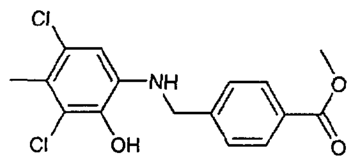 | 4-[(3,5-Dichloro-2-hydroxy-4-methyl-phenylamino)-methyl]-benzoic acid methyl ester | C16H15Cl2NO3 | 340.21 | 340.4 |
| 79 | 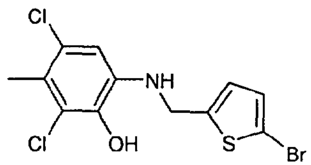 | 6-[(5-Bromo-thiophen-2-ylmethyl)-amino]-2,4-dichloro-3-methyl-phenol | C12H10BrCl2NOS | 367.09 | 367.2 |
| 80 | 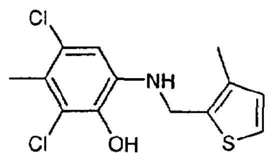 | 2,4-Dichloro-3-methyl-6-[(3-methyl-thiophen-2-ylmethyl)-amino]-phenol | C13H13Cl2NOS | 302.22 | 302.2 |
| 81 | 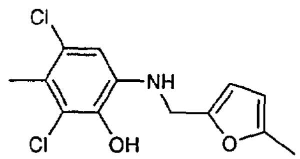 | 2,4-Dichloro-3-methyl-6-[(5-methyl-furan-2-ylmethyl)-amino]-phenol | C13H13Cl2NO2 | 286.16 | 286.2 |
| 82 | 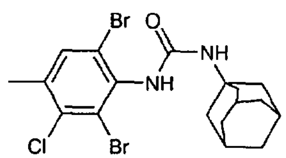 | 1-Adamantan-1-yl-3-(2,6-dibromo-3-chloro-4-methyl-phenyl)-urea | C18H21Br2ClN2O | 476.64 | 476.8 |
| 83 | 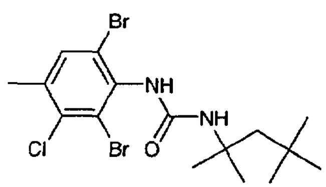 | 1-(2,6-Dibromo-3-chloro-4-methyl-phenyl)-3-(1,1,3,3-tetramethyl-butyl)-urea | C16H23Br2ClN2O | 454.63 | 454.8 |

| | | | | | |
|---|---|---|---|---|---|
| 84 | 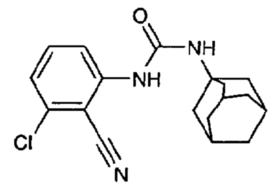 | 1-Adamantan-1-yl-3-(3-chloro-2-cyano-phenyl)-urea | C18H20ClN3O | 329.83 | 329.9 |
| 85 | 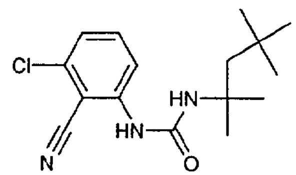 | 1-(3-Chloro-2-cyano-phenyl)-3-(1,1,3,3-tetramethyl-butyl)-urea | C16H22ClN3O | 307.82 | 308.1 |
| 86 | 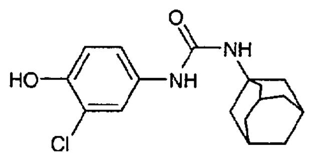 | 1-Adamantan-1-yl-3-(3-chloro-4-hydroxy-phenyl)-urea | C17H21ClN2O2 | 320.82 | 321.0 |
| 87 | 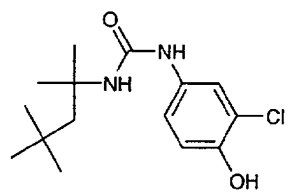 | 1-(3-Chloro-4-hydroxy-phenyl)-3-(1,1,3,3-tetramethyl-butyl)-urea | C15H23ClN2O2 | 298.81 | 299.0 |
| 88 | 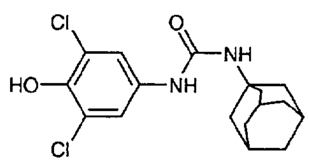 | 1-Adamantan-1-yl-3-(3,5-dichloro-4-hydroxy-phenyl)-urea | C17H20Cl2N2O2 | 355.26 | 355.4 |

| | | | | | |
|---|---|---|---|---|---|
| 89 | 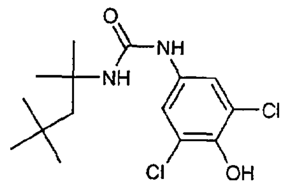 | 1-(3,5-Dichloro-4-hydroxy-phenyl)-3-(1,1,3,3-tetramethyl-butyl)-urea | C15H22Cl2N2O2 | 333.26 | 333.4 |
| 90 | 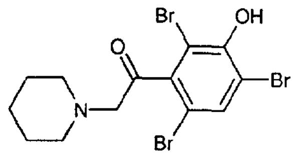 | 1-Piperidin-1-yl-2-(2,4,6-tribromo-3-hydroxy-phenyl)-ethanone | C13H14Br3NO2 | 455.97 | 456.2 |
| 91 | 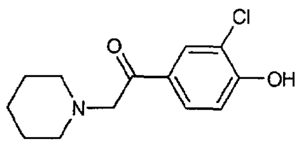 | 2-(3-Chloro-4-hydroxy-phenyl)-1-piperidin-1-yl-ethanone | C13H16ClNO2 | 253.73 | 254.0 |
| 92 | 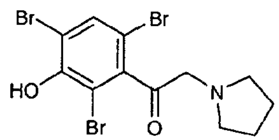 | 1-Pyrrolidin-1-yl-2-(2,4,6-tribromo-3-hydroxy-phenyl)-ethanone | C12H12Br3NO2 | 441.94 | 442.4 |
| 93 | 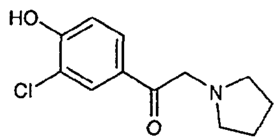 | 2-(3-Chloro-4-hydroxy-phenyl)-1-pyrrolidin-1-yl-ethanone | C12H14ClNO2 | 239.70 | 239.7 |
| 94 | 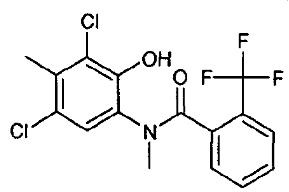 | N-(3,5-Dichloro-2-hydroxy-4-methyl-phenyl)-N-methyl-2-trifluoromethyl-benzamide | C16H12Cl2F3NO2 | 378.18 | 378.2 |

| | | | | | |
|---|---|---|---|---|---|
| 95 | 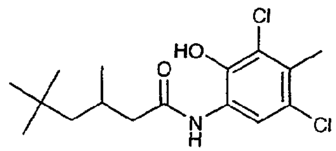 | 3,5,5-Trimethyl-hexanoic acid (3,5-dichloro-2-hydroxy-4-methyl-phenyl)-amide | C16H23Cl2NO2 | 332.27 | 332.3 |
| 96 | 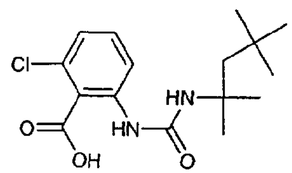 | 2-Chloro-6-[3-(1,1,3,3-tetramethyl-butyl)-ureido]-benzoic acid | C16H23ClN2O3 | 326.82 | 327.0 |
| 97 | 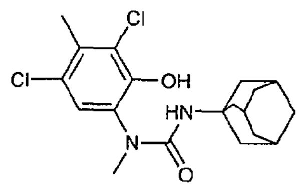 | 3-Adamantan-1-yl-1-(3,5-dichloro-2-hydroxy-4-methyl-phenyl)-1-methyl-urea | C19H24Cl2N2O2 | 383.32 | 383.4 |
| 98 | 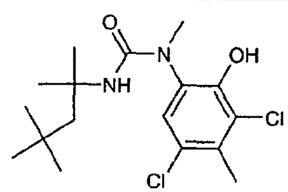 | 1-(3,5-Dichloro-2-hydroxy-4-methyl-phenyl)-1-methyl-3-(1,1,3,3-tetramethyl-butyl)-urea | C17H26Cl2N2O2 | 361.31 | 361.4 |
| 99 | 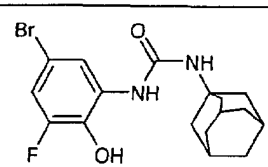 | 1-Adamantan-1-yl-3-(5-bromo-3-fluoro-2-hydroxy-phenyl)-urea | C17H20BrFN2O2 | 383.26 | 383.3 |

| | | | | | |
|---|---|---|---|---|---|
| 100 | 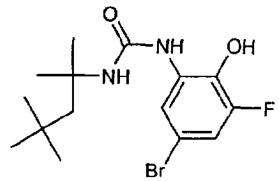 | 1-(5-Bromo-3-fluoro-2-hydroxy-phenyl)-3-(1,1,3,3-tetramethyl-butyl)-urea | C15H22BrFN2O2 | 361.25 | 361.2 |
| 101 | 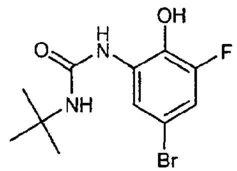 | 1-(5-Bromo-3-fluoro-2-hydroxy-phenyl)-3-tert-butyl-urea | C11H14BrFN2O2 | 305.15 | 305.2 |
| 102 | 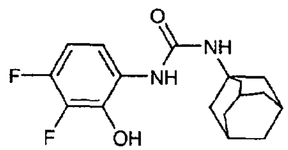 | 1-Adamantan-1-yl-3-(3,4-difluoro-2-hydroxy-phenyl)-urea | C17H20F2N2O2 | 322.35 | 322.5 |
| 103 | 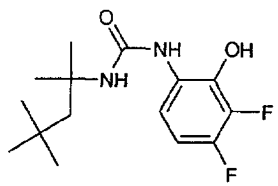 | 1-(3,4-Difluoro-2-hydroxy-phenyl)-3-(1,1,3,3-tetramethyl-butyl)-urea | C15H22F2N2O2 | 300.35 | 300.5 |
| 104 | 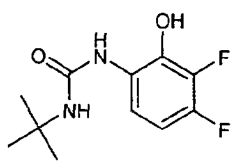 | 1-tert-Butyl-3-(3,4-difluoro-2-hydroxy-phenyl)-urea | C11H14F2N2O2 | 244.24 | 244.3 |
| 105 | 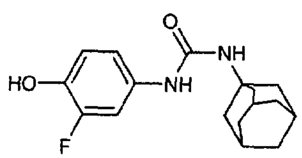 | 1-Adamantan-1-yl-3-(3-fluoro-4-hydroxy-phenyl)-urea | C17H21FN2O2 | 304.36 | 304.4 |

| | | | | | |
|---|---|---|---|---|---|
| 106 | 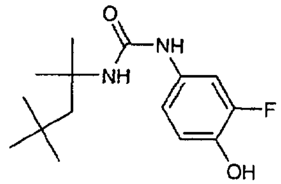 | 1-(3-Fluoro-4-hydroxy-phenyl)-3-(1,1,3,3-tetramethyl-butyl)-urea | C15H23FN2O2 | 282.36 | 282.4 |
| 107 | 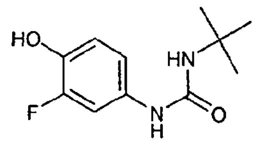 | 1-tert-Butyl-3-(3-fluoro-4-hydroxy-phenyl)-urea | C11H15FN2O2 | 226.25 | 226.4 |
| 108 | 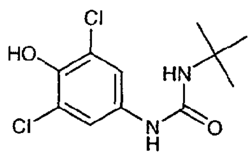 | 1-tert-Butyl-3-(3,5-dichloro-4-hydroxy-phenyl)-urea | C11H14Cl2N2O2 | 277.15 | 277.2 |
| 109 | 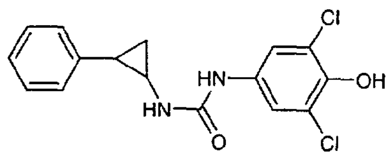 | 1-(3,5-Dichloro-4-hydroxy-phenyl)-3-(2-phenyl-cyclopropyl)-urea | C16H14Cl2N2O2 | 337.20 | 337.3 |
| 110 | 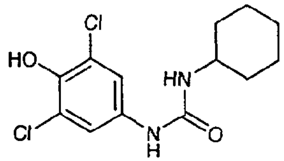 | 1-Cyclohexyl-3-(3,5-dichloro-4-hydroxy-phenyl)-urea | C13H16Cl2N2O2 | 303.19 | 303.4 |
| 111 | 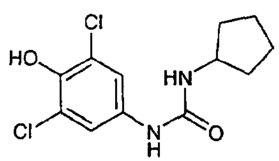 | 1-Cyclopentyl-3-(3,5-dichloro-4-hydroxy-phenyl)-urea | C12H14Cl2N2O2 | 289.16 | 289.4 |

| | | | | | |
|---|---|---|---|---|---|
| 112 | 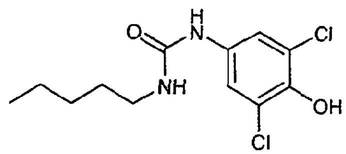 | 1-(3,5-Dichloro-4-hydroxy-phenyl)-3-pentyl-urea | C12H16Cl2N2O2 | 291.18 | 291.2 |
| 113 | 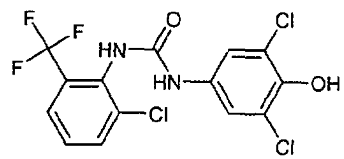 | 1-(2-Chloro-6-trifluoromethyl-phenyl)-3-(3,5-dichloro-4-hydroxy-phenyl)-urea | C14H8Cl3F3N2O2 | 399.58 | 399.8 |
| 114 | 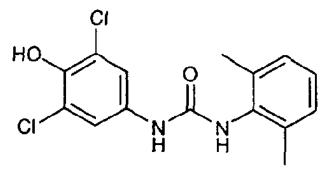 | 1-(3,5-Dichloro-4-hydroxy-phenyl)-3-(2,6-dimethyl-phenyl)-urea | C15H14Cl2N2O2 | 325.19 | 325.3 |
| 115 | 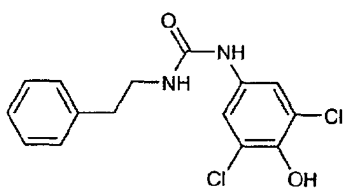 | 1-(3,5-Dichloro-4-hydroxy-phenyl)-3-phenethyl-urea | C15H14Cl2N2O2 | 325.19 | 325.3 |
| 116 | 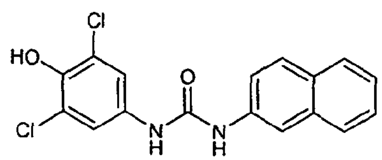 | 1-(3,5-Dichloro-4-hydroxy-phenyl)-3-naphthalen-2-yl-urea | C17H12Cl2N2O2 | 347.20 | 347.2 |

| | | | | | |
|---|---|---|---|---|---|
| 117 | 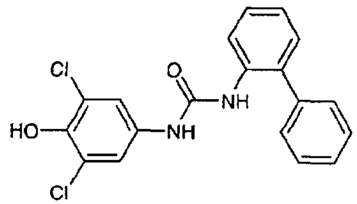 | 1-Biphenyl-2-yl-3-(3,5-dichloro-4-hydroxy-phenyl)-urea | C19H14Cl2N2O2 | 373.24 | 373.3 |
| 118 | 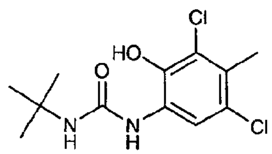 | 1-tert-Butyl-3-(3,5-dichloro-2-hydroxy-4-methyl-phenyl)-urea | C12H16Cl2N2O2 | 291.18 | 291.2 |
| 119 | 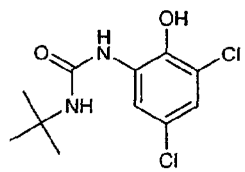 | 1-tert-Butyl-3-(3,5-dichloro-2-hydroxy-phenyl)-urea | C11H14Cl2N2O2 | 277.15 | 277.2 |
| 120 | 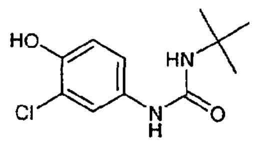 | 1-tert-Butyl-3-(3-chloro-4-hydroxy-phenyl)-urea | C11H15ClN2O2 | 242.70 | 242.9 |
| 121 | 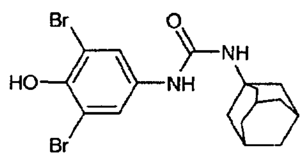 | 1-Adamantan-1-yl-3-(3,5-dibromo-4-hydroxy-phenyl)-urea | C17H20Br2N2O2 | 444.17 | 444.1 |
| 122 | 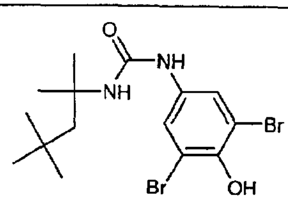 | 1-(3,5-Dibromo-4-hydroxy-phenyl)-3-(1,1,3,3-tetramethyl-butyl)-urea | C15H22Br2N2O2 | 422.16 | 422.3 |

| | | | | | |
|---|---|---|---|---|---|
| 123 | 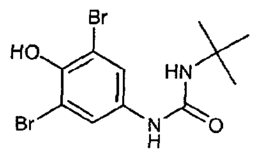 | 1-tert-Butyl-3-(3,5-dibromo-4-hydroxy-phenyl)-urea | C11H14Br2N2O2 | 366.05 | 366.1 |
| 124 | 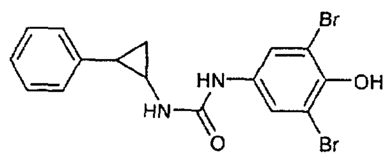 | 1-(3,5-Dibromo-4-hydroxy-phenyl)-3-(2-phenyl-cyclopropyl)-urea | C16H14Br2N2O2 | 426.11 | 426.1 |
| 125 | 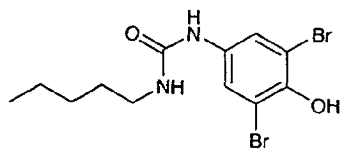 | 1-(3,5-Dibromo-4-hydroxy-phenyl)-3-pentyl-urea | C12H16Br2N2O2 | 380.08 | 380.1 |
| 126 | 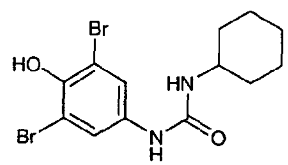 | 1-Cyclohexyl-3-(3,5-dibromo-4-hydroxy-phenyl)-urea | C13H16Br2N2O2 | 392.09 | 392.1 |
| 127 | 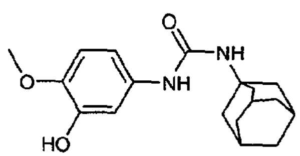 | 1-Adamantan-1-yl-3-(3-hydroxy-4-methoxy-phenyl)-urea | C18H24N2O3 | 316.40 | 317.2 |

| | | | | | |
|---|---|---|---|---|---|
| 128 | 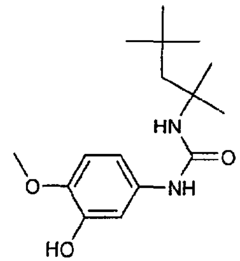 | 1-(3-Hydroxy-4-methoxy-phenyl)-3-(1,1,3,3-tetramethyl-butyl)-urea | C16H26N2O3 | 294.39 | 295.4 |
| 129 | 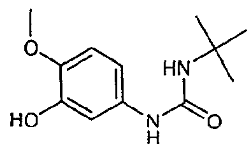 | 1-tert-Butyl-3-(3-hydroxy-4-methoxy-phenyl)-urea | C12H18N2O3 | 238.29 | 239.2 |
| 130 | 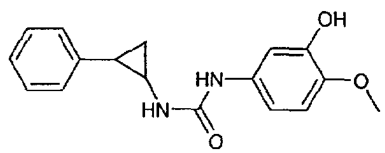 | 1-(3-Hydroxy-4-methoxy-phenyl)-3-(2-phenyl-cyclopropyl)-urea | C17H18N2O3 | 298.34 | 299.3 |
| 131 | 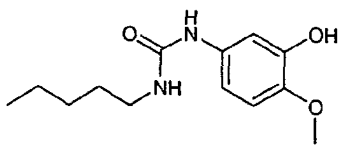 | 1-(3-Hydroxy-4-methoxy-phenyl)-3-pentyl-urea | C13H20N2O3 | 252.31 | 253.4 |
| 132 | 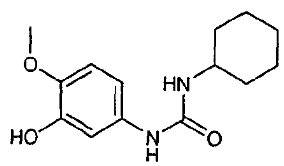 | 1-Cyclohexyl-3-(3-hydroxy-4-methoxy-phenyl)-urea | C14H20N2O3 | 264.32 | 265.2 |

| | | | | | |
|---|---|---|---|---|---|
| 133 | 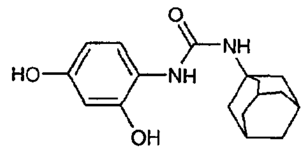 | 1-Adamantan-1-yl-3-(2,4-dihydroxy-phenyl)-urea | C17H22N2O3 | 302.37 | 303.4 |
| 134 | 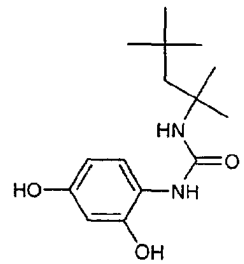 | 1-(2,4-Dihydroxy-phenyl)-3-(1,1,3,3-tetramethyl-butyl)-urea | C15H24N2O3 | 280.37 | 281.3 |
| 135 | 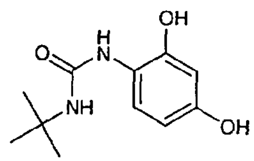 | 1-tert-Butyl-3-(2,4-dihydroxy-phenyl)-urea | C11H16N2O3 | 224.26 | 225.2 |
| 136 | 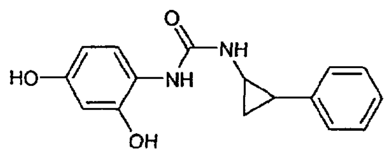 | 1-(2,4-Dihydroxy-phenyl)-3-(2-phenyl-cyclopropyl)-urea | C16H16N2O3 | 284.31 | 285.4 |
| 137 | 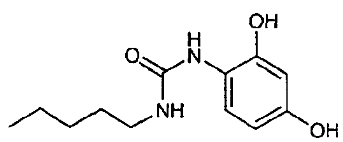 | 1-(2,4-Dihydroxy-phenyl)-3-pentyl-urea | C12H18N2O3 | 238.29 | 239.3 |

| | | | | | |
|---|---|---|---|---|---|
| 138 | 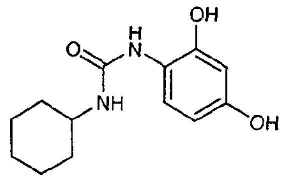 | 1-Cyclohexyl-3-(2,4-dihydroxy-phenyl)-urea | C13H18N2O3 | 250.30 | 251.3 |
| 139 | 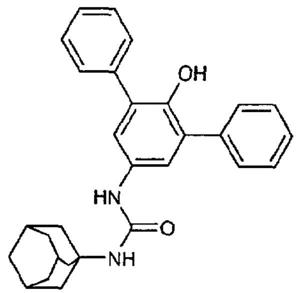 | 1-Adamantan-1-yl-3-(2'-hydroxy-[1,1';3',1"]terphenyl-5'-yl)-urea | C29H30N2O2 | 438.57 | 439.5 |
| 140 | 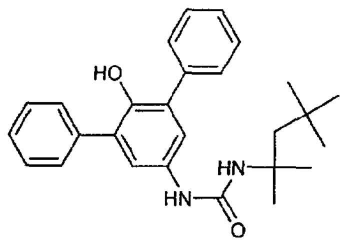 | 1-(2'-Hydroxy-[1,1';3',1"]terphenyl-5'-yl)-3-(1,1,3,3-tetramethyl-butyl)-urea | C27H32N2O2 | 416.56 | 417.6 |
| 141 | 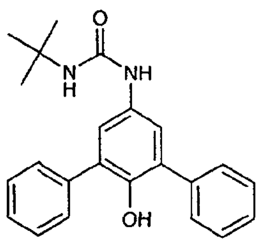 | 1-tert-Butyl-3-(2'-hydroxy-[1,1';3',1"]terphenyl-5'-yl)-urea | C23H24N2O2 | 360.45 | 361.3 |

| | | | | | |
|---|---|---|---|---|---|
| 142 | 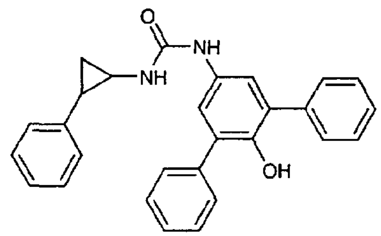 | 1-(2'-Hydroxy-[1,1';3',1"]terphenyl-5'-yl)-3-(2-phenyl-cyclopropyl)-urea | C28H24N2O2 | 420.51 | 421.4 |
| 143 | 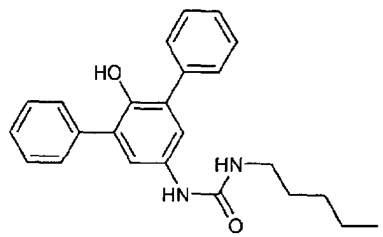 | 1-(2'-Hydroxy-[1,1';3',1"]terphenyl-5'-yl)-3-pentyl-urea | C24H26N2O2 | 374.48 | 375.4 |
| 144 | 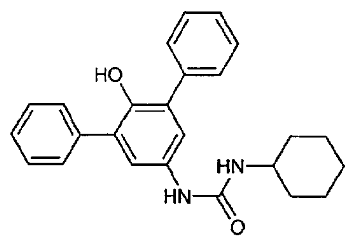 | 1-Cyclohexyl-3-(2'-hydroxy-[1,1';3',1"]terphenyl-5'-yl)-urea | C25H26N2O2 | 386.49 | 387.5 |
| 145 | 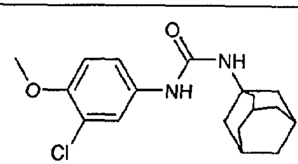 | 1-Adamantan-1-yl-3-(3-chloro-4-methoxy-phenyl)-urea | C18H23ClN2O2 | 334.84 | 335.7 |

| | | | | | |
|---|---|---|---|---|---|
| 146 | 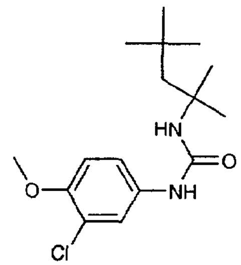 | 1-(3-Chloro-4-methoxy-phenyl)-3-(1,1,3,3-tetramethyl-butyl)-urea | C16H25ClN2O2 | 312.84 | 313.8 |
| 147 | 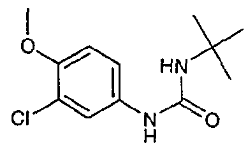 | 1-tert-Butyl-3-(3-chloro-4-methoxy-phenyl)-urea | C12H17ClN2O2 | 256.73 | 257.8 |
| 148 | 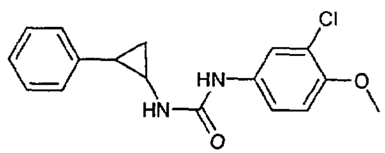 | 1-(3-Chloro-4-methoxy-phenyl)-3-(2-phenyl-cyclopropyl)-urea | C17H17ClN2O2 | 316.79 | 317.8 |
| 149 | 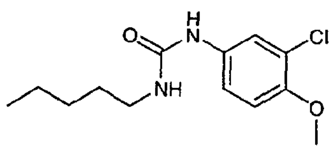 | 1-(3-Chloro-4-methoxy-phenyl)-3-pentyl-urea | C13H19ClN2O2 | 270.76 | 271.8 |
| 150 | 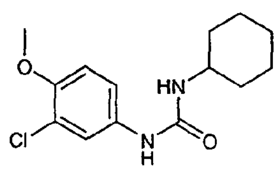 | 1-(3-Chloro-4-methoxy-phenyl)-3-cyclohexyl-urea | C14H19ClN2O2 | 282.77 | 283.8 |
| 151 | 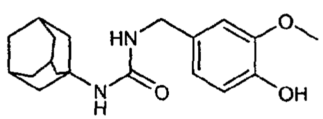 | 1-Adamantan-1-yl-3-(4-hydroxy-3-methoxy-benzyl)-urea | C19H26N2O3 | 330.43 | 331.3 |

| | | | | | |
|---|---|---|---|---|---|
| 152 | 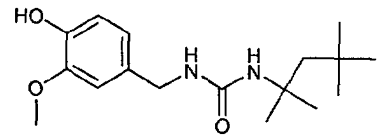 | 1-(4-Hydroxy-3-methoxy-benzyl)-3-(1,1,3,3-tetramethyl-butyl)-urea | C17H28N2O3 | 308.42 | 309.3 |
| 153 | 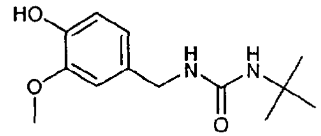 | 1-tert-Butyl-3-(4-hydroxy-3-methoxy-benzyl)-urea | C13H20N2O3 | 252.31 | 253.3 |
| 154 | 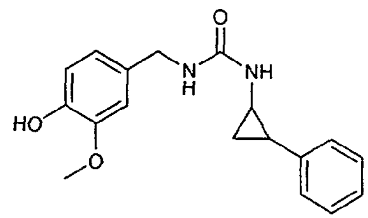 | 1-(4-Hydroxy-3-methoxy-benzyl)-3-(2-phenyl-cyclopropyl)-urea | C18H20N2O3 | 312.37 | 313.3 |
| 155 | 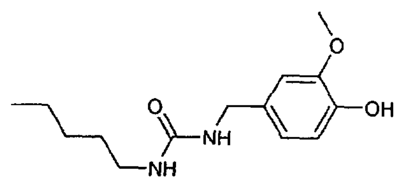 | 1-(4-Hydroxy-3-methoxy-benzyl)-3-pentyl-urea | C14H22N2O3 | 266.34 | 267.3 |
| 156 | 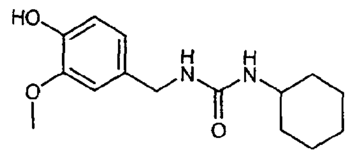 | 1-Cyclohexyl-3-(4-hydroxy-3-methoxy-benzyl)-urea | C15H22N2O3 | 278.35 | 279.4 |
| 157 | 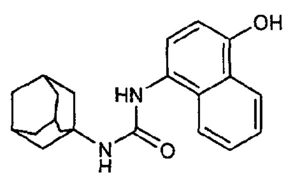 | 1-Adamantan-1-yl-3-(4-hydroxy-naphthalen-1-yl)-urea | C21H24N2O2 | 336.43 | 337.4 |

| | | | | | |
|---|---|---|---|---|---|
| 158 | 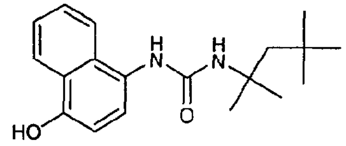 | 1-(4-Hydroxy-naphthalen-1-yl)-3-(1,1,3,3-tetramethyl-butyl)-urea | C19H26N2O2 | 314.43 | 315.3 |
| 159 | 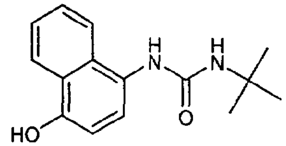 | 1-tert-Butyl-3-(4-hydroxy-naphthalen-1-yl)-urea | C15H18N2O2 | 258.32 | 259.2 |
| 160 | 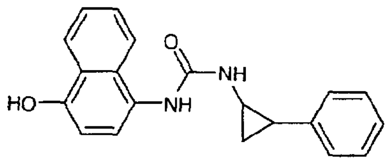 | 1-(4-Hydroxy-naphthalen-1-yl)-3-(2-phenyl-cyclopropyl)-urea | C20H18N2O2 | 318.37 | 319.3 |
| 161 | 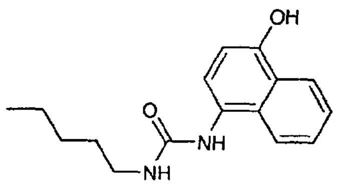 | 1-(4-Hydroxy-naphthalen-1-yl)-3-pentyl-urea | C16H20N2O2 | 272.35 | 273.3 |
| 162 | 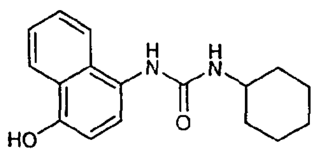 | 1-Cyclohexyl-3-(4-hydroxy-naphthalen-1-yl)-urea | C17H20N2O2 | 284.36 | 285.3 |
| 163 | 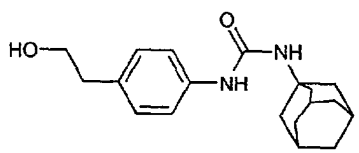 | 1-Adamantan-1-yl-3-[4-(2-hydroxy-ethyl)-phenyl]-urea | C19H26N2O2 | 314.43 | 315.4 |

| | | | | | |
|---|---|---|---|---|---|
| 164 | 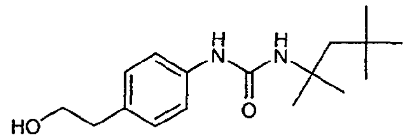 | 1-[4-(2-Hydroxy-ethyl)-phenyl]-3-(1,1,3,3-tetramethyl-butyl)-urea | C17H28N2O2 | 292.42 | 293.3 |
| 165 | 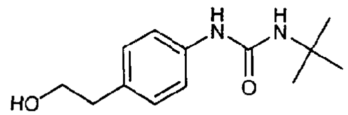 | 1-tert-Butyl-3-[4-(2-hydroxy-ethyl)-phenyl]-urea | C13H20N2O2 | 236.31 | 237.3 |
| 166 | 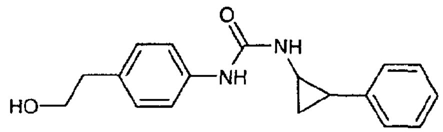 | 1-[4-(2-Hydroxy-ethyl)-phenyl]-3-(2-phenyl-cyclopropyl)-urea | C18H20N2O2 | 296.37 | 297.3 |
| 167 | 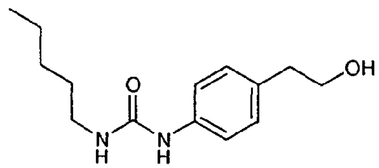 | 1-[4-(2-Hydroxy-ethyl)-phenyl]-3-pentyl-urea | C14H22N2O2 | 250.34 | 251.3 |
| 168 | 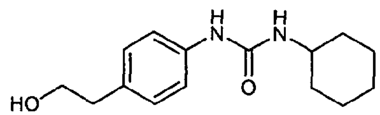 | 1-Cyclohexyl-3-[4-(2-hydroxy-ethyl)-phenyl]-urea | C15H22N2O2 | 262.35 | 263.3 |
| 169 | 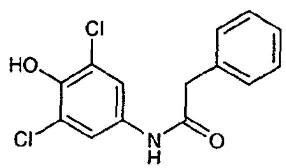 | N-(3,5-Dichloro-4-hydroxy-phenyl)-2-phenyl-acetamide | C14H11Cl2NO2 | 296.15 | 296.2 |

| | | | | | |
|---|---|---|---|---|---|
| 170 | 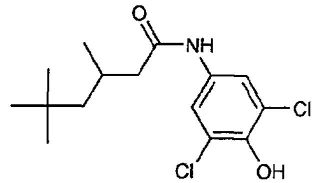 | 3,5,5-Trimethyl-hexanoic acid (3,5-dichloro-4-hydroxy-phenyl)-amide | C15H21Cl2NO2 | 318.24 | 318.4 |
| 171 | 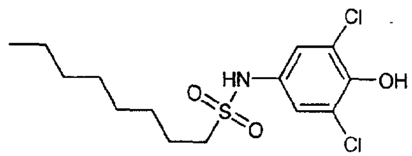 | Octane-1-sulfonic acid (3,5-dichloro-4-hydroxy-phenyl)-amide | C14H21Cl2NO3S | 354.29 | 354.5 |
| 172 | 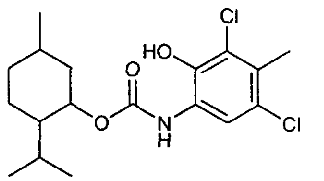 | (3,5-Dichloro-2-hydroxy-4-methyl-phenyl)-carbamic acid 2-isopropyl-5-methyl-cyclohexyl ester | C18H25Cl2NO3 | 374.31 | 374.4 |
| 173 | 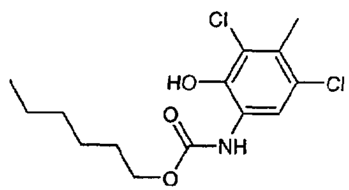 | (3,5-Dichloro-2-hydroxy-4-methyl-phenyl)-carbamic acid hexyl ester | C14H19Cl2NO3 | 320.22 | 320.2 |
| 174 | 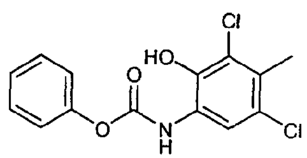 | (3,5-Dichloro-2-hydroxy-4-methyl-phenyl)-carbamic acid phenyl ester | C14H11Cl2NO3 | 312.15 | 320.2 |
| 175 | 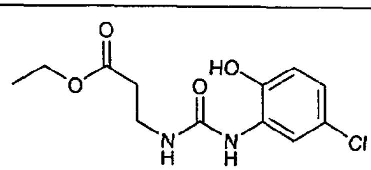 | 3-[3-(5-Chloro-2-hydroxy-phenyl)-ureido]-propionic acid ethyl ester | C13H9Cl2NO3 | 298.13 | 298.2 |

| | | | | | |
|---|---|---|---|---|---|
| 176 | 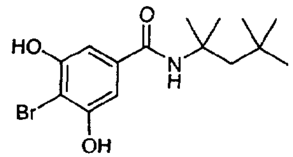 | 4-Bromo-3,5-dihydroxy-N-(1,1,3,3-tetramethyl-butyl)-benzamide | C15H22BrNO3 | 344.2 | 344.3 |
| 177 | 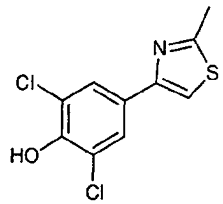 | 2,6-Dichloro-4-(2-methyl-thiazol-4-yl)-phenol | C10H7Cl2NOS | 260.1 | 260.4 |
| 178 | 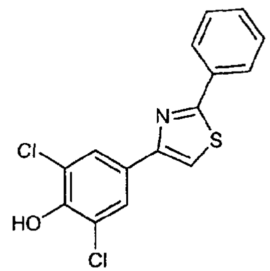 | 2,6-Dichloro-4-(2-phenyl-thiazol-4-yl)-phenol | C15H9Cl2NOS | 322.2 | 322.2 |
| 179 | 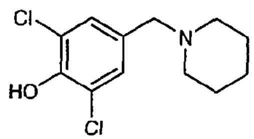 | 2,6-Dichloro-4-piperidin-1-ylmethyl-phenol | C12H15Cl2NO | 260.2 | 260.3 |
| 180 | 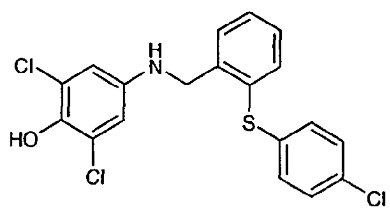 | 2,6-Dichloro-4-[2-(4-chloro-phenylsulfanyl)-benzylamino]-phenol | C19H14Cl3NOS | 410.7 | 409.9 |

| | | | | | |
|---|---|---|---|---|---|
| 181 | 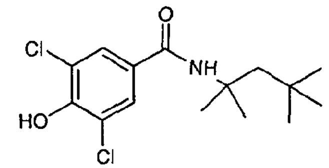 | 3,5-Dichloro-4-hydroxy-N-(1,1,3,3-tetramethyl-butyl)-benzamide | C15H21Cl2NO2 | 318.2 | 318.1 |
| 182 | 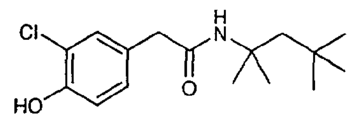 | 2-(3-Chloro-4-hydroxy-phenyl)-N-(1,1,3,3-tetramethyl-butyl)-acetamide | C16H24ClNO2 | 297.8 | 298.1 |
| 183 | 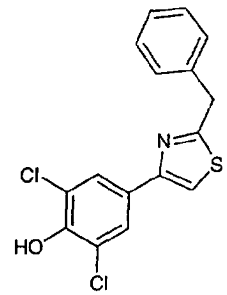 | 4-(2-Benzyl-thiazol-4-yl)-2,6-dichloro-phenol | C16H11Cl2NOS | 336.2 | 336.1 |
| 184 | 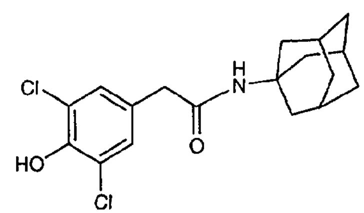 | N-Adamantan-1-yl-2-(3,5-dichloro-4-hydroxy-phenyl)-acetamide | C18H21Cl2NO2 | 354.3 | 354.1 |
| 185 | 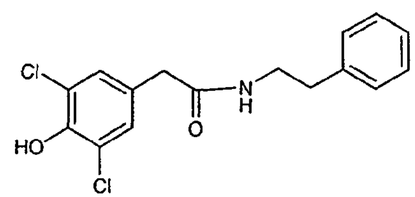 | 2-(3,5-Dichloro-4-hydroxy-phenyl)-N-phenethyl-acetamide | C16H15Cl2NO2 | 324.2 | 324.1 |

| | | | | | |
|---|---|---|---|---|---|
| 186 | 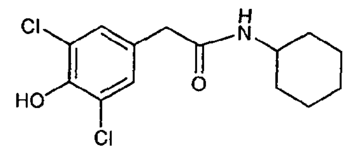 | N-Cyclohexyl-2-(3,5-dichloro-4-hydroxy-phenyl)-acetamide | C14H17Cl2NO2 | 302.2 | 302.1 |
| 187 | 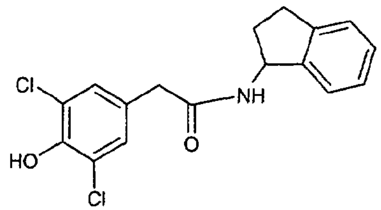 | 2-(3,5-Dichloro-4-hydroxy-phenyl)-N-indan-1-yl-acetamide | C17H15Cl2NO2 | 336.2 | 335.9 |
| 188 | 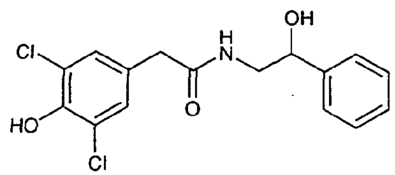 | 2-(3,5-Dichloro-4-hydroxy-phenyl)-N-(2-hydroxy-2-phenyl-ethyl)-acetamide | C16H15Cl2NO3 | 340.2 | 339.9 |
| 189 | 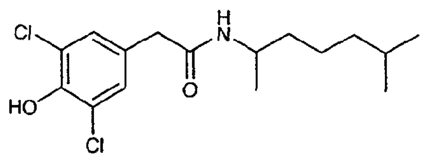 | 2-(3,5-Dichloro-4-hydroxy-phenyl)-N-(1,5-dimethyl-hexyl)-acetamide | C16H23Cl2NO2 | 332.3 | 332.1 |
| 190 | 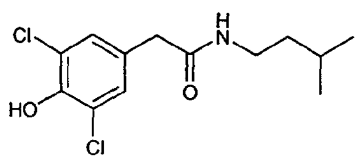 | 2-(3,5-Dichloro-4-hydroxy-phenyl)-N-(3-methyl-butyl)-acetamide | C13H17Cl2NO2 | 290.2 | 290.2 |

| | | | | | |
|---|---|---|---|---|---|
| 191 | 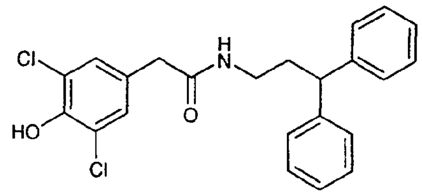 | 2-(3,5-Dichloro-4-hydroxy-phenyl)-N-(3,3-diphenyl-propyl)-acetamide | C23H21Cl2NO2 | 414.3 | 414.1 |
| 192 | 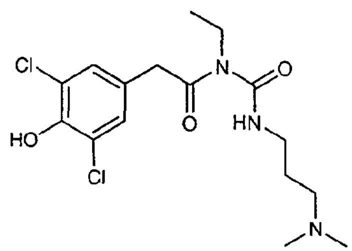 | 1-[2-(3,5-Dichloro-4-hydroxy-phenyl)-acetyl]-3-(3-dimethylamino-propyl)-1-ethyl-urea | C16H23Cl2N3O3 | 376.3 | 376.0 |
| 193 | 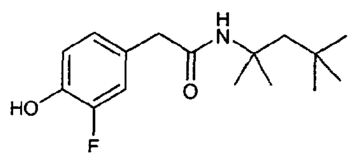 | 2-(3-Fluoro-4-hydroxy-phenyl)-N-(1,1,3,3-tetramethyl-butyl)-acetamide | C16H24FNO2 | 281.4 | 282.0 |
| 194 | 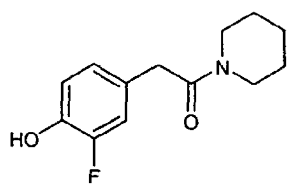 | 2-(3-Fluoro-4-hydroxy-phenyl)-1-piperidin-1-yl-ethanone | C13H16FNO2 | 237.3 | 238.2 |

| | | | | | |
|---|---|---|---|---|---|
| 195 | 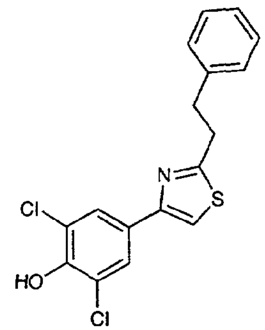 | 2,6-Dichloro-4-(2-phenethyl-thiazol-4-yl)-phenol | C17H13Cl2NOS | 350.3 | 350.2 |
| 196 | 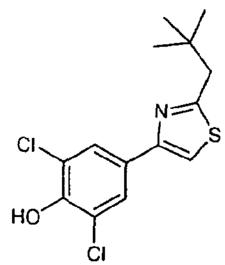 | 2,6-Dichloro-4-[2-(2,2-dimethyl-propyl)-thiazol-4-yl]-phenol | C14H15Cl2NOS | 316.2 | 316.1 |
| 197 | 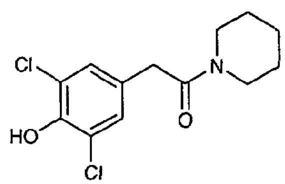 | 2-(3,5-Dichloro-4-hydroxy-phenyl)-1-piperidin-1-yl-ethanone | C13H15Cl2NO2 | 288.2 | 288.3 |
| 198 | 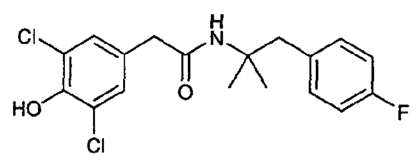 | 2-(3,5-Dichloro-4-hydroxy-phenyl)-N-[2-(4-fluoro-phenyl)-1,1-dimethyl-ethyl]-acetamide | C18H18Cl2FNO2 | 370.2 | 370.0 |

| | | | | | |
|---|---|---|---|---|---|
| 199 | 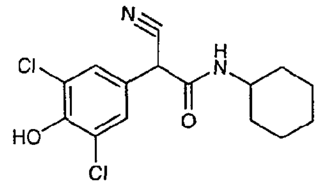 | 2-Cyano-N-cyclohexyl-2-(3,5-dichloro-4-hydroxy-phenyl)-acetamide | C15H16Cl2N2O2 | 327.2 | 327.1 |
| 200 | 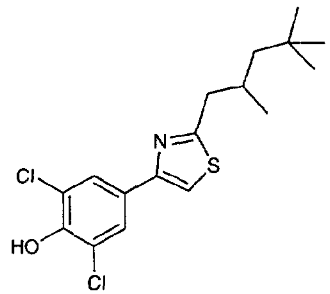 | 2,6-Dichloro-4-[2-(2,4,4-trimethyl-pentyl)-thiazol-4-yl]-phenol | C17H21Cl2NOS | 358.3 | 358.2 |
| 201 | 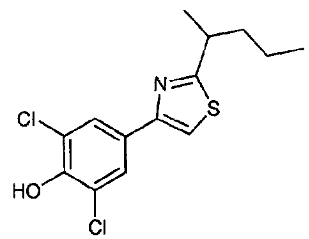 | 2,6-Dichloro-4-[2-(1-methyl-butyl)-thiazol-4-yl]-phenol | C14H15Cl2NOS | 316.2 | 316.2 |
| 202 | 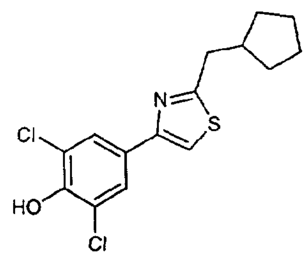 | 2,6-Dichloro-4-(2-cyclopentylmethyl-thiazol-4-yl)-phenol | C15H15Cl2NOS | 328.3 | 328.2 |

| | | | | | |
|---|---|---|---|---|---|
| 203 | 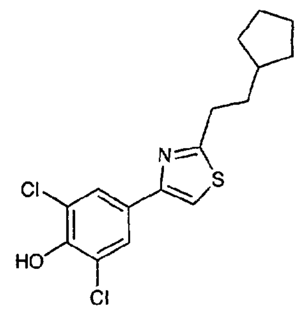 | 2,6-Dichloro-4-[2-(2-cyclopentyl-ethyl)-thiazol-4-yl]-phenol | C16H17Cl2NOS | 342.3 | 342.3 |
| 204 | 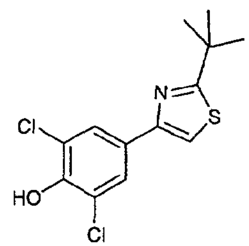 | 4-(2-tert-Butyl-thiazol-4-yl)-2,6-dichloro-phenol | C13H13Cl2NOS | 302.2 | 302.2 |
| 205 | 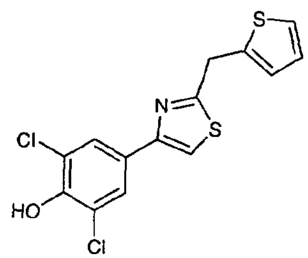 | 2,6-Dichloro-4-(2-thiophen-2-ylmethyl-thiazol-4-yl)-phenol | C14H9Cl2NOS2 | 342.3 | 342.1 |

| | | | | | |
|---|---|---|---|---|---|
| 206 | 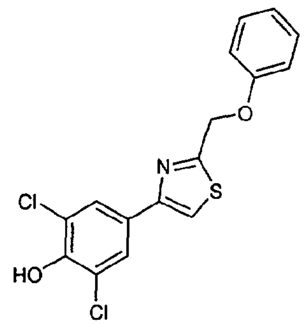 | 2,6-Dichloro-4-(2-phenoxymethyl-thiazol-4-yl)-phenol | C16H11Cl2NO2S | 352.2 | 352.2 |
| 207 | 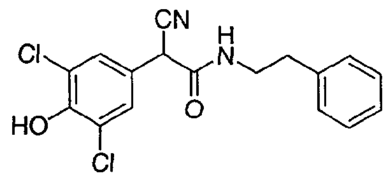 | 2-Cyano-2-(3,5-dichloro-4-hydroxy-phenyl)-N-phenethyl-acetamide | C17H14Cl2N2O2 | 349.2 | 349.0 |
| 208 | 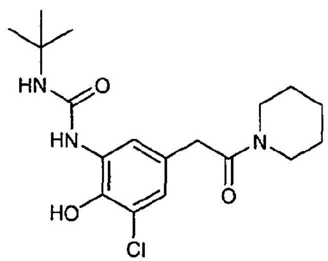 | 1-tert-Butyl-3-[3-chloro-2-hydroxy-5-(2-oxo-2-piperidin-1-yl-ethyl)-phenyl]-urea | C18H26ClN3O3 | 367.9 | 368.1 |
| 209 | 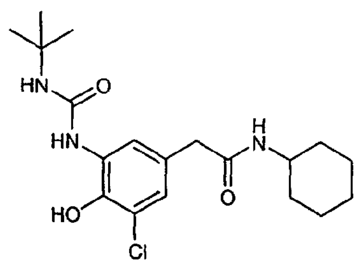 | 2-[3-(3-tert-Butyl-ureido)-5-chloro-4-hydroxy-phenyl]-N-cyclohexyl-acetamide | C19H28ClN3O3 | 381.9 | 382.1 |

| | | | | | |
|---|---|---|---|---|---|
| 210 | 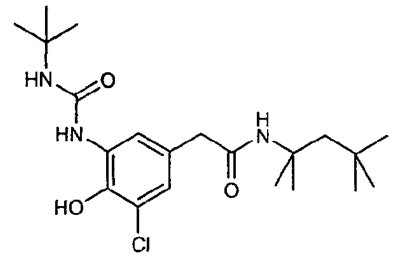 | 2-[3-(3-tert-Butyl-ureido)-5-chloro-4-hydroxy-phenyl]-N-(1,1,3,3-tetramethyl-butyl)-acetamide | C21H34ClN3O3 | 412.0 | 412.1 |
| 211 | 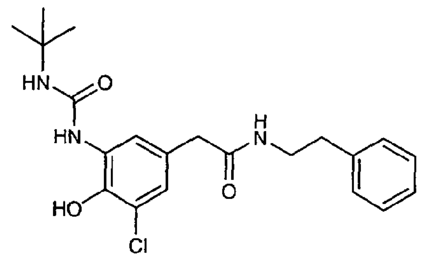 | 2-[3-(3-tert-Butyl-ureido)-5-chloro-4-hydroxy-phenyl]-N-phenethyl-acetamide | C21H26ClN3O3 | 403.9 | 404.1 |
| 212 | 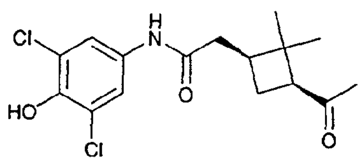 | 2-(3-Acetyl-2,2-dimethyl-cyclobutyl)-N-(3,5-dichloro-4-hydroxy-phenyl)-acetamide | C16H19Cl2NO3 | 344.23 | 344.4 |
| 213 | 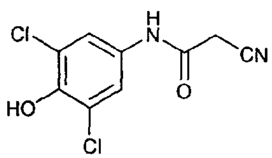 | 2-Cyano-N-(3,5-dichloro-4-hydroxy-phenyl)-acetamide | C9H6Cl2N2O2 | 245.06 | 245.2 |
| 214 | 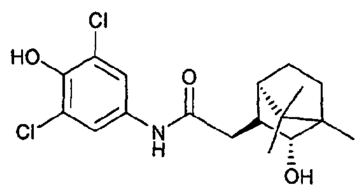 | N-(3,5-Dichloro-4-hydroxy-phenyl)-2-(3-hydroxy-4,7,7-trimethyl-bicyclo[2.2.1]hept-2- | C18H23Cl2NO3 | 372.29 | 372.2 |

| | | | | | |
|---|---|---|---|---|---|
| | | yl)-acetamide | | | |
| 215 | 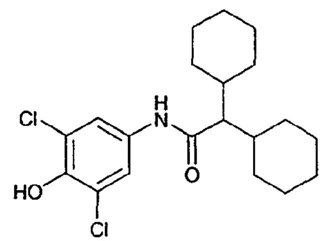 | 2,2-Dicyclohexyl-N-(3,5-dichloro-4-hydroxy-phenyl)-acetamide | C20H27Cl2NO2 | 384.34 | 384.0 |
| 216 | 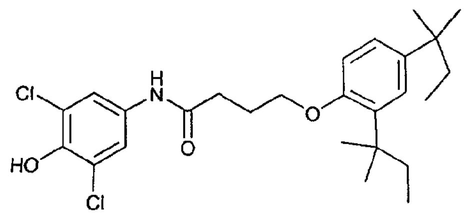 | 4-[2,4-Bis-(1,1-dimethyl-propyl)-phenoxy]-N-(3,5-dichloro-4-hydroxy-phenyl)-butyramide | C26H35Cl2NO3 | 480.47 | 480.5 |
| 217 | 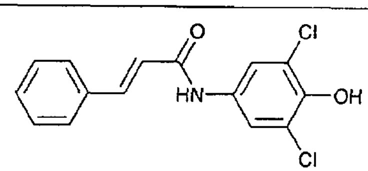 | N-(3,5-Dichloro-4-hydroxy-phenyl)-3-phenyl-acrylamide | C15H11Cl2NO2 | 308.16 | 308.1 |
| 218 | 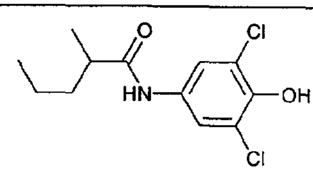 | 2-Methyl-pentanoic acid (3,5-dichloro-4-hydroxy-phenyl)-amide | C12H15Cl2NO2 | 276.16 | 276.3 |

| | | | | | |
|---|---|---|---|---|---|
| 219 | 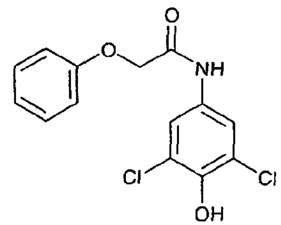 | N-(3,5-Dichloro-4-hydroxy-phenyl)-2-phenoxy-acetamide | C14H11Cl2NO3 | 312.15 | 312.0 |
| 220 | 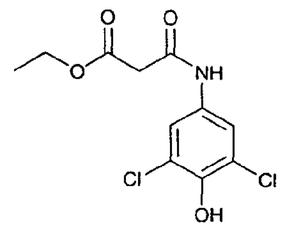 | N-(3,5-Dichloro-4-hydroxy-phenyl)-malonamic acid ethyl ester | C11H11Cl2NO4 | 292.12 | 292.1 |
| 221 | 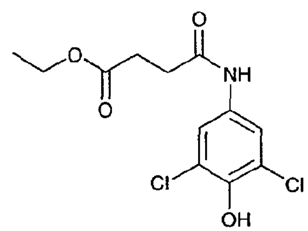 | N-(3,5-Dichloro-4-hydroxy-phenyl)-succinamic acid ethyl ester | C12H13Cl2NO4 | 306.14 | 306.0 |
| 222 | 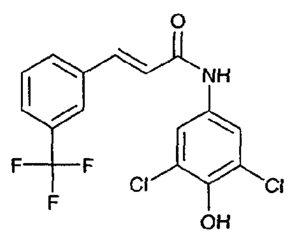 | N-(3,5-Dichloro-4-hydroxy-phenyl)-3-(3-trifluoromethyl-phenyl)-acrylamide | C16H10Cl2F3NO2 | 376.16 | 376.2 |

| | | | | | |
|---|---|---|---|---|---|
| 223 | 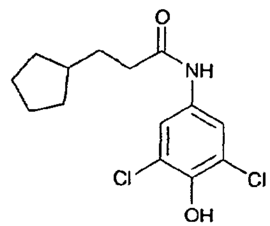 | 3-Cyclopentyl-N-(3,5-dichloro-4-hydroxy-phenyl)-propionamide | C14H17Cl2NO2 | 302.20 | 302.2 |
| 224 | 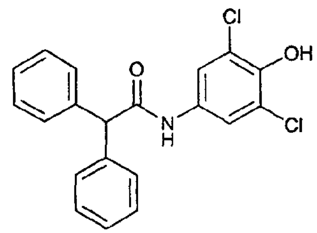 | N-(3,5-Dichloro-4-hydroxy-phenyl)-2,2-diphenyl-acetamide | C20H15Cl2NO2 | 372.24 | 372.1 |
| 225 | 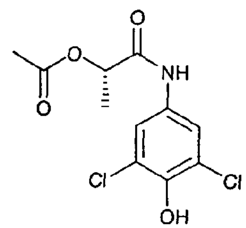 | Acetic acid 1-(3,5-dichloro-4-hydroxy-phenylcarbamoyl)-ethyl ester | C11H11Cl2NO4 | 292.12 | 292.1 |
| 226 | 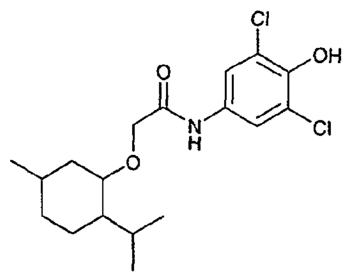 | N-(3,5-Dichloro-4-hydroxy-phenyl)-2-(2-isopropyl-5-methyl-cyclohexyloxy)-acetamide | C18H25Cl2NO3 | 374.30 | 374.8 |

| | | | | | |
|---|---|---|---|---|---|
| 227 | 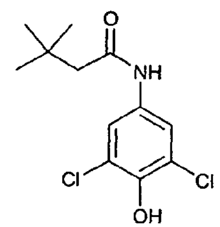 | N-(3,5-Dichloro-4-hydroxy-phenyl)-3,3-dimethyl-butyramide | C12H15Cl2NO2 | 276.16 | 276.4 |
| 228 | 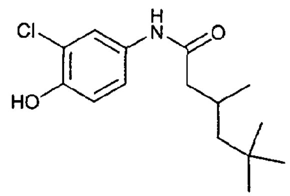 | 3,5,5-Trimethyl-hexanoic acid (3-chloro-4-hydroxy-phenyl)-amide | C15H22ClNO2 | 283.79 | 284.4 |
| 229 | 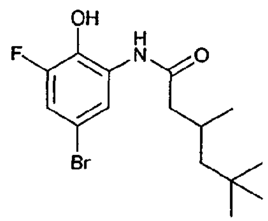 | 3,5,5-Trimethyl-hexanoic acid (5-bromo-3-fluoro-2-hydroxy-phenyl)-amide | C15H21BrFNO2 | 346.24 | 346.5 |
| 230 | 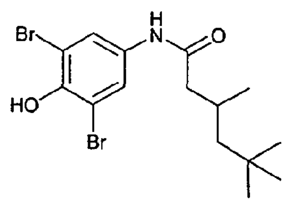 | 3,5,5-Trimethyl-hexanoic acid (3,5-dibromo-4-hydroxy-phenyl)-amide | C15H21Br2NO2 | 407.14 | 408.3 |

| | | | | | |
|---|---|---|---|---|---|
| 231 | 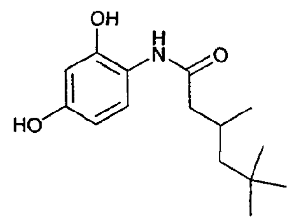 | 3,5,5-Trimethyl-hexanoic acid (2,4-dihydroxy-phenyl)-amide | C15H23NO3 | 265.35 | 266.3 |
| 232 | 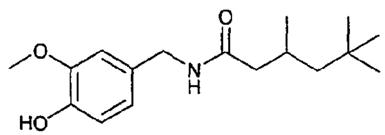 | 3,5,5-Trimethyl-hexanoic acid 4-hydroxy-3-methoxy-benzylamide | C17H27NO3 | 293.40 | 294.3 |
| 233 | 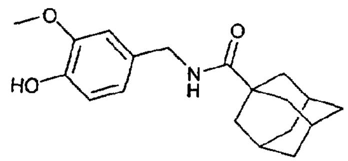 | Adamantane-1-carboxylic acid 4-hydroxy-3-methoxy-benzylamide | C19H25NO3 | 315.41 | 316.3 |
| 234 | 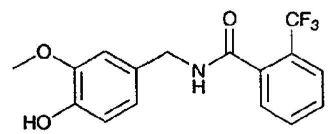 | N-(4-Hydroxy-3-methoxy-benzyl)-2-trifluoromethyl-benzamide | C16H14F3NO3 | 325.28 | 326.2 |
| 235 | 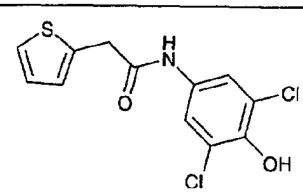 | N-(3,5-Dichloro-4-hydroxy-phenyl)-2-thiophen-2-yl-acetamide | C12H9Cl2NO2S | 302.18 | 302.0 |

| | | | | | |
|---|---|---|---|---|---|
| 236 | 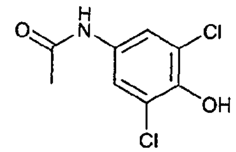 | N-(3,5-Dichloro-4-hydroxy-phenyl)-acetamide | $C_8H_7Cl_2NO_2$ | 220.05 | 220.2 |
| 237 | 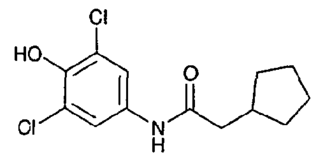 | 2-Cyclopentyl-N-(3,5-dichloro-4-hydroxy-phenyl)-acetamide | $C_{13}H_{15}Cl_2NO_2$ | 288.17 | 288.5 |
| 238 | 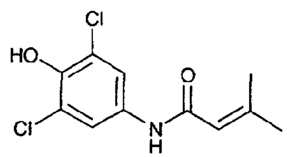 | 3-Methyl-but-2-enoic acid (3,5-dichloro-4-hydroxy-phenyl)-amide | $C_{11}H_{11}Cl_2NO_2$ | 260.12 | 260.1 |
| 239 | 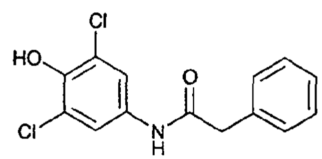 | N-(3,5-Dichloro-4-hydroxy-phenyl)-2-phenyl-acetamide | $C_{14}H_{11}Cl_2NO_2$ | 296.15 | 296.1 |
| 240 | 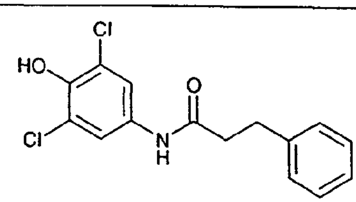 | N-(3,5-Dichloro-4-hydroxy-phenyl)-3-phenyl-propionamide | $C_{15}H_{13}Cl_2NO_2$ | 310.18 | 310.1 |
| 241 | 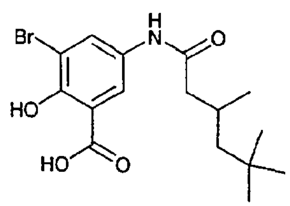 | 3-Bromo-2-hydroxy-5-(3,5,5-trimethyl-hexanoylamino)-benzoic acid | $C_{16}H_{22}BrNO_4$ | 372.25 | 372.5 |

| | | | | | |
|---|---|---|---|---|---|
| 242 | 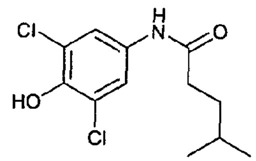 | 4-Methyl-pentanoic acid (3,5-dichloro-4-hydroxy-phenyl)-amide | C12H15Cl2NO2 | 276.16 | 276.4 |
| 243 | 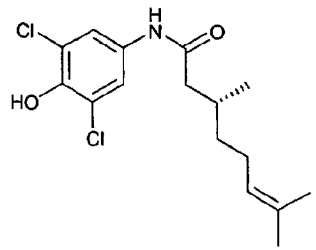 | 3,7-Dimethyl-oct-6-enoic acid (3,5-dichloro-4-hydroxy-phenyl)-amide | C16H21Cl2NO2 | 330.25 | 330.7 |
| 244 | 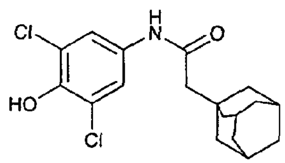 | 2-Adamantan-1-yl-N-(3,5-dichloro-4-hydroxy-phenyl)-acetamide | C18H21Cl2NO2 | 354.27 | 354.4 |
| 245 | 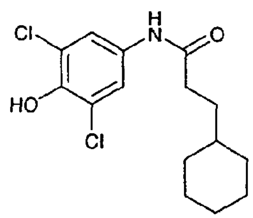 | 3-Cyclohexyl-N-(3,5-dichloro-4-hydroxy-phenyl)-propionamide | C15H19Cl2NO2 | 316.22 | 316.5 |

| | | | | | |
|---|---|---|---|---|---|
| 246 | 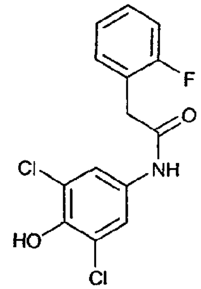 | N-(3,5-Dichloro-4-hydroxy-phenyl)-2-(2-fluoro-phenyl)-acetamide | C14H10Cl2FNO2 | 314.14 | 314.5 |
| 247 | 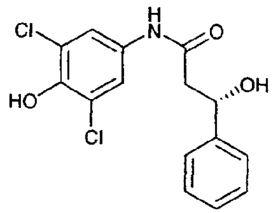 | N-(3,5-Dichloro-4-hydroxy-phenyl)-3-hydroxy-3-phenyl-propionamide | C15H13Cl2NO3 | 326.17 | 326.2 |
| 248 | 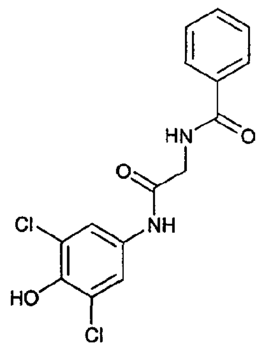 | N-[(3,5-Dichloro-4-hydroxy-phenylcarbamoyl)-methyl]-benzamide | C15H12Cl2N2O3 | 339.17 | 339.2 |
| 249 | 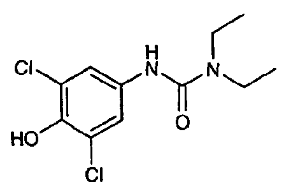 | 3-(3,5-Dichloro-4-hydroxy-phenyl)-1,1-diethyl-urea | C11H14Cl2N2O2 | 277.31 | 277.2 |

| | | | | | |
|---|---|---|---|---|---|
| 250 | 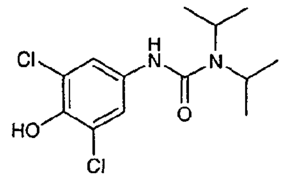 | 3-(3,5-Dichloro-4-hydroxy-phenyl)-1,1-diisopropyl-urea | C13H18Cl2N2O2 | 305.22 | 305.3 |
| 251 | 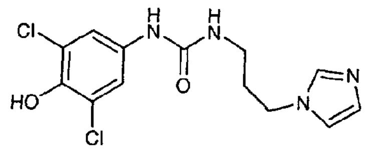 | 1-(3,5-Dichloro-4-hydroxy-phenyl)-3-(3-imidazol-1-yl-propyl)-urea | C13H14Cl2N4O2 | 329.31 | 329.1 |
| 252 | 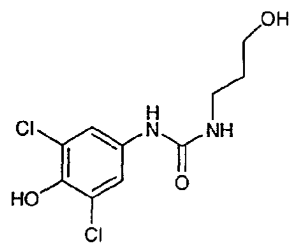 | 1-(3,5-Dichloro-4-hydroxy-phenyl)-3-(3-hydroxy-propyl)-urea | C10H12Cl2N2O3 | 278.02 | 279.1 |
| 253 | 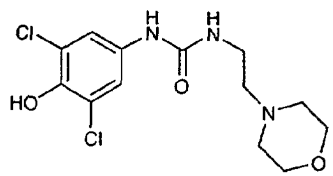 | 1-(3,5-Dichloro-4-hydroxy-phenyl)-3-(2-morpholin-4-yl-ethyl)-urea | C13H17Cl2N3O3 | 334.20 | 334.1 |
| 254 | 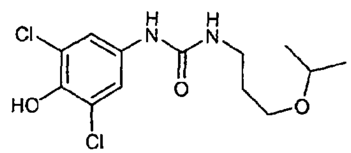 | 1-(3,5-Dichloro-4-hydroxy-phenyl)-3-(3-isopropoxy-propyl)-urea | C13H18Cl2N2O3 | 321.20 | 321.1 |

| | | | | | |
|---|---|---|---|---|---|
| 255 | 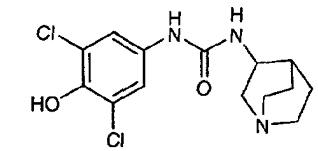 | 1-(1-Aza-bicyclo[2.2.2]oct-3-yl)-3-(3,5-dichloro-4-hydroxy-phenyl)-urea | C14H17Cl2N3O2 | 330.21 | 330.1 |
| 256 | 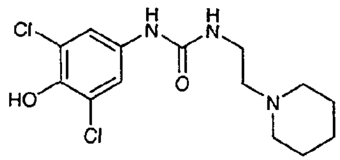 | 1-(3,5-Dichloro-4-hydroxy-phenyl)-3-(2-piperidin-1-yl-ethyl)-urea | C14H19Cl2N3O2 | 332.23 | 332.1 |
| 257 | 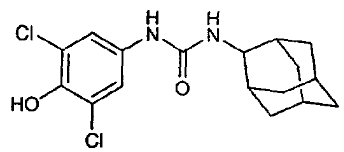 | 1-Adamantan-2-yl-3-(3,5-dichloro-4-hydroxy-phenyl)-urea | C17H20Cl2N2O2 | 355.26 | 355.1 |
| 258 | 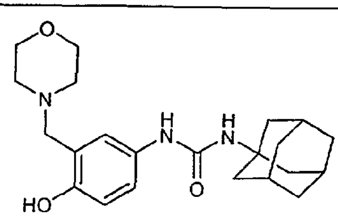 | 1-Adamantan-1-yl-3-(4-hydroxy-3-morpholin-4-ylmethyl-phenyl)-urea | C22H31N3O3 | 385.51 | 386.3 |
| 259 | 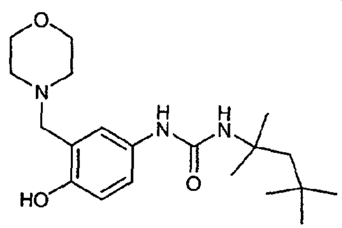 | 1-(4-Hydroxy-3-morpholin-4-ylmethyl-phenyl)-3-(1,1,3,3-tetramethyl-butyl)-urea | C20H33N3O3 | 363.49 | 364.3 |

| | | | | | |
|---|---|---|---|---|---|
| 260 | 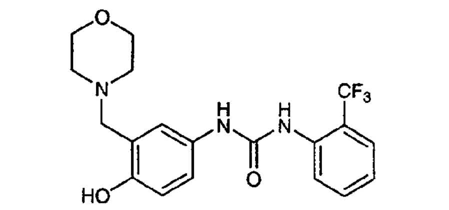 | 1-(4-Hydroxy-3-morpholin-4-ylmethyl-phenyl)-3-(2-trifluoromethyl-phenyl)-urea | C19H20F3N3O3 | 395.38 | 396.3 |
| 261 | 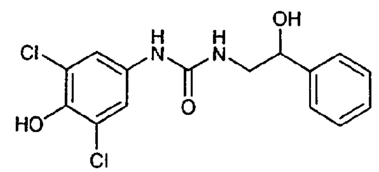 | 1-(3,5-Dichloro-4-hydroxy-phenyl)-3-(2-hydroxy-2-phenyl-ethyl)-urea | C15H14Cl2N2O3 | 341.19 | 341.1 |
| 262 | 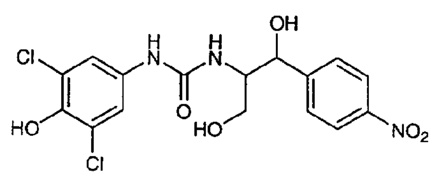 | 1-(3,5-Dichloro-4-hydroxy-phenyl)-3-[2-hydroxy-1-hydroxymethyl-2-(4-nitro-phenyl)-ethyl]-urea | C16H15Cl2N3O6 | 416.21 | 416.1 |
| 263 | 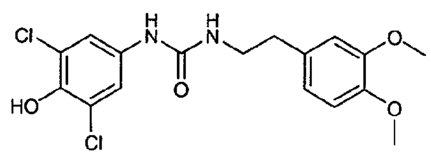 | 1-(3,5-Dichloro-4-hydroxy-phenyl)-3-[2-(3,4-dimethoxy-phenyl)-ethyl]-urea | C17H18Cl2N2O4 | 385.24 | 385.1 |
| 264 | 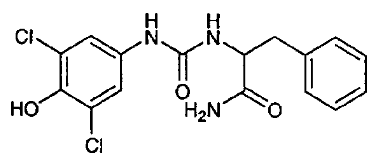 | 2-[3-(3,5-Dichloro-4-hydroxy-phenyl)-ureido]-3-phenyl-propionamide | C16H15Cl2N3O3 | 368.21 | 368.1 |

| | | | | | |
|---|---|---|---|---|---|
| 265 | 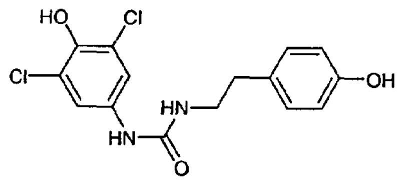 | 1-(3,5-Dichloro-4-hydroxy-phenyl)-3-[2-(4-hydroxy-phenyl)-ethyl]-urea | C15H14Cl2N2O3 | 341.28 | 341.1 |
| 266 | 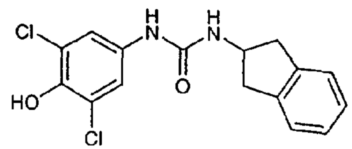 | 1-(3,5-Dichloro-4-hydroxy-phenyl)-3-indan-2-yl-urea | C16H14Cl2N2O2 | 337.20 | 337.1 |
| 267 | 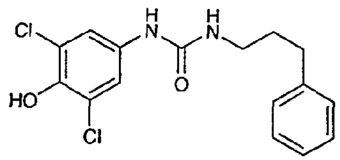 | 1-(3,5-Dichloro-4-hydroxy-phenyl)-3-(3-phenyl-propyl)-urea | C16H16Cl2N2O2 | 339.22 | 339.1 |
| 268 | 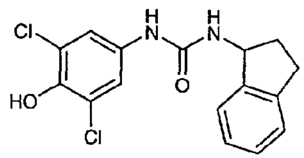 | 1-(3,5-Dichloro-4-hydroxy-phenyl)-3-indan-1-yl-urea | C16H14Cl2N2O2 | 337.20 | 337.1 |
| 269 | 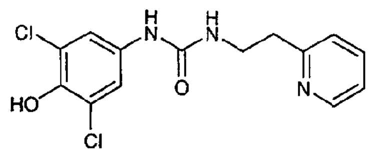 | 1-(3,5-Dichloro-4-hydroxy-phenyl)-3-(2-pyridin-2-yl-ethyl)-urea | C14H13Cl2N3O2 | 326.18 | 326.1 |
| 270 | 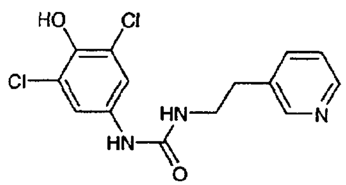 | 1-(3,5-Dichloro-4-hydroxy-phenyl)-3-(2-pyridin-3-yl-ethyl)-urea | C14H13Cl2N3O2 | 326.18 | 326.1 |

| | | | | | |
|---|---|---|---|---|---|
| 271 | 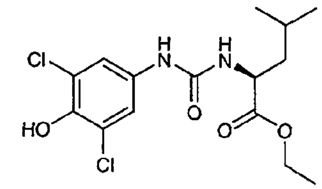 | 2-[3-(3,5-Dichloro-4-hydroxy-phenyl)-ureido]-4-methyl-pentanoic acid ethyl ester | C15H20Cl2N2O4 | 363.24 | 363.1 |
| 272 | 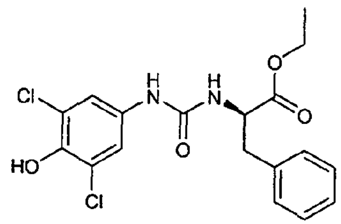 | 2-[3-(3,5-Dichloro-4-hydroxy-phenyl)-ureido]-3-phenyl-propionic acid ethyl ester | C18H18Cl2N2O4 | 397.24 | 397.1 |
| 273 | 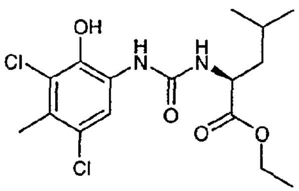 | 2-[3-(3,5-Dichloro-2-hydroxy-4-methyl-phenyl)-ureido]-4-methyl-pentanoic acid ethyl ester | C16H22Cl2N2O4 | 377.26 | 377.1 |
| 274 | 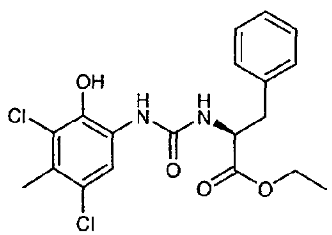 | 2-[3-(3,5-Dichloro-2-hydroxy-4-methyl-phenyl)-ureido]-3-phenyl-propionic acid ethyl ester | C19H20Cl2N2O4 | 411.28 | 411.1 |
| 275 | 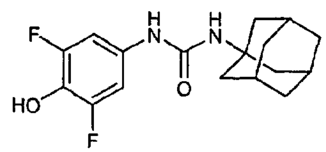 | 1-Adamantan-1-yl-3-(3,5-difluoro-4-hydroxy-phenyl)-urea | C17H20F2N2O2 | 322.25 | 323.2 |

| | | | | | |
|---|---|---|---|---|---|
| 276 | 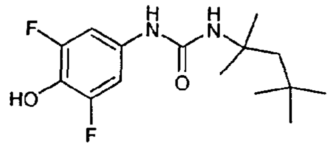 | 1-(3,5-Difluoro-4-hydroxy-phenyl)-3-(1,1,3,3-tetramethyl-butyl)-urea | C15H22F2N2O2 | 300.34 | 301.3 |
| 277 | 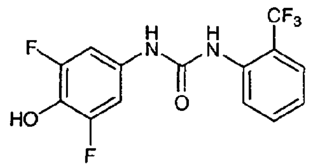 | 1-(3,5-Difluoro-4-hydroxy-phenyl)-3-(2-trifluoromethyl-phenyl)-urea | C14H9F5N2O2 | 332.23 | 333.2 |
| 278 | 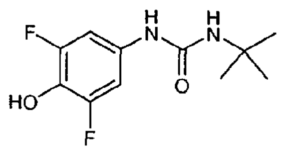 | 1-tert-Butyl-3-(3,5-difluoro-4-hydroxy-phenyl)-urea | C11H14F2N2O2 | 244.24 | 245.2 |
| 279 | 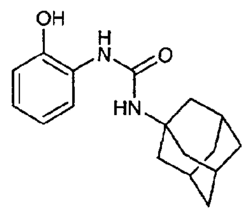 | 1-Adamantan-1-yl-3-(2-hydroxy-phenyl)-urea | C17H22N2O2 | 286.37 | 287.3 |
| 280 | 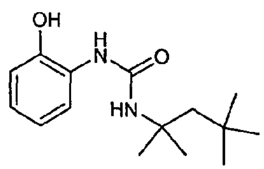 | 1-(2-Hydroxy-phenyl)-3-(1,1,3,3-tetramethyl-butyl)-urea | C15H24N2O2 | 264.36 | 265.3 |

| | | | | | |
|---|---|---|---|---|---|
| 281 | 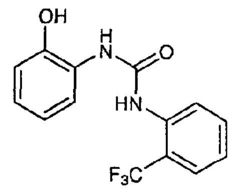 | 1-(2-Hydroxy-phenyl)-3-(2-trifluoromethyl-phenyl)-urea | C14H11F3N2O2 | 296.24 | 297.3 |
| 282 | 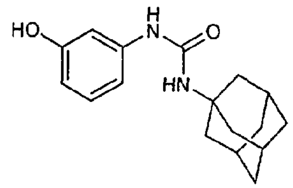 | 1-Adamantan-1-yl-3-(3-hydroxy-phenyl)-urea | C17H22N2O2 | 286.37 | 287.3 |
| 283 | 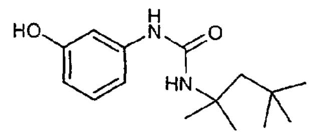 | 1-(3-Hydroxy-phenyl)-3-(1,1,3,3-tetramethyl-butyl)-urea | C15H24N2O2 | 264.36 | 265.3 |
| 284 | 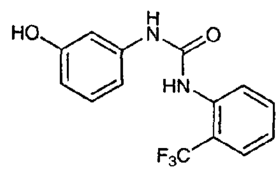 | 1-(3-Hydroxy-phenyl)-3-(2-trifluoromethyl-phenyl)-urea | C14H11F3N2O2 | 296.24 | 297.3 |
| 285 | 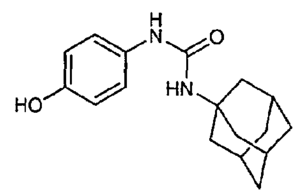 | 1-Adamantan-1-yl-3-(3-hydroxy-phenyl)-urea | C17H22N2O2 | 286.37 | 287.3 |

| | | | | | |
|---|---|---|---|---|---|
| 286 | 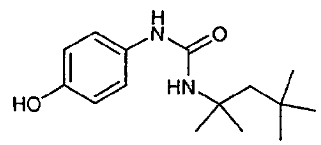 | 1-(3-Hydroxy-phenyl)-3-(1,1,3,3-tetramethyl-butyl)-urea | C15H24N2O2 | 264.36 | 265.3 |
| 287 | 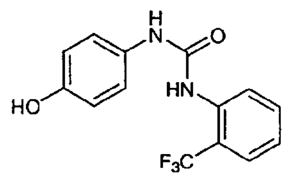 | 1-(3-Hydroxy-phenyl)-3-(2-trifluoromethyl-phenyl)-urea | C14H11F3N2O2 | 296.24 | 297.3 |
| 288 | 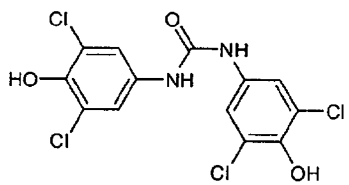 | 1,3-Bis-(3,5-dichloro-4-hydroxy-phenyl)-urea | C13H8Cl4N2O3 | 382.03 | 382.9 |
| 289 | 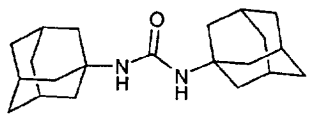 | 1,3-Di-adamantan-1-yl-urea | C21H32N2O | 328.49 | 329.3 |
| 290 | 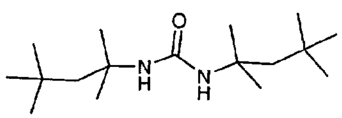 | 1,3-Bis-(1,1,3,3-tetramethyl-butyl)-urea | C17H36N2O | 284.48 | 285.3 |

| | | | | | |
|---|---|---|---|---|---|
| 291 | 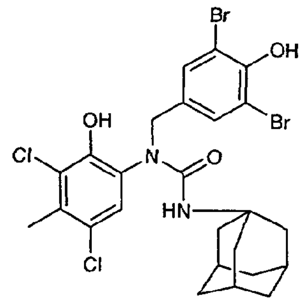 | 3-Adamantan-1-yl-1-(3,5-dibromo-4-hydroxy-benzyl)-1-(3,5-dichloro-2-hydroxy-4-methyl-phenyl)-urea | C25H26Br2Cl2N2O3 | 633.20 | 633.0 |
| 292 | 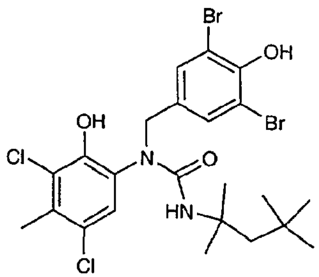 | 1-(3,5-Dibromo-4-hydroxy-benzyl)-1-(3,5-dichloro-2-hydroxy-4-methyl-phenyl)-3-(1,1,3,3-tetramethyl-butyl)-urea | C23H28Br2Cl2N2O3 | 611.19 | 610.3 |
| 293 | 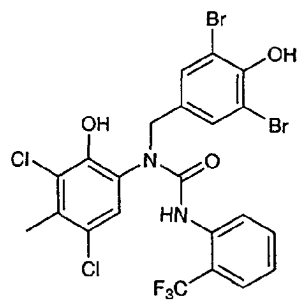 | 1-(3,5-Dibromo-4-hydroxy-benzyl)-1-(3,5-dichloro-2-hydroxy-4-methyl-phenyl)-3-(2-trifluoromethyl-phenyl)-urea | C22H15Br2Cl2F3N2O3 | 643.08 | 644.8 |

| | | | | | |
|---|---|---|---|---|---|
| 294 | 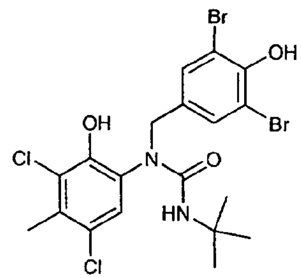 | 3-tert-Butyl-1-(3,5-dibromo-4-hydroxy-benzyl)-1-(3,5-dichloro-2-hydroxy-4-methyl-phenyl)-urea | C19H20Br2Cl2N2O3 | 555.05 | 554.9 |
| 295 | 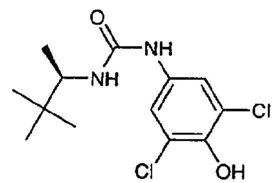 | 1-(3,5-Dichloro-4-hydroxy-phenyl)-3-(1,2,2-trimethyl-propyl)-urea | C13H18Cl2N2O2 | 305.20 | 305.1 |
| 296 | 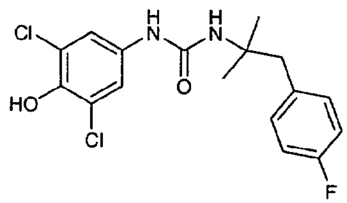 | 1-(3,5-Dichloro-4-hydroxy-phenyl)-3-[2-(4-fluoro-phenyl)-1,1-dimethyl-ethyl]-urea | C17H17Cl2FN2O2 | 371.23 | 371.1 |
| 297 | 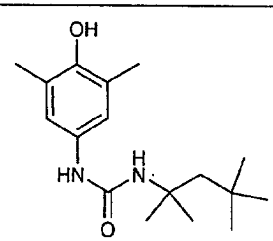 | 1-(4-Hydroxy-3,5-dimethyl-phenyl)-3-(1,1,3,3-tetramethyl-butyl)-urea | C17H28N2O2 | 292.42 | 293.3 |

| | | | | | |
|---|---|---|---|---|---|
| 298 | 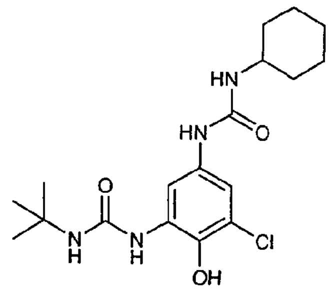 | 1-tert-Butyl-3-[3-chloro-5-(3-cyclohexyl-ureido)-2-hydroxy-phenyl]-urea | C18H27ClN4O3 | 382.88 | 383.4 |
| 299 | 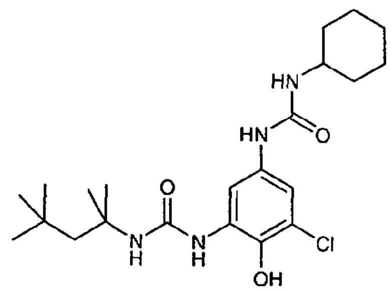 | 1-[3-Chloro-5-(3-cyclohexyl-ureido)-2-hydroxy-phenyl]-3-(1,1,3,3-tetramethyl-butyl)-urea | C22H35ClN4O3 | 438.99 | 439.8 |
| 300 | 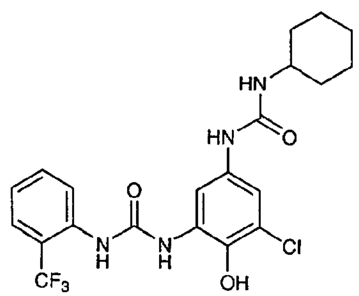 | 1-[3-Chloro-5-(3-cyclohexyl-ureido)-2-hydroxy-phenyl]-3-(2-trifluoromethyl-phenyl)-urea | C21H22ClF3N4O3 | 470.87 | 471.7 |

| | | | | | |
|---|---|---|---|---|---|
| 301 | 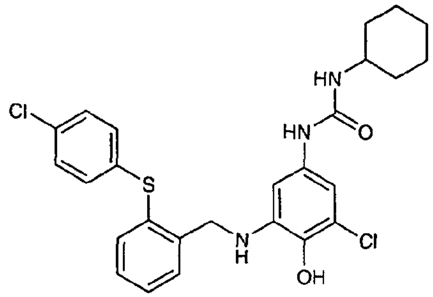 | 1-{3-Chloro-5-[2-(4-chloro-phenylsulfanyl)-benzylamino]-4-hydroxy-phenyl}-3-cyclohexyl-urea | C26H27Cl2N3O2S | 516.48 | 517.4 |
| 302 | 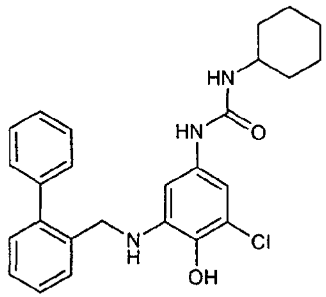 | 1-{3-[(Biphenyl-2-ylmethyl)-amino]-5-chloro-4-hydroxy-phenyl}-3-cyclohexyl-urea | C26H28ClN3O2 | 449.97 | 450.9 |
| 303 | 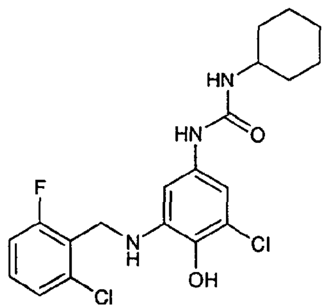 | 1-[3-Chloro-5-(2-chloro-6-fluoro-benzylamino)-4-hydroxy-phenyl]-3-cyclohexyl-urea | C20H22Cl2FN3O2 | 426.31 | 426.4 |

| | | | | | |
|---|---|---|---|---|---|
| 304 | 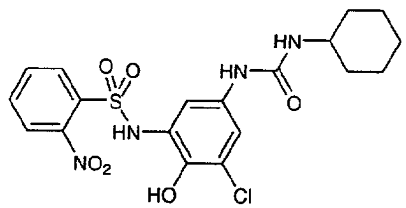 | N-[3-Chloro-5-(3-cyclohexyl-ureido)-2-hydroxy-phenyl]-2-nitro-benzenesulfonamide | C19H21ClN4O6S | 468.91 | 469.8 |
| 305 | 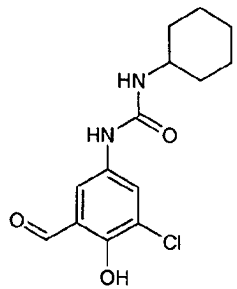 | 1-(3-Chloro-5-formyl-4-hydroxy-phenyl)-3-cyclohexyl-urea | C14H17ClN2O3 | 296.75 | 296.8 |
| 306 | 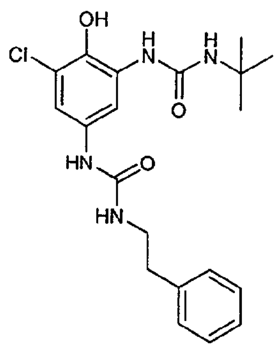 | 1-tert-Butyl-3-[3-chloro-2-hydroxy-5-(3-phenethyl-ureido)-phenyl]-urea | C20H25ClN4O3 | 404.89 | 404.9 |

| | | | | | |
|---|---|---|---|---|---|
| 307 | 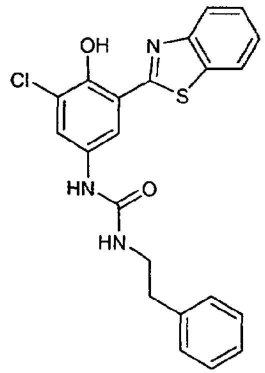 | 1-(3-Benzothiazol-2-yl-5-chloro-4-hydroxy-phenyl)-3-phenethyl-urea | C22H18ClN3O2S | 423.92 | 423.9 |
| 308 | 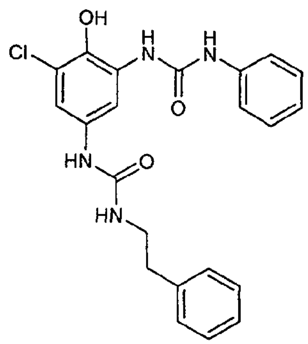 | 1-[3-Chloro-2-hydroxy-5-(3-phenethyl-ureido)-phenyl]-3-phenyl-urea | C22H21ClN4O3 | 424.88 | 424.9 |
| 309 | 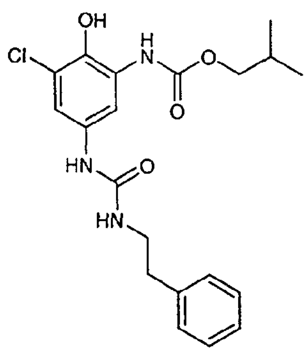 | [3-Chloro-2-hydroxy-5-(3-phenethyl-ureido)-phenyl]-carbamic acid isobutyl ester | C20H24ClN3O4 | 405.88 | 406.8 |

| | | | | | |
|---|---|---|---|---|---|
| 310 | 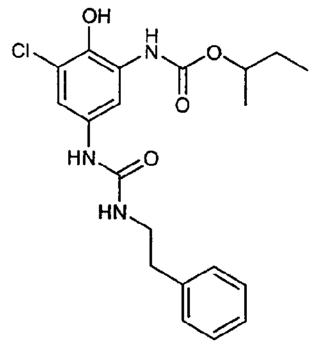 | [3-Chloro-2-hydroxy-5-(3-phenethyl-ureido)-phenyl]-carbamic acid sec-butyl ester | C20H24ClN3O4 | 405.88 | 406.8 |
| 311 | 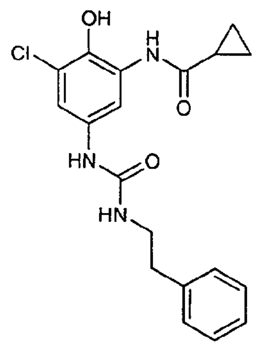 | Cyclopropanecarboxylic acid [3-chloro-2-hydroxy-5-(3-phenethyl-ureido)-phenyl]-amide | C19H20ClN3O3 | 373.83 | 373.8 |
| 312 | 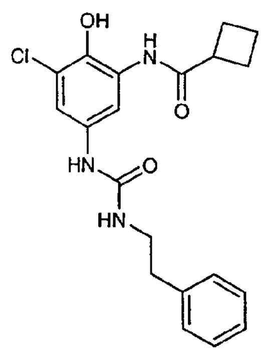 | Cyclobutanecarboxylic acid [3-chloro-2-hydroxy-5-(3-phenethyl-ureido)-phenyl]-amide | C20H22ClN3O3 | 387.86 | 388.8 |

| | | | | | |
|---|---|---|---|---|---|
| 313 | 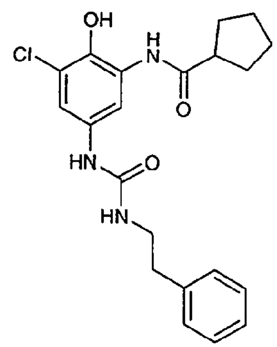 | Cyclopentanecarboxylic acid [3-chloro-2-hydroxy-5-(3-phenethyl-ureido)-phenyl]-amide | C21H24ClN3O3 | 401.89 | 401.9 |
| 314 | 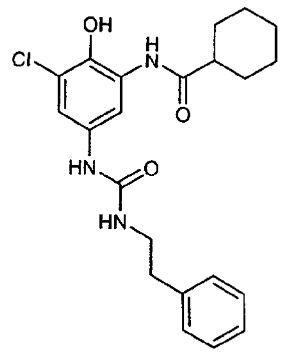 | Cyclohexanecarboxylic acid [3-chloro-2-hydroxy-5-(3-phenethyl-ureido)-phenyl]-amide | C22H26ClN3O3 | 415.92 | 415.9 |
| 315 | 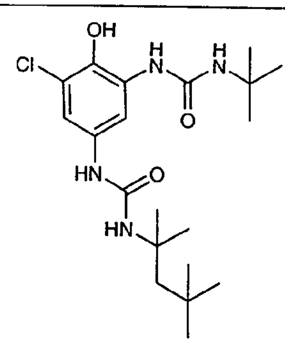 | 1-tert-Butyl-3-{3-chloro-2-hydroxy-5-[3-(1,1,3,3-tetramethyl-butyl)-ureido]-phenyl}-urea | C20H33ClN4O3 | 412.95 | 413.8 |

| | | | | | |
|---|---|---|---|---|---|
| 316 | 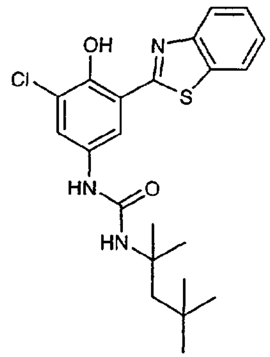 | 1-(3-Benzothiazol-2-yl-5-chloro-4-hydroxy-phenyl)-3-(1,1,3,3-tetramethyl-butyl)-urea | C22H26ClN3O2S | 431.98 | 432.8 |
| 317 | 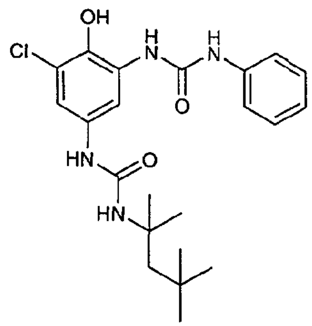 | 1-{3-Chloro-2-hydroxy-5-[3-(1,1,3,3-tetramethyl-butyl)-ureido]-phenyl}-3-phenyl-urea | C22H29ClN4O3 | 432.94 | 432.8 |
| 318 | 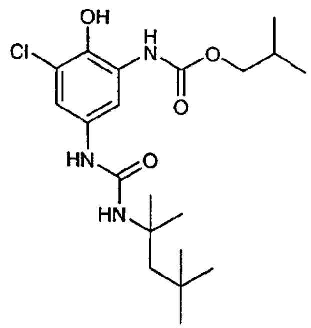 | {3-Chloro-2-hydroxy-5-[3-(1,1,3,3-tetramethyl-butyl)-ureido]-phenyl}-carbamic acid isobutyl ester | C20H32ClN3O4 | 413.94 | 413.8 |

| | | | | | |
|---|---|---|---|---|---|
| 319 | 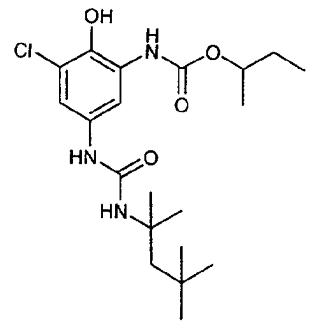 | {3-Chloro-2-hydroxy-5-[3-(1,1,3,3-tetramethyl-butyl)-ureido]-phenyl}-carbamic acid sec-butyl ester | C20H32ClN3O4 | 413.94 | 414.1 |
| 320 | 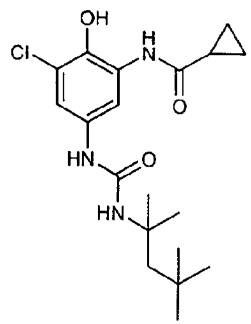 | Cyclopropanecarboxylic acid {3-chloro-2-hydroxy-5-[3-(1,1,3,3-tetramethyl-butyl)-ureido]-phenyl}-amide | C19H28ClN3O3 | 381.90 | 381.9 |
| 321 | 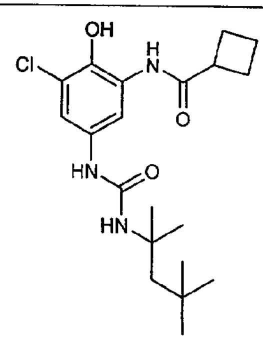 | Cyclobutanecarboxylic acid {3-chloro-2-hydroxy-5-[3-(1,1,3,3-tetramethyl-butyl)-ureido]-phenyl}-amide | C20H30ClN3O3 | 395.92 | 395.9 |

| | | | | | |
|---|---|---|---|---|---|
| 322 | 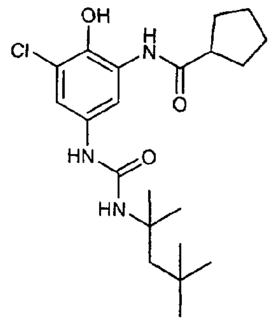 | Cyclopentanecarboxylic acid {3-chloro-2-hydroxy-5-[3-(1,1,3,3-tetramethyl-butyl)-ureido]-phenyl}-amide | C21H32ClN3O3 | 409.95 | 410.1 |
| 323 | 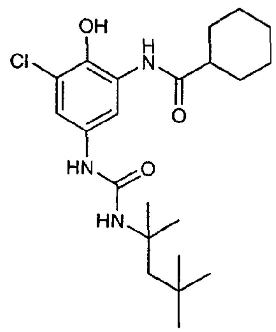 | Cyclohexanecarboxylic acid {3-chloro-2-hydroxy-5-[3-(1,1,3,3-tetramethyl-butyl)-ureido]-phenyl}-amide | C22H34ClN3O3 | 423.98 | 424.0 |
| 324 | 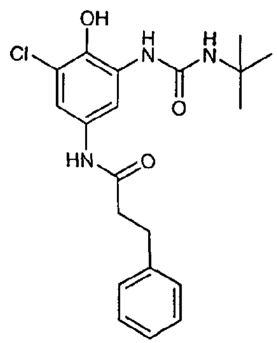 | N-[3-(3-tert-Butyl-ureido)-5-chloro-4-hydroxy-phenyl]-3-phenyl-propionamide | C20H24ClN3O3 | 389.88 | 389.9 |

| | | | | | |
|---|---|---|---|---|---|
| 325 | 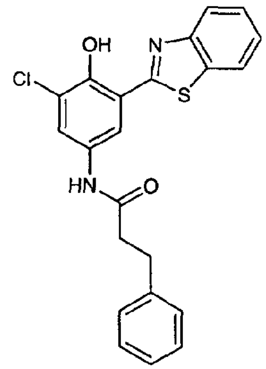 | N-(3-Benzothiazol-2-yl-5-chloro-4-hydroxy-phenyl)-3-phenyl-propionamide | C22H17ClN2O2S | 408.90 | 408.9 |
| 326 | 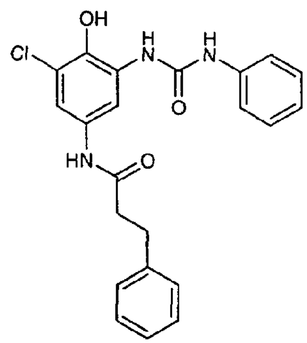 | N-[3-Chloro-4-hydroxy-5-(3-phenyl-ureido)-phenyl]-3-phenyl-propionamide | C22H20ClN3O3 | 409.87 | 410.1 |
| 327 | 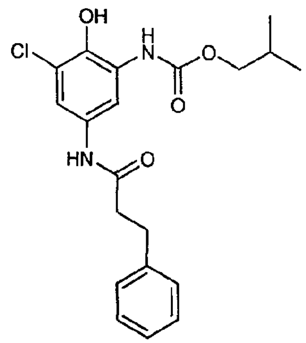 | [3-Chloro-2-hydroxy-5-(3-phenyl-propionylamino)-phenyl]-carbamic acid isobutyl ester | C20H23ClN2O4 | 390.86 | 390.8 |

| | | | | | |
|---|---|---|---|---|---|
| 328 | 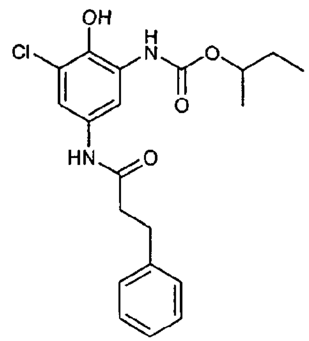 | [3-Chloro-2-hydroxy-5-(3-phenyl-propionylamino)-phenyl]-carbamic acid sec-butyl ester | C20H23ClN2O4 | 390.86 | 390.8 |
| 329 | 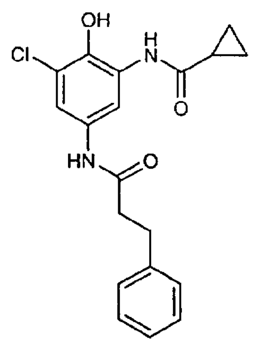 | Cyclopropanecarboxylic acid [3-chloro-2-hydroxy-5-(3-phenyl-propionylamino)-phenyl]-amide | C19H19ClN2O3 | 358.82 | 358.9 |
| 330 | 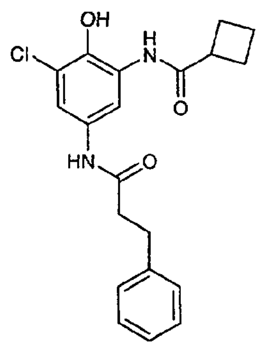 | Cyclobutanecarboxylic acid [3-chloro-2-hydroxy-5-(3-phenyl-propionylamino)-phenyl]-amide | C20H21ClN2O3 | 372.85 | 373.8 |

| | | | | | |
|---|---|---|---|---|---|
| 331 | 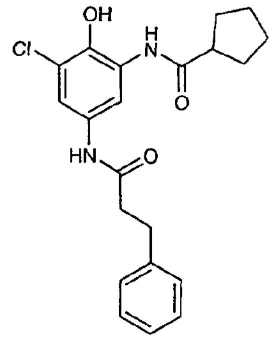 | Cyclopentanecarboxylic acid [3-chloro-2-hydroxy-5-(3-phenyl-propionylamino)-phenyl]-amide | C21H23ClN2O3 | 386.87 | 386.8 |
| 332 | 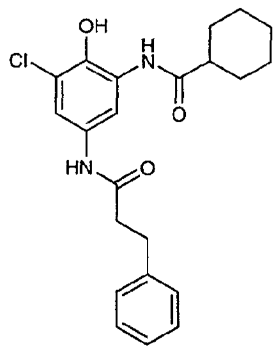 | Cyclohexanecarboxylic acid [3-chloro-2-hydroxy-5-(3-phenyl-propionylamino)-phenyl]-amide | C22H24ClN2O3 | 400.90 | 401.8 |
| 333 | 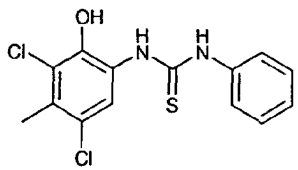 | 1-(3,5-Dichloro-2-hydroxy-4-methyl-phenyl)-3-phenyl-thiourea | C14H12Cl2N2OS | 326.0 | 327.1 |
| 334 | 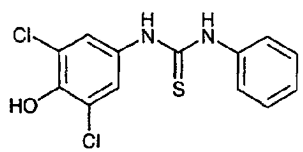 | 1-(3,5-Dichloro-4-hydroxy-phenyl)-3-phenyl-thiourea | C13H10Cl2N2OS | 312.0 | 313.0 |

| | | | | | |
|---|---|---|---|---|---|
| 335 | 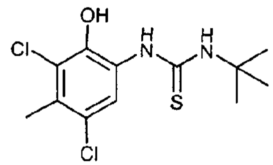 | 1-tert-Butyl-3-(3,5-dichloro-2-hydroxy-4-methyl-phenyl)-thiourea | C12H16Cl2N2OS | 306.0 | 307.1 |
| 336 | 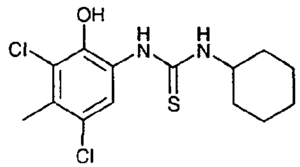 | 1-Cyclohexyl-3-(3,5-dichloro-2-hydroxy-4-methyl-phenyl)-thiourea | C14H18Cl2N2OS | 332.0 | 333.0 |
| 337 | 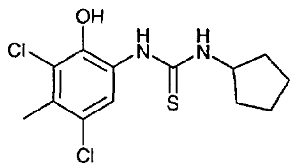 | 1-Cyclopentyl-3-(3,5-dichloro-2-hydroxy-4-methyl-phenyl)-thiourea | C13H16Cl2N2OS | 318.0 | 319.0 |
| 338 | 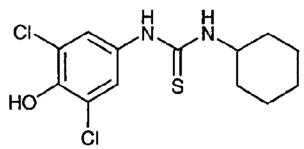 | 1-Cyclohexyl-3-(3,5-dichloro-4-hydroxy-phenyl)-thiourea | C13H16Cl2N2OS | 318.0 | 319.1 |
| 339 | 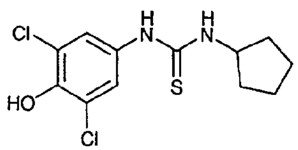 | 1-Cyclopentyl-3-(3,5-dichloro-4-hydroxy-phenyl)-thiourea | C12H14Cl2N2OS | 304.0 | 305.0 |
| 340 | 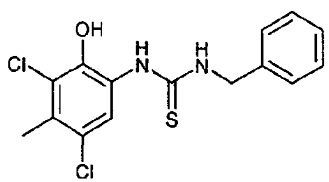 | 1-Benzyl-3-(3,5-dichloro-2-hydroxy-4-methyl-phenyl)-thiourea | C15H14Cl2N2OS | 340.0 | 341.1 |

| | | | | | |
|---|---|---|---|---|---|
| 341 | 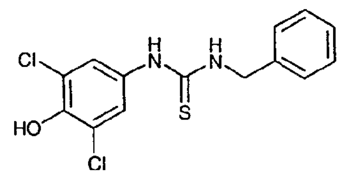 | 1-Benzyl-3-(3,5-dichloro-4-hydroxy-phenyl)-thiourea | C14H12Cl2N2OS | 326.0 | 327.0 |
| 342 | 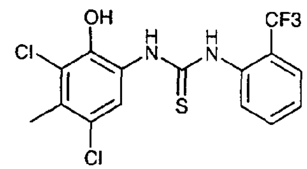 | 1-(3,5-Dichloro-2-hydroxy-4-methyl-phenyl)-3-(2-trifluoromethyl-phenyl)-thiourea | C15H11Cl2F3N2OS | 394.0 | 395.1 |
| 343 | 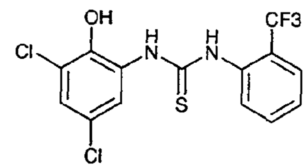 | 1-(3,5-Dichloro-2-hydroxy-phenyl)-3-(2-trifluoromethyl-phenyl)-thiourea | C14H9Cl2F3N2OS | 380.0 | 381.0 |
| 344 | 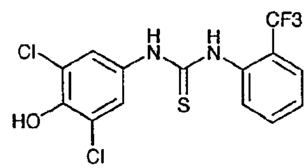 | 1-(3,5-Dichloro-4-hydroxy-phenyl)-3-(2-trifluoromethyl-phenyl)-thiourea | C14H9Cl2F3N2OS | 380.0 | 381.0 |
| 345 | 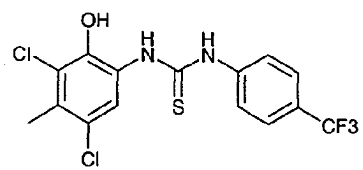 | 1-(3,5-Dichloro-2-hydroxy-4-methyl-phenyl)-3-(4-trifluoromethyl-phenyl)-thiourea | C15H11Cl2F3N2OS | 394.0 | 395.1 |

| | | | | | |
|---|---|---|---|---|---|
| 346 | 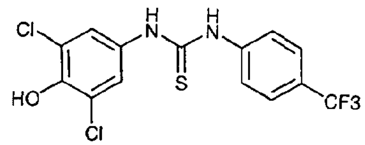 | 1-(3,5-Dichloro-4-hydroxy-phenyl)-3-(4-trifluoromethyl-phenyl)-thiourea | C14H9Cl2F3N2OS | 380.0 | 381.0 |
| 347 | 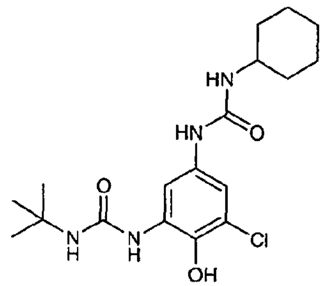 | 1-tert-Butyl-3-[3-chloro-5-(3-cyclohexyl-ureido)-2-hydroxy-phenyl]-urea | C18H27ClN4O3 | 382.9 | 383.0 |
| 348 | 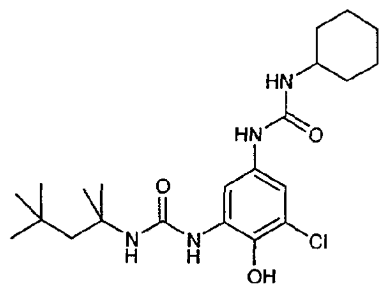 | 1-[3-Chloro-5-(3-cyclohexyl-ureido)-2-hydroxy-phenyl]-3-(1,1,3,3-tetramethyl-butyl)-urea | C22H35ClN4O3 | 439.0 | 439.1 |
| 349 | 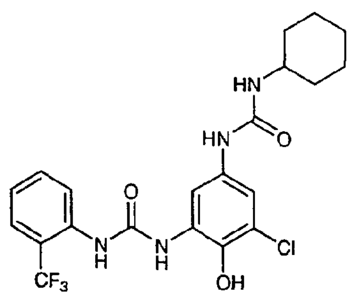 | 1-[3-Chloro-5-(3-cyclohexyl-ureido)-2-hydroxy-phenyl]-3-(2-trifluoromethyl-phenyl)-urea | C21H22ClF3N4O3 | 470.9 | 471.1 |

| | | | | | |
|---|---|---|---|---|---|
| 350 | 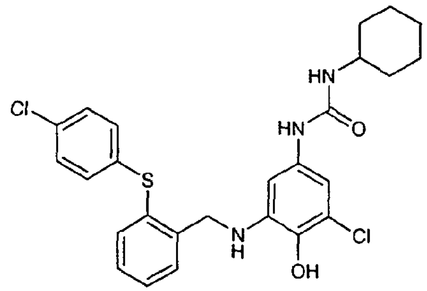 | 1-{3-Chloro-5-[2-(4-chloro-phenylsulfanyl)-benzylamino]-4-hydroxy-phenyl}-3-cyclohexyl-urea | C26H27Cl2N3O2S | 516.5 | 516.1 |
| 351 | 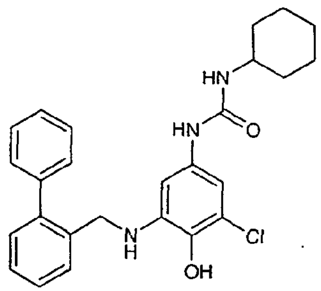 | 1-{3-[(Biphenyl-2-ylmethyl)-amino]-5-chloro-4-hydroxy-phenyl}-3-cyclohexyl-urea | C26H28ClN3O2 | 450.0 | 450.1 |
| 352 | 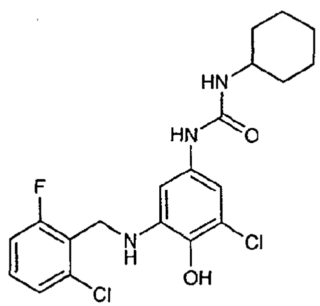 | 1-[3-Chloro-5-(2-chloro-6-fluoro-benzylamino)-4-hydroxy-phenyl]-3-cyclohexyl-urea | C20H22Cl2FN3O2 | 426.3 | 426.2 |

| | | | | | |
|---|---|---|---|---|---|
| 353 | 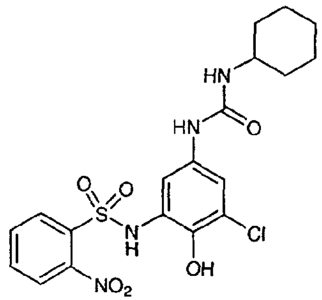 | N-[3-Chloro-5-(3-cyclohexyl-ureido)-2-hydroxy-phenyl]-2-nitro-benzenesulfonamide | C19H21ClN4O6S | 468.9 | 469.1 |
| 354 | 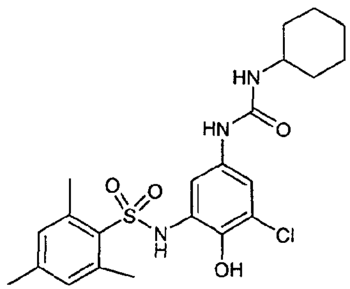 | N-[3-Chloro-5-(3-cyclohexyl-ureido)-2-hydroxy-phenyl]-2,4,6-trimethyl-benzenesulfonamide | C22H28ClN3O4S | 466.0 | 466.2 |
| 355 | 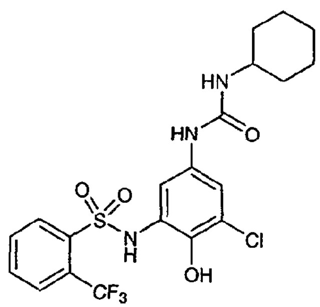 | N-[3-Chloro-5-(3-cyclohexyl-ureido)-2-hydroxy-phenyl]-2-trifluoromethyl-benzenesulfonamide | C20H21ClF3N3O4S | 491.9 | 492.2 |

| | | | | | |
|---|---|---|---|---|---|
| 356 | 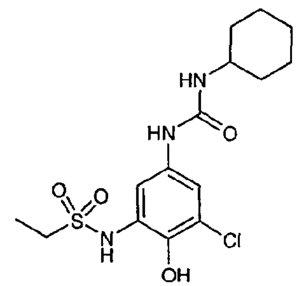 | Ethanesulfonic acid [3-chloro-5-(3-cyclohexyl-ureido)-2-hydroxy-phenyl]-amide | C15H22ClN3O4S | 375.9 | 376.1 |
| 357 | 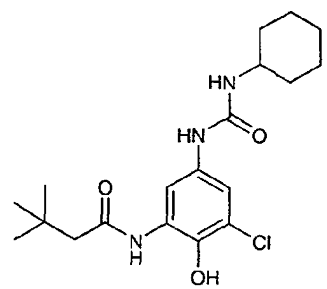 | N-[3-Chloro-5-(3-cyclohexyl-ureido)-2-hydroxy-phenyl]-3,3-dimethyl-butyramide | C19H28ClN3O3 | 381.90 | 382.2 |
| 358 | 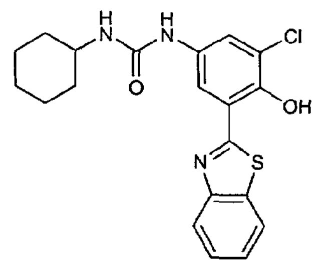 | 1-(3-Benzothiazol-2-yl-5-chloro-4-hydroxy-phenyl)-3-cyclohexyl-urea | C20H20ClN3O2S | 401.9 | 402.2 |

| | | | | | |
|---|---|---|---|---|---|
| 359 | 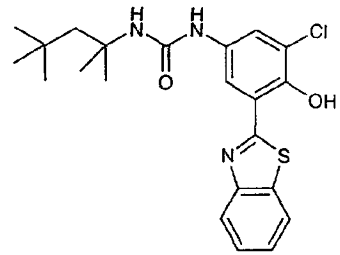 | 1-(3-Benzothiazol-2-yl-5-chloro-4-hydroxy-phenyl)-3-(1,1,3,3-tetramethyl-butyl)-urea | C22H26ClN3O2S | 432.0 | 432.3 |
| 360 | 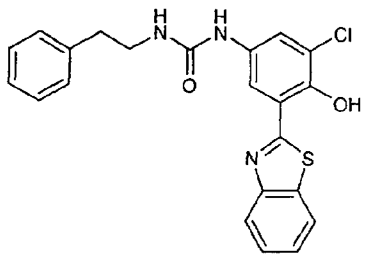 | 1-(3-Benzothiazol-2-yl-5-chloro-4-hydroxy-phenyl)-3-phenethyl-urea | C22H18ClN3O2S | 423.9 | 424.2 |
| 361 | 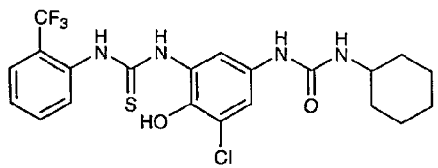 | 1-{3-Chloro-4-hydroxy-5-[3-(2-trifluoromethyl-phenyl)-thioureido]-phenyl}-3-cyclohexyl-urea | C21H22ClF3N4O2S | 486.9 | 487.1 |
| 362 | 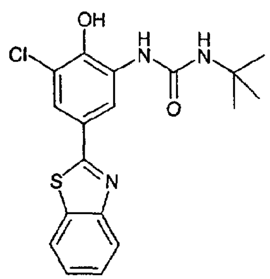 | 1-(5-Benzothiazol-2-yl-3-chloro-2-hydroxy-phenyl)-3-tert-butyl-urea | C18H18ClN3O2S | 375.9 | 376.1 |

| | | | | | |
|---|---|---|---|---|---|
| 363 | 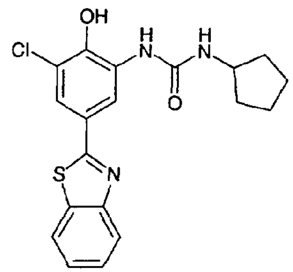 | 1-(5-Benzothiazol-2-yl-3-chloro-2-hydroxy-phenyl)-3-cyclopentyl-urea | C19H18ClN3O2S | 387.9 | 388.1 |
| 364 | 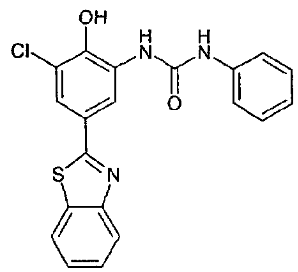 | 1-(5-Benzothiazol-2-yl-3-chloro-2-hydroxy-phenyl)-3-phenyl-urea | C20H14ClN3O2S | 395.9 | 396.2 |
| 365 | 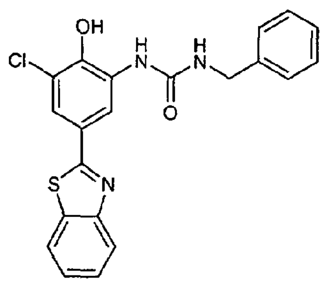 | 1-(5-Benzothiazol-2-yl-3-chloro-2-hydroxy-phenyl)-3-benzyl-urea | C21H16ClN3O2S | 409.9 | 410.1 |

| | | | | | |
|---|---|---|---|---|---|
| 366 | 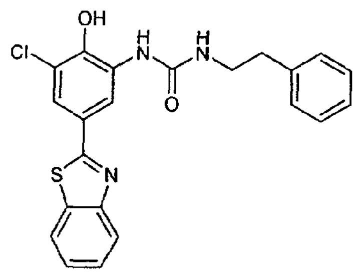 | 1-(5-Benzothiazol-2-yl-3-chloro-2-hydroxy-phenyl)-3-phenethyl-urea | C22H18ClN3O2S | 423.9 | 424.1 |
| 367 | 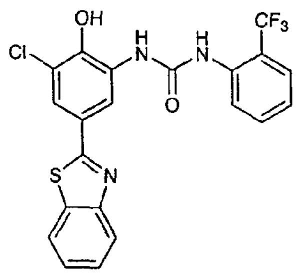 | 1-(5-Benzothiazol-2-yl-3-chloro-2-hydroxy-phenyl)-3-(2-trifluoromethyl-phenyl)-urea | C21H13ClF3N3O2S | 463.9 | 464.1 |
| 368 | 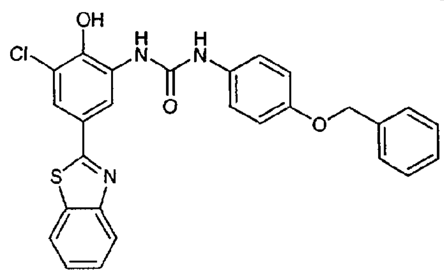 | 1-(5-Benzothiazol-2-yl-3-chloro-2-hydroxy-phenyl)-3-(4-benzyloxy-phenyl)-urea | C27H20ClN3O3S | 502.0 | 502.1 |

| | | | | | |
|---|---|---|---|---|---|
| 369 | 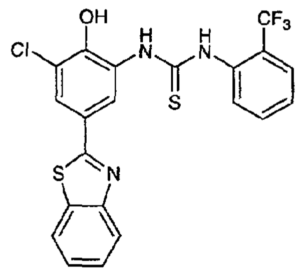 | 1-(5-Benzothiazol-2-yl-3-chloro-2-hydroxy-phenyl)-3-(2-trifluoromethyl-phenyl)-thiourea | C21H13ClF3N3OS2 | 479.9 | 480.0 |
| 370 | 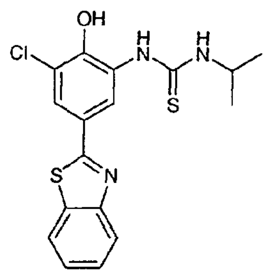 | 1-(5-Benzothiazol-2-yl-3-chloro-2-hydroxy-phenyl)-3-isopropyl-thiourea | C17H16ClN3OS2 | 377.9 | 378.1 |
| 371 | 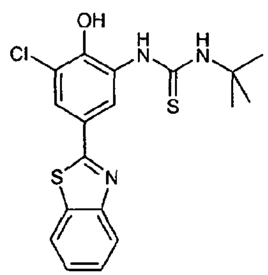 | 1-(5-Benzothiazol-2-yl-3-chloro-2-hydroxy-phenyl)-3-tert-butyl-thiourea | C18H18ClN3OS2 | 391.9 | 392.1 |

| | | | | | |
|---|---|---|---|---|---|
| 372 | 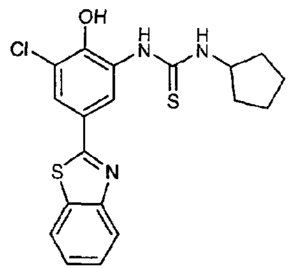 | 1-(5-Benzothiazol-2-yl-3-chloro-2-hydroxy-phenyl)-3-cyclopentyl-thiourea | C19H18ClN3OS2 | 403.9 | 404.1 |
| 373 | 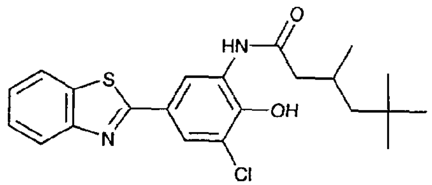 | 3,5,5-Trimethyl-hexanoic acid (5-benzothiazol-2-yl-3-chloro-2-hydroxy-phenyl)-amide | C22H25ClN2O2S | 417.0 | 417.2 |
| 374 | 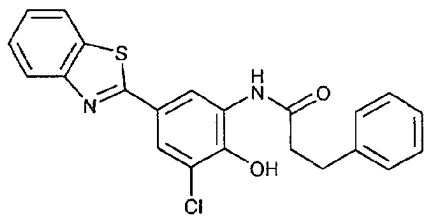 | N-(5-Benzothiazol-2-yl-3-chloro-2-hydroxy-phenyl)-3-phenyl-propionamide | C22H17ClN2O2S | 408.9 | 409.2 |
| 375 | 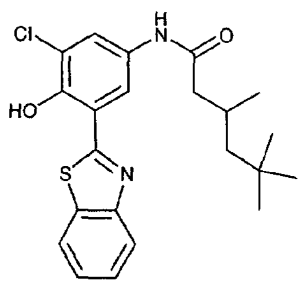 | 3,5,5-Trimethyl-hexanoic acid (3-benzothiazol-2-yl-5-chloro-4-hydroxy-phenyl)-amid | C22H25ClN2O2S | 417.0 | 417.7 |

| | | | | | |
|---|---|---|---|---|---|
| 376 | 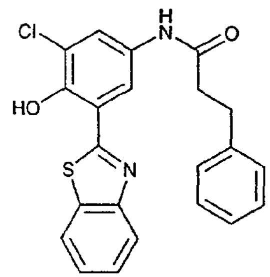 | N-(3-Benzothiazol-2-yl-5-chloro-4-hydroxy-phenyl)-3-phenyl-propionamide | C22H17ClN2O2S | 408.9 | 409.2 |
| 377 | 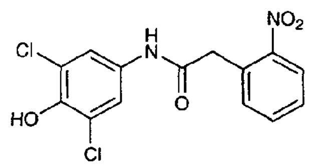 | N-(3,5-Dichloro-4-hydroxy-phenyl)-2-(2-nitro-phenyl)-acetamide | C14H10Cl2N2O4 | 341.15 | 341.5 |
| 378 | 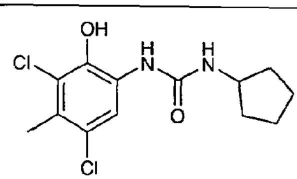 | 1-Cyclopentyl-3-(3,5-dichloro-2-hydroxy-4-methyl-phenyl)-urea | C13H16Cl2N2O2 | 303.18 | 303.5 |
| 379 | 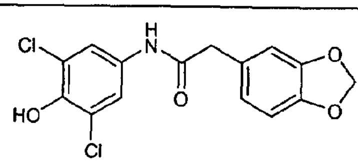 | 2-Benzo[1,3]dioxol-5-yl-N-(3,5-dichloro-4-hydroxy-phenyl)-acetamide | C15H11Cl2NO4 | 340.16 | 340.8 |

or a pharmaceutical acceptable salt or prodrug thereof.

Even more preferred compounds according to the present invention are those mentioned in any of the tables herein and those further disclosed and/or characterized in the examples.

As used herein, each of the following terms, used alone or in conjunction with other terms, are defined as follows (except where noted to the contrary):

The term "alkyl" refers to a saturated aliphatic radical containing from one to ten carbon atoms or a mono- or polyunsaturated aliphatic hydrocarbon radical containing from two to twelve carbon atoms, containing at least one double and triple bound, respectively. "Alkyl" refers to both branched and unbranched alkyl groups. Preferred alkyl groups are straight chain alkyl groups containing from one to eight carbon atoms. More preferred alkyl groups are straight chain alkyl groups containing from one to six carbon atoms and branched alkyl groups containing from three to six carbon atoms. It should be understood that any combination term using an "alk" or "alkyl" prefix refers to analogs according to the above definition of "alkyl". For example, terms such as "alkoxy", "alkylthio" refer to alkyl group linked to a second group via an oxygen or sulfur atom. "Alkanoyl" refers to an alkyl group linked to a carbonyl group (C=O).

The term "cycloalkyl" refers to the cyclic analog of an alkyl group, as defined above, optionally unsaturated and/or substituted. Preferred cycloalkyl groups are saturated cycloalkyl groups, more particularly those containing from three to eight carbon atoms, and even more preferably three to six carbon atoms.

The term "aryl" refers to aromatic groups having in the range of 6 to 14 carbon atoms and "substituted aryl" refers to aryl groups further bearing one or more substituents.

Each of the above defined "alkyl", "cycloalkyl", and "aryl" shall be understood to include their halogenated analogs, whereby the halogenated analogs may comprise one or several halogen atoms. The halogenated analogs thus comprise any halogen radical as defined in the following.

The term "halo" refers to a halogen radical selected from fluoro, chloro, bromo, iodo. Preferred halo groups are fluoro, chloro and bromo.

The term "heteroaryl" refers to a stable 5 to 8 membered, preferably 5 or 6 membered monocyclic or 8 to 11 membered bicyclic aromatic heterocycle radical. Each heterocycle consists of carbon atoms and from 1 to 4 heteroatoms selected from the group consisting of nitrogen, oxygen, sulfur. The heterocycle may be attached by any atom of the cycle which results in the creation of a stable structure. Preferred heteroaryl radicals as used herein include, for example, furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzthiazolyl, benzoxazolyl, purinyl, quinolizinyl, quinolinyl, isoquinolinyl, cinnolinyl, phthalazinyl, quinazolinyl, quinoxalinyl, naphthridinyl, pteridinyl, carbazolyl, acridinyl, phenazinyl, phenothiazinyl and phenoxazinyl.

The term "heterocyclyl" refers to a stable 5 to 8 membered, preferably 5 or 6 membered monocyclic or 8 to 11 membered bicyclic heterocycle radical which may be either saturated or unsaturated, and is non-aromatic. Each heterocycle consists of carbon atom(s) and from 1 to 4 heteroatoms selected from the group consisting of nitrogen, oxygen and sulfur. The heterocycle may be attached by any atom of the cycle, which results in the creation of a stable structure. Preferred heterocycle radicals as used herein include, for example, pyrrolinyl, pyrrolidinyl, pyrazolinyl, pyrazolidinyl, piperidinyl, morpholinyl, thiomorpholinyl, pyranyl, thiopyranyl, piperazinyl, indolinyl, azetidinyl, tetrahydropyranyl, tetrahydrothiopyranyl, tetrahydrofuranyl, hexahydropyrimidinyl, hexahydropyridazinyl, 1,4,5,6-tetrahydropyrimidin-2-ylamine, dihydro-oxazolyl, 1,2-thiazinanyl-1,1-dioxide, 1,2,6-thiadiazinanyl-1,1-dioxide, isothiazolidinyl-1,1-dioxide and imidazolidinyl-2,4-dione.

The terms "heterocyclyl", "heteroaryl" and "aryl", when associated with another moiety, unless otherwise specified, shall have the same meaning as given above. For example, "aroyl" refers to phenyl or naphthyl linked to a carbonyl group (C=O).

Each aryl or heteroaryl unless otherwise specified includes its partially or fully hydrogenated derivative. For example, quinolinyl may include decahydroquinolinyl and tetrahydroquinolinyl, naphthyl may include its hydrogenated derivatives such as tetrahydranaphthyl.

As used herein above and throughout this application, "nitrogen" and "sulfur" include any oxidized form of nitrogen and sulfur and the quaternized form of any basic nitrogen such as sulfoxide, sulfone, nitrone, N-oxide.

As used herein a wording defining the limits of a range of length such as e. g. "1 to 5" means any integer from 1 to 5, i. e. 1, 2, 3, 4 and 5. In other words, any range defined by two integers explicitly mentioned is meant to comprise any integer defining said limits and any integer comprised in said range.

As used herein the term substituted shall mean that one or more H atom of the group or compound which is substituted, is replaced by a different atom, a group of atoms, a molecule or a molecule moiety. Such atom, group of atoms, molecule or molecule moiety is also referred to herein as substituent.

The substituent can be selected from the group comprising hydroxy, alkoxy, mercapto, cycloalkyl, heterocyclic, aryl, heteroaryl, aryloxy, halogen, trifluoromethyl, difluoromethyl, cyano, nitrone, amino, amido, -C(O)H, acyl, oxyacyl, carboxyl, carbamate, sulfonyl, sulphonamide and sulfuryl. Any of the substituents may be substituted itself by any of the aforementioned substituents. This applies preferably to cycloalkyl, heterocylic, aryl, heteroaryl and aryloxy. It is

also preferred that alkoxy and mercapto are those of a lower alkyl group. It is to be acknowledged that any of the definition provided herein also applies to any substituent.

In a further aspect the present invention is related to the use of any of the aforementioned compounds according to the present invention as an inhibitor to rotamases. In connection with the characteristics of the compounds according to the present invention to be active as an inhibitor of (a) rotamase(s), it is sufficient that the respective compound is at least suitable to inhibit at least one rotamase. The compounds according to the present invention which may be used as inhibitors, are also referred to as rotamase inhibitors herein.

Rotamases as such are known in the art and, for example, described in the introductory part of this specification which is incorporated by reference. Rotamases as used herein shall mean cyclophilins, FK-506 binding proteins and the rotamases of the Pinl/parvulin class. The Pin1/parvulin class includes Pins 1 to 3, PinL/parvulin, dodo, and Es1/Pft1. Suitable assays to determine whether a compound is suitable to inhibit a rotamase are known to the one skilled in the art and also described in the present examples. Basically, a rotamase is provided the activity of which or non-activity of which may be determined. A candidate inhibitor, i. e. a compound which is to be tested whether it is active as an inhibitor to rotamase, is added to the rotamase and tested whether upon the addition and/or influence of the candidate inhibitor the activity of the rotamase is changed relative to the activity without candidate rotamase inhibitor. If the rotamase activity is decreased by the candidate rotamase inhibitor, said candidate rotamase inhibitor is a rotamase inhibitor according to the present invention.

In another aspect of the present invention the compounds according to the present invention may be used in a method for inhibiting a rotamase. In such case a rotamase is provided and a candidate rotamase inhibitor is added thereto whereupon the activity of rotamase is decreased. Optionally, such decrease in rotamase activity is measured. The techniques used theretofore are basically the same as outlined in connection with the use of the compounds according to the present invention as rotamase inhibitors.

The compounds according to the present invention are preferably reversible rotamase inhibitors.

By "reversible" herein is meant that the inhibitor binds non-covalently to the enzyme, and is to be distinguished from irreversible inhibition. See Walsh, Enzymatic Reaction Mechanisms, Freeman & Co., N.Y., 1979. "Reversible" in this context is a term understood by those skilled in the art. Preferably the rotamase inhibitors according to the present invention are competitive inhibitors, that is, they compete with substrate in binding reversibly to the enzyme, with the binding of inhibitor and substrate being mutually exclusive.

In a preferred embodiment of the compounds according to the present invention being active as a rotamase inhibitor, the dissociation constant for inhibition of a rotamase with the inhibitor, generally referred to and characterized by those in the art as $K_i$, is at most about 100 µM. By the term "binding constant" or "dissociation constant" or grammatical equivalents herein is meant the equilibrium dissociation constant for the reversible association of inhibitor with enzyme. The dissociation constants are defined and determined as described below. The determination of dissociation constants is known in the art. For example, for reversible inhibition reactions such as those of the present invention, the reaction scheme is as follows:

$$E + I \underset{k_2}{\overset{k_1}{\rightleftharpoons}} E^*I \quad \text{(Equation 1)}$$

The enzyme (E) and the inhibitor (I) combine to give an enzyme-inhibitor complex (E*I). This step is assumed to be rapid and reversible, with no chemical changes taking place; the enzyme and the inhibitor are held together by non-covalent forces. In this reaction, $k_1$ is the second order rate constant for the formation of the E*I reversible complex. $k_2$ is the first order rate constant for the dissociation of the reversible E*I complex. In this reaction, $Ki = k_2/k_1$.

The measurement of the equilibrium constant $K_i$ proceeds according to techniques well known in the art, as described in the examples. For example, assays generally use synthetic chromogenic or fluorogenic substrates. The respective $K_i$ values may be estimated using the Dixon plot as described by Irwin Segel in Enzyme Kinetics: Behavior and analysis of rapid equilibrium and steady-state enzyme systems, 1975, Wiley-Interscience Publication, John Wiley & Sons, New York, or for competitive binding inhibitors from the following calculation:

$$1-(\nu_i/\nu_o)=[I]/[I]+K_i\ (1+([S]/K_m))) \qquad \text{(Equation 2)}$$

wherein $\nu_o$ is the rate of substrate hydrolysis in the absence of inhibitor, and $\nu_i$ is the rate in the presence of competitive inhibitor.

It is to be understood that dissociation constants are a particularly useful way of quantifying the efficiency of an enzyme with a particular substrate or inhibitor, and are frequently used in the art as such. If an inhibitor exhibits a very low $K_i$ value, it is an efficient inhibitor. Accordingly, the rotamase inhibitors of the present invention have dissociation constants, $K_i$, of at most about 100 µM. Preferably, the rotamase inhibitors according to the present invention exhibit dissociation constants of at most about 10 µM, more preferably about 1 µM, most preferably of at most about 100 nM.

The rotamase inhibitors of the present invention may be easily screened for their inhibitory effect. The inhibitor is first tested against different classes of rotamases for which the targeting group of the inhibitor was chosen, as outlined above. The activity of rotamases is typically measured by using a protease coupled assay with chromogenic substrates and conformer specific proteases. Basically, upon the conformer specific protease activity the chromogenic substrate is converted into a compound which has an absorption characteristic which is different from the starting chromogenic substrate and may thus be selectively measured. This reaction is accelerated in presence of the rotamase and decelerated in the presence of rotamase-inhibitors. Alternatively, many rotamases and their corresponding chromogenic substrates are commercially available. Thus, a variety of rotamases are routinely assayed with synthetic chromogenic substrates in the presence and absence of the rotamase inhibitor, to confirm the inhibitory action of the compound, using techniques well known in the art. The effective inhibitors are then subjected to kinetic analysis to calculate the $K_i$ values, and the dissociation constants determined.

If a compound inhibits at least one rotamase, it is a rotamase inhibitor for the purposes of the present invention. Preferred embodiments of the rotamase inhibitors according to the present invention are compounds and inhibitors, respectively, that exhibit the correct kinetic parameters Ki below 100 µM against the targeted rotamases.

In a further aspect of the present invention any of the compounds used as rotamase inhibitors or as a medicament may be labelled.

By a "labelled rotamase inhibitor" herein is meant a rotamase inhibitor that has at least one element, isotope or chemical compound attached to enable the detection of the rotamase inhibitor or the rotamase inhibitor bound to a rotamase. In general, labels as used herein, fall into three classes: a) isotopic labels, which may be radioactive or heavy isotopes; b) immune labels, which may be antibodies or antigens; and c) colored or fluorescent dyes. The labels may be incorporated into the rotamase inhibitor at any position. Examples of useful labels include $^{14}C$, $^{13}C$, $^{15}N$, $^{3}H$, biotin, and fluorescent labels as are well known in the art.

In a further aspect the compounds according to the present invention, particularly those having rotamase inhibitory activity, may be used for removing, identifying and/or inhibiting contaminating rotamases in a sample.

Therefore, the rotamase inhibitors of the present invention are, for example, added to a sample where the catalytic activity by contaminating rotamases is undesirable. Alternatively, the rotamase inhibitors of the present invention may be bound to a chromatographic support, using techniques well known in the art, to form an affinity chromatography column. A sample containing an undesirable rotamase is run through the column to remove the rotamase. Alternatively, the same methods may be used to identify new rotamases. In doing so, a new rotamase contained in a sample may bind to the rotamase inhibitor bound to the chromatographic support and upon elution, preferably a specific elution, from said chromatographic support, characterized and compared to other rotamase activities with regard to, among others, specificities. The characterization of the rotamase as such is known to the one skilled in the art.

In a further aspect the present invention is related to the use of the compounds according to the present invention as a medicament and for the manufacture of a medicament, respectively.

This use of the compounds according to the present invention is based on the fact that the compounds according to the present invention are inhibitors of rotamases and rotamases in turn have been identified in both procaryotic and eucaryotic cells such as in bacteria, fungi, insect and mammalian cells. In this cellular environment rotamases are known to have an impact on cell proliferation and mitosis, respectively. Because of this, rotamase inhibitors may be used for the treatment of a wide variety of disorders involving cell cycle regulation, both procaryotic and eucaryotic cell cycle regulation. The term "treatment" as used herein comprises both treatment and prevention of a disease. It also comprises follow-up treatment of a disease. Follow-up treatment is realized upon a treatment of a disease using compounds preferably different from the one according to the present invention. For example, after stimulating the growth of a cell, tissue or the like by the application of a respective compound such as, e. g., erythropoietin, it might be necessary to stop an overshooting reaction of cell proliferation which may be obtained using the compounds according to the present invention.

As used herein, the term "disease" describes any disease, diseased condition or pathological condition. Such disease may also be defined as abnormal condition. Also, in case of a pathogen, disease means a condition where a pathogen or an unwanted organism is present or present in a concentration or compartment where it is undesired and thus subject to reduction in numbers, removal, elimination and/or destruction by using the compounds according to the present invention.

Cell proliferative disorders contemplated for treatment using the compounds according to the present invention and for the methods disclosed herein include disorders characterized by unwanted or undesired, inappropriate or uncontrolled cell growth. Preferably, the disease is selected from the group comprising neurodegenerative diseases,

stroke, inflammatory diseases, immune based disorders, infectious diseases, heart diseases, fibrotic disorders, cardiovascular diseases and cell proliferative diseases. Rotamases comprise families of ubiquitous and highly conserved enzymes who have been reported to play important roles in biological processes like protein folding, proteolysis, protein dephosphorylation, peptide transport function, cell cycle regulation, protein synthesis. Furthermore various isomerases have been shown to have regulatory functions as stable or dynamic part of heterooligomeric complexes containing physiologically relevant proteins e.g. hormone receptors, ion channels, kinases, and growth factor receptors.

Preferably, the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral atrophy, prion diseases, and motor neuron diseases.

The compounds according to the present invention are additionally useful in inhibiting cell cycle (mitosis) or cell division in pathogenic organisms and are, therefore, useful for treating infectious diseases.

In a preferred embodiment the infectious is selected from the group comprising fungal, viral, bacterial and parasite infection.

Fungal infections contemplated for treatment using the compounds and methods according to the present invention include systemic fungal infections, dermatophytoses and fungal infections of the genito-urinary tract. Fungal infections, preferably systemic fungal infections, include those caused by *Histoplasma, Coccidioides, Cryptococcus, Blastomyces, Paracoccidioides, Aspergillus, Nocardia, Sporothrix, Rhizopus, Absidia, Mucor, Hormodendrum, Phialophora, Rhinosporidium,* and the like. Dermatophyte infections include those caused by *Microsporum, Trichophyton, Epidermophyton, Candida, Pityrosporum,* and the like. Fungal disorders of the genito-urinary tract include infections caused by *Candida, Cryptococcus, Aspergillus, Zygomycodoides,* and the like. Infection by such organisms causes a wide variety of disorders such as ringworm, thrush or candidiasis, San Joaquin fever or Valley fever or coccidiodomycosis, Gilchrist's disease or blastomycosis, aspergillosis, cryptococcosis, histioplasmosis, paracoccidiomycosis, zygomycosis, mycotic keratitis, nail hair and skin disease, Lobo's disease, lobomycosis, chromoblastomycosis, mycetoma, and the like. These infections can be particularly serious, and even fatal, in patients with a depressed immune system such as organ transplant recipients and persons with acquired immunodefficiency syndrome (AIDS). Insofar a patient group which can be treated using the inhibitors according to the present invention are persons with AIDS, particularly those suffering from any of the aforementioned infectious diseases.

In a further embodiment the bacterial infection is selected from the group comprising infections caused by both Gram-positive and Gram-negative bacteria, including infections caused by Staphylococcus, Clostridium, Streptococcus, Enterococcus, Diplococcus, *Hemophilus, Neisseria, Erysipelothricosis, Listeria, Bacillus, Salmonella, Shigella, Escherichia, Klebsiella, Enterobacter, Serratia, Proteus, Morganella, Providencia, Yersinia, Camphylobacter, Mycobacteria, Helicobacter, Legionalla, Nocardia,* and the like.

In a preferred embodiment the bacterial infection causes a wide variety of diseases. Said disorders are selected, among others, from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

In another embodiment the disease is a viral infection, more particularly a viral infection caused by a virus selected from the group comprising retrovirus, HIV, Papilloma virus, Polio virus, Epstein-Barr, Herpes virus, Hepatitis virus, Papova virus, Influenza virus, Rabies, JC, encephalitis causing virus, hemorrhagic fever causing virus (such Ebola Virus and Marburg Virus).

In a further embodiment the parasite infection is selected from the group comprising infections caused by *Trypanosoma, Leishmania, Trichinella,* Echinococcus, *Nematodes, Classes Cestoda, Trematoda, Monogenea, Toxoplasma, Giardia,* Balantidium, *Paramecium, Plasmodium* or *Entamoeba.*

The disease may further be a cell proliferative disorder which preferably is selected from the group characterized by unwanted, inappropriate or uncontrolled cell growth. Particular examples include cancer, fibrotic disorders, non-neoplastic growths. The neoplastic cell proliferative disorder is preferably selected from the group comprising solid tumors, and hematopoeitic cancers such as lymphoma and leukemia.

More preferably, the solid tumor is selected from the group comprising carcinoma, sarcoma, osteoma, fibrosarcoma, and chondrosarcoma.

More preferably, the cell proliferative disorder is selected from the group comprising breast cancer, prostate cancer, colon cancer, brain cancer, lung cancer, pancreatic cancer, gastric cancer, bladder cancer, kidney cancer and head and neck cancer. Preferably, the lung cancer is non-small lung cancer and small lung cancer.

In case the disease is a non-proliferative cell proliferative disorder, it is preferably selected from the group comprising fibrotic disorder. Preferably, the fibrotic disorder is fibrosis.

The disease may also be a non-neoplastic cell proliferative disorder which is selected from the group comprising prostatic hypertrophy, preferably benign prostatic hypertrophy, endometriosis, psoriasis, tissue repair and wound healing.

Fibrotic disorders which may be treated using the compounds according to the present invention are generally characterized by inappropriate overproliferation of non-cancerous fibroblasts. Examples thereof include fibromyalgia, fibrosis (cystic, hepatic, idopathic pulmonary, pericardial and the like), cardiac fibromas, fibromuscular hyperplasia, restenosis, atherosclerosis, fibromyositis, and the like.

In another embodiment the immune based and/or inflammatory disease is an autoimmune disease or autoimmune disorder. In a further embodiment, the immune based and/or inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease, and dermatomyositis.

In a further preferred embodiment the immune based and/or inflammatory disease is an inflammatory condition.

In a still further embodiment the immune based and/or inflammatory disease is selected from the group comprising inflammation associated with burns, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

In a further embodiment of the various aspects of the present invention the rotamase is human Pin1 and the rotamase involved in the mechanism underlying the various diseases is human Pin1, respectively.

It is also within the present invention that the compounds according to the present invention may be used for the treatment of a patient suffering from a disease or disease condition as defined above. Such treatment comprises the administration of one or several of the compounds according to the present invention or a medicament or pharmaceutical composition described herein.

Toxicity and therapeutic efficacy of a compound can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Compounds which exhibit large therapeutic indices are preferred. The data obtained from these cell culture assays and animal studies can be used in formulating a range of dosages suitable for use in humans. The dosage may vary within this range depending upon a variety of factors, e.g., the dosage form employed, the route of administration utilized, the condition of the subject, and the like

For any compound used according to the present invention, the therapeutically effective dose can be estimated initially from cell culture assays by determining an $IC_{50}$ (i.e., the concentration of the test substance which achieves a half-maximal inhibition of rotamase activity). A dose can then be formulated in animal models to achieve a circulating plasma concentration range that includes the $IC_{50}$ as determined in cell culture. Such information can be used to more accurately determine useful doses in humans. Levels in plasma may be measured, for example by HPLC.

It should be noted that the attending physician would know how to and when to terminate, interrupt, or adjust administration due to toxicity, to organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient. Typically, the dose will be between about 1-10 mg/kg of body weight. About 1 mg to about 50 mg will preferably be administered to a child, and between 25 mg and about 1000 mg will preferably be administered to an adult.

A program comparable to that discussed above may be used in veterinary medicine. The exact dose will depend on the disorder to be treated and the amount of rotamases to be inhibited, and will be ascertainable by one skilled in the art using known techniques. For example, as outlined above, some disorders are associated with increased levels of rotamases.

Depending on the specific conditions being treated, such agents may be formulated and administrated systemically or locally. Techniques for formulation and administration may be found in "Remington's Pharmaceutical Sciences", 1990, 18th ed., Mack Publishing Co., Easton, PA. The administration of a compound according to the present invention can be done in a variety of ways, including, but not limited to, orally, subcutaneously, intravenously, intranasally, transdermally, intraperitoneally, intramuscularly, intrapulmonary, vaginally, rectally, or intraocularly, just to name a few. In some instances, for example, in the treatment of wounds and inflammation, the rotamase inhibitors may be directly applied as a solution or spray.

In a further aspect the present invention is related to a medicament or a pharmaceutical composition comprising at least one active compound and at least one pharmaceutically acceptable carrier, excipient or diluent. As

used herein, the active compound is a compound according to the present invention, a pharmaceutically salt or base thereof or a prodrug thereof, if not indicated to the contrary.

For injection, compounds of the invention may be formulated in aqueous solution, preferably in physiologically compatible buffers such as Hank's solution, Ringer's solution, or physiologically saline buffer. For transmucosal administration, penetrants appropriate to the barrier to be permeated are used in the formulation. Such penetrants are generally known in the art.

The use of pharmaceutical acceptable carriers to formulate the compounds according to the present invention into dosages or pharmaceutical compositions suitable for systemic administration is within the scope of the present invention. With proper choice of carrier and suitable manufacturing practice, the compositions of the present invention, in particular those formulated as solutions, may be administered parenterally, such as by intravenous injection. The compounds can be readily formulated using pharmaceutically acceptable carriers well known in the art into dosages suitable for oral administration. Such carriers enable the compounds according to the present invention to be formulated as tablets, pills, capsules, dragees, liquids, gels, syrups, slurries, suspensions and the like, for oral ingestion by a subject to be treated.

Compounds according to the present invention or medicaments comprising them intended to be administered intracellularly may be administered using techniques well known to those of ordinary skill in the art. For example, such agents may be encapsulated into liposomes, then administered as described above. Liposomes are spherical lipid bilayers with aqueous interiors. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Delivery systems involving liposomes are discussed in International Patent Publication No. WO 91/19501, as well as U.S. Patent No. 4,880,635 to Janoff et al. The publications and patents provide useful descriptions of techniques for liposome drug delivery and are incorporated by reference herein in their entirety.

Pharmaceutical compositions comprising a compound according to the present invention for parenteral administration include aqueous solutions of the active compound(s) in watersoluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injections suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions.

Pharmaceutical compositions comprising a compound according to the present invention for oral use can be obtained by combining the active compound(s) with solid excipient, optionally grinding the resulting mixture, and processing the mixture of granules, after adding suitable auxiliaries, if desired, to obtain tablets or dragee cores.

Suitable excipients are, in particular, fillers such as sugars, including lactose, sucrose, mannitol, sorbitol, and the like; cellulose preparations, such as, for example, maize starch, wheat starch, rice starch, potato starch, gelatin, gum tragacanth, methyl cellulose, hydroxypropylmethyl cellulose, sodium carboxymethyl cellulose, polyvinylpyrrolidone (PVP) and the like, as well as mixtures of any two or more thereof. If desired, disintegrating agents may be added, such as cross-linked polyvinyl pyrrolidone, agar, alginic acid or a salt thereof such as sodium alginate, and the like.

Dragee cores as a pharmaceutical composition comprising a compound according to the present invention are provided with suitable coatings. For this purpose, concentrated sugar solutions may be used, which may optionally contain gum arabic, talc, polyvinyl pyrrolidone, carbopol gel, polyethylene glycol, titanium dioxide, lacquer solutions, suitable organic solvents or solvent mixtures, and the like. Dyestuffs or pigments may be added to the tablets or dragee coatings for identification or to characterize different combinations of active compound doses.

Pharmaceutical preparations comprising a compound according to the present invention which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

A "patient" for the purposes of the present invention, i. e. to whom a compound according to the present invention or a pharmaceutical composition according to the present invention is administered, includes both humans and other animals and organisms. Thus the compounds, pharmaceutical compositions and methods are applicable to or in connection with both human therapy and veterinary applications. For example, the veterinary applications include, but are not limited to, canine, bovine, feline, porcine, caprine, equine, and ovine animals, as well as other domesticated animals including reptiles, such as iguanas, turtles and snakes, birds such as finches and members of the parrot family, lagomorphs such as rabbits, rodents such as rats, mice, guinea pigs and hamsters, amphibians, fish, and arthropods.

Valuable non-domesticated animals, such as zoo animals, may also be treated. In the preferred embodiment the patient is a mammal, and in the most preferred embodiment the patient is human.

The pharmaceutical composition according to the present invention comprises at least one compound according to the present invention, preferably a rotamase inhibitor according to the present application, in a form suitable for administration to a patient. Preferably, a compound according to the present application is in a water soluble form, such as being present as a pharmaceutically acceptable salt, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The pharmaceutical compositions according to the present invention may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Additives are well known in the art, and are used in a variety of formulations.

The compounds according to the present invention are, in a further embodiment, administered to a subject either alone or in a pharmaceutical composition where the compound(s) is mixed with suitable carriers or excipient(s). In treating a subject, a therapeutically effective dose of compound (i.e. active ingredient) is administered. A therapeutically effective dose refers to that amount of the active ingredient that produces amelioration of symptoms or a prolongation of survival of a subject which can be determined by the one skilled in the art doing routine testing.

On the other hand, the compounds according to the present invention which have a rotamase inhibitory activity may as such or contained in a pharmaceutical composition according to the present invention be used in drug potential applications.

For example, therapeutic agents such as antibiotics or antitumor drugs can be inactivated through the catalytic action of endogenous rotamases, thus rendering the administered drug less effective or inactive. Accordingly, the rotamase inhibitors of the invention may be administered to a patient in conjunction with a therapeutic agent in order to potentiate or increase the activity of the drug. This co-administration may be by simultaneous administration, such as a mixture of the rotamase inhibitor and the drug, or by separate simultaneous or sequential administration.

According to the present invention the compounds disclosed herein, referred to as compounds according to the present invention, may be used as a medicament or for the manufacture of medicament or in a method of treatment of a patient in need thereof. Insofar any of these compounds constitute a pharmaceutical compound. The use of this kind of compound also comprises the use of pharmaceutically acceptable derivatives of such compounds.

In addition, the compounds according to the present invention may be transformed upon application to an organism such as a patient, into the pharmaceutically active compound. Insofar the compounds according to the present invention may be prodrugs which, however, are nevertheless used for the manufacture of the medicaments as disclosed herein given the fact that at least in the organism they are changed in a form which allows the desired

It is to be understood that any of the pharmaceutical compositions according to the present invention may be used for any of the diseases described herein.

The pharmaceutical compositions according to the present invention may be manufactured in a manner that itself known, e.g., by means of conventional mixing, dissolving, granulating, dragee-mixing, levigating, emulsifying, encapsulating, entrapping, lyophilizing, processes, or the like.

In a further aspect of the present invention the compounds of the present invention may be used as insecticides as they may prevent cell cycle mitosis in insect cells and thus can be used to control the growth and proliferation of a variety of insect pests. This aspect of the present invention has important applications in agriculture, such as in the field, in the storage of agricultural products and the like. Additionally, the compounds according to the present invention are useful for controlling insect populations, preferably in places inhabited by men, such as homes, offices and the like.

Any of the compounds according to the present invention containing one or more asymmetric carbon atoms may occur as racemates and racemic mixtures, single enantiomers, diastereomeric mixtures and individual diastereomers. All such isomeric forms of these compounds are expressly included in the present invention. Each stereogenic carbon may be in the R or S configuration, or a combination of configurations.

It shall be understood by one of ordinary skill in the art that all compounds of the invention are those which are chemically stable. This applies to any of the various uses of the compounds according to the present invention

disclosed herein.

In determining the suitability of any of the compounds according to the present applications for the various uses, besides the particular profile to be met by such a compound, also it has to be checked whether it is stable to proteolytic degradation. The resistance of the compound use as rotamase inhibitor or pharmaceutical may be tested against a variety of non-commercially available rotamases in vitro to determine its proteolytic stability. Promising candidates may then be routinely screened in animal models, for example using labelled inhibitors, to determine the in vivo stability and efficacy. In any of the aforementioned uses the compound may be present in a crude or purified form. Methods for purifying the compounds according to the present invention are known to the one skilled in the art.

The invention is now further illustrated by reference to the following figure and examples from which further advantages, features and embodiments may be taken. It is understood that these examples are given for purpose of illustration only and not for purpose of limitation. All references cited herein are incorporated by reference.

Fig. 1 shows FACS results of compound 89.

Example 1: Material and Methods

In order that the invention herein described may be more fully understood, the following detailed description is set forth. As used herein, the following abbreviations are used:

Ar is argon;
d is doublet;
DCM is dichloromethane;
DIPEA is *N,N*-diisopropylethylamine;
DMF is N,N-dimethylformamide;
DMSO is *N*,*N*-dimethylsulfoxide;
eq is equivalent;
$NEt_3$ is triethylamine;
HCl is chlorhydric acid;
HPLC is high performance liquid chromatography;
h is hour;
Hz is hertz;
m is multiplet;
mL is milliliter;
$NaHCO_3$ is sodium hydrogencarbonate;
s is singulet;
THF is tetrahydrofuran.

**Method A: Urea formation in solution**

Amine salt and DIPEA (1 eq each) or amine (1 eq) was dissolved in dry dioxan and a solution of the isocyanate (1 eq) in DCM or DMSO was added under Ar in one portion. The solution was stirred for 3 h at room temperature. The solution was diluted with 2 ml DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added to remove unreacted isocyanate, amine and electrophilic impurities respectively. After 18 h at 40°C, the solution was filtered off and the solvent was removed under reduced pressure. The obtained crude ureas were purified by HPLC.

**Method B: Coupling of substituted anilines with sulfonyl chlorides**

Aniline (30 mg) and $NEt_3$ (1.2 eq, 2.2 eq. when HCl salt) were dissolved in dry acetonitrile (0.5 mL). Sulfonyl chloride (1 eq) was dissolved in dry acetonitrile (0.5 mL) and added to the solution. The reaction mixture was stirred under argon for 12 h at 40°C. Tris-(2-aminoethyl)-amine polystyrene (30 mg), (polystyrylmethyl) trimethylammonium bicarbonate (30 mg), N-(2-mercaptoethyl)amino methyl polystyrene (30 mg), and methylisocyanate polystyrene (30 mg) were given to the solution and stirred for additional 12 h at 40°C. After filtration the solvent was removed under reduced pressure. The crude reaction product was purified by preparative HPLC using acetonitrile and water as mobile phase.

**Method C: Coupling of substituted anilines with acid chlorides**

Aniline (30 mg) and $NEt_3$ (1 eq, 2 eq. when HCl salt) were dissolved at 0°C in dry DCM (0.5 mL) with 10%

DMSO. Acid chloride (1 eq) was dissolved at 0°C in dry DCM (0.5 mL) and added to the solution. The reaction mixture was stirred under argon for 2 h while slowly warming up to room temperature. Work up was performed by pouring the reaction mixture with DCM over incubated HYDROMATRIX layers. 2 mL basic layer (saturated $NaHCO_3$ solution 2 ml/g HYDROMATRIX), 2 mL acidic layer (2M HCl 2 ml/g HYDROMATRIX), and 2 mL of dry HYDROMATRIX layer in a 10 ml syringe were used. The solvent was removed under reduced pressure. The crude reaction product was purified by preparative HPLC using acetonitrile and water as mobile phase.

**Method D: Reductive amination of aldehydes with hydroxyl anilines**

Amine hydrochloride (0.11 mmol, 1 eq), aldehyde (0.11 mmol, 1 eq), and DIPEA (0.11 mmol, 1 eq) were dissolved in anhydrous THF (1 mL), and molecular sieves 4Å (10 mg) was added to the solution. After shaking for 1.5 h at room temperature, (polystyrylmethyl)trimethylammonium cyanoborohydride (4.3 mmol/g, 0.22 mmol) was added to the reaction mixture. After shaking for 8 h at room temperature, 4-benzyloxybenzaldehyde polystyrene (3 mmol/g, 0.22 mmol), 3-(4-(hydrazinosufonyl)phenyl)propionyl AM resin (1.5 mmol/g, 0.22 mmol), and N-(2-mercaptoethyl)aminomethyl polystyrene (2.1 mmol/g, 0.22 mmol) were added, after which the reaction mixture was shaken at room for 18 h. Filtration, washing with DCM, and evaporation of the solvent in vacuo afforded the crude product, which was purified by reversed phase HPLC.

**Method E: Condensation of amines with hydroxyl carboxylic acids**

1-Ethyl-3(3'-dimethylaminophropyl)carbodiimide hydrochloride (0.15 mmol, 1.36 eq), hydroxyl carboxylic acid (0.15 mmol, 1.36 eq), and 1-hydroxy-7-azabenzotriazole (0.15 mmol, 1.36 eq) were dissolved in DMF (0.7 mL). After shaking for 30 min at room temperature, a solution of amine (0.11 mmol) in DMF (0.7 mL) was added to the reaction mixture. After shaking for 2 h at room temperature, amine (0.22 mmol) was added, after which the reaction mixture was shaken at 60 °C overnight. Then the solvent was evaporated in vacuo, and the residue was dissolved in DCM (7 mL). HYDROMATRIX™ (0.3 g) which was previously treated with HCl (2N, 0.6 mL) was added to the solution, and the mixture was shaken for 30 min. Filtration, washing with DCM, and evaporation of the solvent in vacuo afforded the crude product, which was purified by reversed phase HPLC.

**Method F: Carbamate formation in solution**

Amine or amine salt (1 eq) and sodium bicarbonate (1 or 2 eq) were dissolved in a mixture of MeOH / $H_2O$ (3:1 ). The mixture was treated with chloroformate (1 eq), which was added in three portions over 10 minutes. After 30 minutes at room temperature, the precipitating product was collected by filtration and washed with water. The obtained crude carbamates were purified by HPLC.

**Method G: Carbamate formation in solution**

To an ice cooled mixture of amine or amine salt (1 eq) and DIPEA (1.1 eq or 2.2 eq) in dry DCM was added an ice cooled solution of chloroformate (1.1 eq) in DCM in one portion. After 1.5-8 h at room temperature the solvent was removed under reduced pressure. The obtained crude carbamates were purified by HPLC.

**Method H: Carbamate formation in solution**

To a mixture of amine or amine salt (1 eq) and sodium bicarbonate (1 or 2.5 eq) in dry DCM was added chloroformate (1 eq) in one portion. After 4 hours at room temperature the sodium bicarbonate was filtered off and the solvent was removed under reduced pressure. The obtained crude carbamates were purified by HPLC.

**Method I: Thiourea formation in solution**

Amine salt and DIPEA (1 eq each) or amine (1 eq) was dissolved in dry dioxan and a solution of the thioisocyanate (1 eq) in DCM or DMSO was added under Ar in one portion. The solution was stirred for 3 h at room temperature. The solution was diluted with 2 ml DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added to remove unreacted isocyanate, amine and electrophilic impurities respectively. After 18 h at 40°C, the solution was filtered off and the solvent was removed under reduced pressure. The obtained crude thioureas were purified by HPLC.

**Method J: Synthesis of (3-amino-5-chloro-4-hydroxy-phenyl)-ureas.**

Isocyanate (5.0 mmol) was added to a stirred solution of 4-Amino-2-chloro-phenol (5.0 mmol) in anhydrous $CH_2Cl_2$ (23 mL) and THF (4 mL) at room temperature. After stirring for 12 h, the solvent was evaporated in vacuo. The residue was then dissolved in HOAc (95 mL) and added in one single portion to a stirred solution of $NaNO_2$ (1.17 g, 17.0 mmol) in $H_2O$ (8.4 mL). The flask was sealed with a stopper and the reaction mixture was stirred for 1.5 min at room temperature. The reaction was stopped by the addition of saturated aqueous $NaHCO_3$ (190 mL). After stirring for 10 min at room temperature, the yellow precipitate was filtered, washed with $H_2O$ (3 x 30 mL), and dried in vacuo. The yellow residue was then dissolved in a mixture of toluene (75 mL) and MeOH (90 mL). Raney nickel (0.5 g) was washed with MeOH (5 x 10 mL) and added to the reaction mixture. Then the reaction mixture was vigorously stirred under a hydrogen atmosphere at 1 bar at room temperature for 2 h. Filtration through a pad of Celite and evaporation of the solvent afforded the aniline, which was converted into the corresponding diureas by method A, the corresponding anilines by method D, the corresponding sulfonamides by method B, the corresponding amides by method C or E, or the corresponding thioureas by method I.

**Method K: Synthesis of (5-benzothiazol-2-yl-3-chloro-2-hydroxy-phenyl)-ureas, (5-benzothiazol-2-yl-3-chloro-2-hydroxy-phenyl)-thioureas, (3-benzothiazol-2-yl-5-chloro-4-hydroxy-phenyl)-ureas, (3-benzothiazol-2-yl-5-chloro-4-hydroxy-phenyl)-thioureas, (5-benzothiazol-2-yl-3-chloro-2-hydroxy-phenyl)-amides, and (3-benzothiazol-2-yl-5-chloro-4-hydroxy-phenyl)-amides.**

3-Chloro-2-hydroxy-5-nitro-benzoic acid or 3-chloro-4-hydroxy-5-nitro-benzoic acid (0.41 g, 1.9 mmol) was dissolved in polyphosphoric acid (12.3 g) at 110 °C. 2-Aminothiophenol (0.36 mg, 2.9 mmol) was added and the resulting solution was stirred at 110 °C for 5 h. After cooling, ammonia (35% in $H_2O$, 12 mL) was added to the reaction mixture. The precipitate was filtered, washed with $H_2O$ (3 x 10 mL) and dried in vacuo. The residue was then dissolved in a mixture of MeOH (30 mL) and THF (70 mL). Raney nickel (0.5 g) was washed with MeOH (5 x 10 mL) and added to the reaction mixture. Then the reaction mixture was vigorously stirred under a hydrogen atmosphere at 1 bar at room temperature for 1 h. Filtration through a pad of Celite and evaporation of the solvent afforded the aniline, which was converted into the corresponding ureas by method A, the corresponding amides by method C or E, or the corresponding thioureas by method I.

**Method L: Molecular cloning techniques, protein expression and purification**

Biochemicals, media and molecular weight markers (if not declared in the section below) were obtained from Roth (Karlsruhe, Germany), Gibco (Paisley, UK) and VWR International (Merck, Darmstadt, Germany).

All plasmids coding for rotamases were constructed by standard recombinant DNA techniques (Sambrook, J.; Fritsch, E. F.; Maniatis, T. (1989) Molecular Cloning: A Laboratory Manual, second ed., Cold Spring Harbor Laboratory Press, Cold Spring Harbor, NY.) and their authenticity was confirmed by DNA sequencing. Generally, all rotamases were expressed with an N-terminal $(His)_6$ tag and were purified using a $Ni^{2+}$-NTA agarose column, followed by desalting with Pharmacia PD-10 colums, cation-exchange chromatography (HiTrap SP FF) and gelfiltration. The detailed cloning, protein expression and purification procedures are explained below using NIMA-interaction protein as an example.

The complete NIMA-interaction protein-coding sequence was amplified using the PrimeZyme™-Kit (Biometra, Göttingen, Germany), cDNA from HEK-293 cells as template and two primers (5'-GATCGAGGATCCGCGGACGAGGAGAAGCTGCCG-3' and 5'-TCGATCAAGCTTTCA CTCAGTGCGGAGGATGATGTG-3', purchased from MWG-biotech, Ebersberg, Germany). The resulting PCR-product was purified by preparative agarose gel electrophoresis (GeneGleanII-Kit, Vista, USA), digested with BamH1 and HindIII and ligated into pQE30 (Qiagen, Hilden, Germany, DNA-modifying enzymes from Roche, Mannheim, Germany). After transformation into *E. coli* M15 recombinants were screened by restriction analysis and small-scale overexpression studies. Positive clones were verified by DNA-sequencing. The clone pTTPHQ1 was used for purification of recombinant human NIMA-interacting protein, containing an N-terminal extension with the sequence $MRGS(H)_6GS$, followed by the complete sequence of NIMA-interacting protein (lacking the original N-terminal methionine).

For overexpression cells of *E. coli* pTTPHQ1/M15 were grown in 2 L of LB-media (supplemented with 100μg/mL Ampicillin, 25μg/mL Kanamycin) until an $OD_{600}$ of 0,7 was reached. Then 2,5mM IPTG was added and the cells were grown for further 5 hours. The cells were harvested by centrifugation, resuspended in 40mL buffer A (50mM phosphate buffer [pH 8,0], 0,3M NaCl, 1mM PMSF) and passed repeatedly through a French pressure cell from SLM Aminco (Buettelborn, Germany). All the following procedures were carried out at 4 °C. Cell debris were removed by ultracentrifugation at 35000 rpm for 40 min (centrifuge and 45Ti rotor from Beckman, Palo Alto, California, USA). The supernatant was applied to a 1ml $Ni^{2+}$-charged His-Trap-chelating column (Äkta-FPLC-system and all chromatographic

media used from Amersham Pharmacia Biotech, Uppsala, Sweden). Unspecifically bound material was removed from the matrix by 50mM phosphate buffer (pH 6,0), 0,3M NaCl, 10% glycerol (v,v). Bound proteins were eluted from the column with a linear gradient of 0-0,5M imidazole in buffer A. After desalting and buffer exchange in buffer B (30mM Tris/Cl, pH 8,0) by using PD10-columns pooled protein fractions were applied to a cation-exchange column (1ml HiTrap SP FF) and bound proteins were eluted by a linear gradient of 0-1M KCl in buffer B. Further purification of the enriched recombinant human NIMA-interacting protein was achieved by gel filtration. Pooled fractions from the cation exchange column were concentrated to a volume of <1mL (Vivaspin6, MWCO 8000kDa, Vivascience, USA) and applied to a Superdex200pg 16/60 HiLoad FPLC column equilibrated with buffer A. After gel filtration followed by a buffer exchange in 0.01 M HEPES (pH 7.5), 0.15 M KCl, 0.015 M $MgCl_2$, 2mM DTT recombinant human NIMA-interacting protein was homogenous in the SDS-PAGE as verified by silver staining. This protocol is similar to the one of Hennig et al. (Selective Inactivation of Parvulin-like peptidyl-prolyl cis/trans isomerases by Juglon, Biochemistry. 1998, 37(17):5953-5960) and Hottenrott et al. (THE ESCHERICHIA COLI SLYD IS A METAL ION-REGULATED PEPTIDYL-PROLYL CIS/TRANS-ISOMERASE. J. Biol. Chem. 272:15697-15701, 1997).

Example 2: 1-Adamantan-1-yl-3-(3,5-dichloro-2-hydroxy-4-methyl-phenyl)-urea

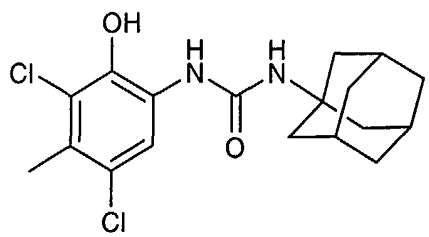

To a solution of 6-amino-2,4-dichloro-3-methyl-phenol hydrochloride (113.5 mg, 1 eq) and DIPEA (48 µL, 1 eq) in dioxan (1.1 mL) was added 1-adamantylisocyanate (88.5 mg, 1 eq) in 580 µL DMSO in one portion. The solution was stirred at room temperature for 3 h. The solution was diluted with 2 mL DCM and scavenger resins (tris-(2-aminoethyl)-amine polystyrene (3 eq), methylisocyanate polystyrene (3 eq) and *N*-(2-mercaptoethyl)aminomethyl polystyrene (3 eq)) were added. After 18 h at 40°C the solution was filtered off and the solvent was removed under reduced pressure. The crude product was purified by HPLC to obtain 118 mg (64 %) of the title compound as a white powder.
NMR-$^1$H (DMSO-$d_6$) $\delta$ = 1.62 ($s_b$, 6H), 1.92 ($s_b$, 6H), 2.05 ($s_b$, 3H), 2.29 (s, 3H), 6.79 (s, 1H), 7.95 (s, 1H), 8.15 (s, 1 H), 9.82 (s, 1 H).
NMR-$^{13}$C (DMSO-$d_6$) $\delta$ = 17.1, 29.3, 36.1, 41.5, 50.3, 116.3, 122.1, 123.8, 124.7, 129.9, 140.6, 154.3; MS (m/z): 369.2 [M+H$^+$].

Example 3: 1-(3,5-Dichloro-4-hydroxy-phenyl)-3-(1,1,3,3-tetramethyl-butyl)-urea

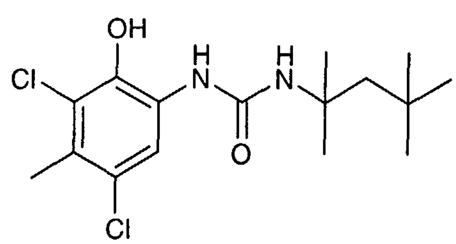

To a solution of 4-amino-2,6-dichloro-phenol (44.5 mg, 1 eq) in dioxan (2.0 mL) was added 2-isocyanato-2,4,4-trimethyl-pentane (38 mg, 1 eq) in one portion. The solution was stirred at room temperature for 3 h. The solution was diluted with 2 mL DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added. After 18 h at 35°C the solution

was filtered off and the solvent was removed under reduced pressure. The crude material was purified by HPLC to obtain 67 mg (81 %) of the title compound as a white powder.
NMR-$^1$H (DMSO-$d_6$) δ = 0.95 (s, 9H), 1.28 (s, 6H), 1.69 (s, 2H), 5.90 (s, 1H), 7.33 (s, 2H), 8.22 (s, 1H), 9.50 ($s_b$, 1H). NMR-$^{13}$C (DMSO-$d_6$) δ = 29.7, 31.2, 50.5, 53.2, 117.3, 1222.4, 134.0, 142.8, 153.9; MS (m/z): 333.2 [M+H$^+$].

Example 4: 2 *N*-(2-Hydroxy-4-methyl-phenyl)-C-phenyl-methanesulfonamide

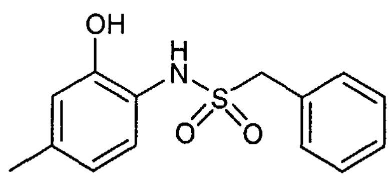

The compound was obtained in 32% yield (21.2 mg) using the protocol described in method A. NMR-$^1$H (DMSO-$d_6$) δ = 2.13 (s, 3H), 4.97 (s, 2H), 6.70 (d, 1H, *J* = 8.2 Hz), 6.78 (s, 1H), 6.83 (d, 1H, *J* = 8.2 Hz), 7.43 (m, 2H), 7.49 (m, 2H); MS (*m*/*z*): 278.1 [M$^+$].

Example 5: Propane-2-sulfonic acid (3,5-dichloro-2-hydroxy-4-methyl-phenyl)-amide

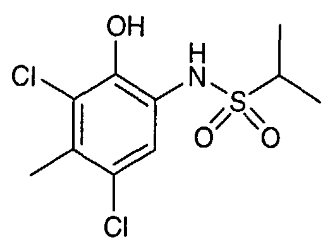

The compound was obtained in 47% yield (22.1 mg) using the protocol described in method B. NMR-$^1$H (DMSO-$d_6$) δ = 2.25 (d, 6H, *J* = 6.8), 2.27 (s, 3H), 3.97 (hep, 1H, *J* = 6.8), 5.39 (s, 1H), 6.91 (s, 1H); MS (*m*/*z*): 298.1 [MH$^+$].

Example 6: N-(3,5-Dichloro-4-hydroxy-phenyl)-2-phenyl-acetamide

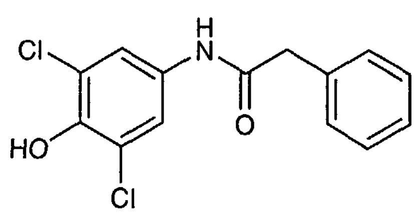

The compound was obtained in 22% yield (11.0 mg) using the protocol described in method C. NMR-$^1$H (DMSO-$d_6$) δ = 3.06 (s, 2H), 7.25 (m, 1H), 7.31 (m, 2H), 7.32 (m, 2H), 7.61 (m, 2H), 9.87 (m, 1H); MS (*m*/*z*): 296.2 [MH$^+$].

Example 7: N-(3,5-Dichloro-2-hydroxy-4-methyl-phenyl)-N-methyl-2-trifluoromethylbenzamide

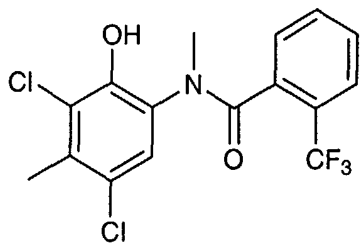

Aniline HCL salt (300 mg, 1.3 mmol) and $K_2CO_3$ (500 mg) were dissolved in DMSO (5 mL). $CH_3I$ (187 mg, 1.3 mmol) was added and the suspension was stirred for 48 h at room temperature. After filtration, the solvent was removed under reduced pressure. The crude reaction product was purified by preparative HPLC using acetonitrile and water as mobile phase to give the *N*-methyl amino phenol derivative (130 mg, 31 %).
The amide was obtained in 56% yield (19.7 mg) using the protocol described in method C. NMR-$^1$H (DMSO-$d_6$) $\delta$ = 2.41 (s, 3H), 3.75 (s, 3H), 7.70-7.85 (m, 4H), 8.05 (d, 1H, *J* = 5.0); MS (*m*/*z*): 377.9 [$MH^+$].

Example 8: 2,4-Dichloro-3-methyl-6-(3-methyl-benzylamino)-phenol

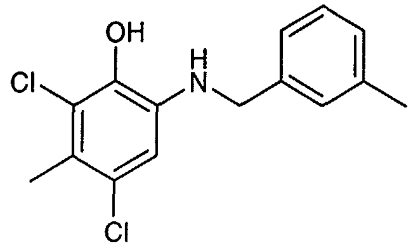

According to method D 6-amino-2,4-dichloro-3-methyl-phenol hydrochloride (25 mg, 0.11 mmol) and 3-methyl-benzaldehyde (13 mg, 0.11 mmol) gave 2,4-dichloro-3-methyl-6-(3-methyl-benzylamino)-phenol (16 mg, 49%). NMR-$^1$H (DMSO-$d_6$) $\delta$ 2.21 (s, 3 H), 2.27 (s, 3 H), 4.27 (s, 2 H), 5.80 (br s, 1 H), 6.35 (s, 1 H), 7.02-7.24 (m, 4 H), 9.18 (br s, 1 H); MS (*m*/*z*): 296.2 [$M+H^+$].

Example 9: *N*-Benzyl-4-bromo-3,5-dihydroxy-benzamide

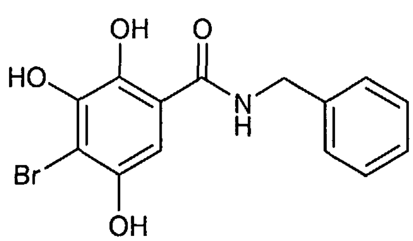

According to method E 4-bromo-3,5-dihydroxy-benzoic acid (35 mg, 0.15 mmol) and benzylamine (48 mg, 0.45 mmol) gave *N*-Benzyl-4-bromo-3,5-dihydroxy-benzamide (19 mg, 39%).
NMR-$^1$H (DMSO-$d_6$) δ 4.40 (d, *J* = 6.0 Hz, 2H), 6.86 (s, 2H), 7.20-7.36 (m, 5H), 8.91 (t, *J* = 6.2 Hz, 1H), 10.24 (s, 2H); MS (*m*/*z*): 322.1 [M+H$^+$].

Example 10: 2-(3-Chloro-4-hydroxy-phenyl)-1-piperidin-1-yl-ethanone

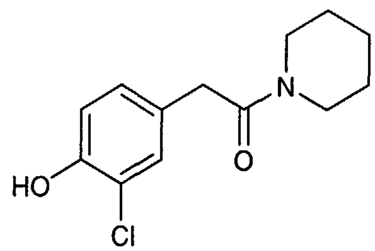

According to method E (3-chloro-4-hydroxy-phenyl)-acetic acid (28 mg, 0.15 mmol) and piperidine (38 mg, 0.45 mmol) gave 2-(3-chloro-4-hydroxy-phenyl)-1-piperidin-1-yl-ethanone (12 mg, 32%).
NMR-$^1$H (DMSO-$d_6$) δ 1.29-1.42 (m, 4 H), 1.48-1.59 (m, 2 H), 3.34-3.45 (m, 4 H), 3.57 (s, 2 H), 6.88 (d, *J* = 8.3 Hz, 1 H), 6.97 (dd, *J* = 8.3, 2.0 Hz, 1 H), 7.17 (d, *J* = 2.0 Hz, 1H), 10.00 (s, 1 H); MS (*m*/*z*): 254.3 [M+H$^+$].

Example 11: (3,5-Dichloro-4-hydroxy-phenyl)-carbamic acid phenyl ester

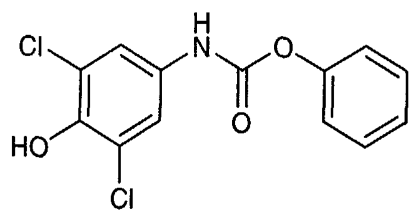

According to method F 4-amino-2,6-dichloro-phenol (67.5 mg, 1 eq) and phenylchloroformate (47 µL, 1 eq) gave 55.7 mg (49 %) of the title compound as a white solid.
NMR-$^1$H (DMSO-$d_6$) δ = 7.25 (m, 2H), 7.43 (m, 2 H), 7.50 (s, 2H)
NMR-$^{13}$C (DMSO-$d_6$) δ = 118.5, 121.9, 122.5, 125.5, 129.4, 131.7, 144.7, 150.3, 151.6

Example 12: (3,5-Dichloro-2-hydroxy-4-methyl-phenyl)-carbamic acid phenyl ester

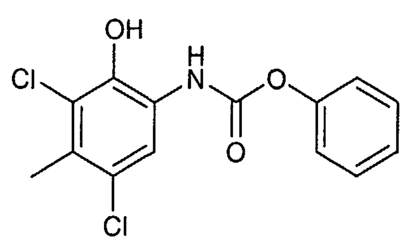

According to method F 6-amino-2,4-dichloro-3-methylphenol hydrochloride (57 mg, 1 eq) and phenylchloro-

formate (31.3 µl, 1 eq) gave 24.3 mg (31 %) of the title compound as a white solid.
NMR-$^1$H (DMSO-$d_6$) δ = 2.38 (s, 3 H), 6.76 (m, 3H), 7.15 (m, 3H)
NMR- $^{13}$C (DMSO-$d_6$) δ = 16.5, 109.1, 115.2, 118.8, 126.7, 128.5, 129.5, 129.7, 139.4, 153.4, 157.4

Example 13: (3,5-Dichloro-2-hydroxy-phenyl)-carbamic acid phenyl ester

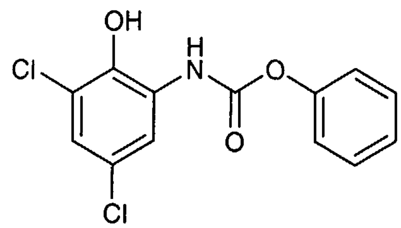

According to method F 2-amino-4,6-dichloro-phenol (25 mg, 1 eq) and phenylchloroformate (23 µL, 1 eq) gave 10.9 mg (26 %) of the title compound as a white solid.
NMR-$^1$H (DMSO-$d_6$) δ = 7.43 (m, 2 H), 7.25 (m, 2H), 7.5 (s, 2H)
NMR-$^{13}$C (DMSO-$d_6$) δ= 109.1, 118.8, 121.4, 128.2, 129.4, 132.6, 153.2, 157.3

Example 14: (3,5-Dichloro-2-hydroxy-4-methyl-phenyl)-carbamic acid 2-isopropyl-5-methyl-cyclohexyl ester

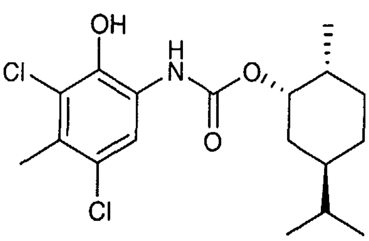

According to method G 6-amino-2,4-dichloro-3-methyl-phenol hydrochloride (35 mg, 1 eq) and (-)-menthylchloroformate (36.1 µL, 1.1 eq) in DCM gave 22 mg (39 %) of the title compound as a white solid.
NMR-$^1$H (DMSO-$d_6$) δ= 0.84-0.90 (m, 3H), 1.22-1.41 (m, 6H), 2.35 (s, 3H), 3.28-3.33 (m, 2H), 4.06 (t, 2H), 7.57 (s, 1H), 8.74 (s, 1H).

Example 15: (3,5-Dichloro-4-hydroxy-phenyl)-carbamic acid 2-isopropyl-5-methylcyclohexyl ester

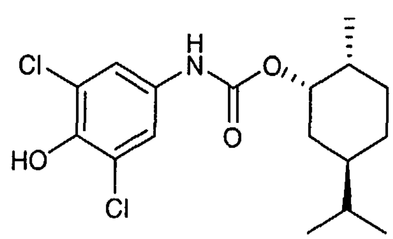

According to method G 4-amino-2,6-dichloro-phenol (40 mg, 1 eq) and (-)-menthylchloroformate (54 µl, 1.1 eq) in DCM gave 44.1 mg (54 %) of the title compound as a white solid.
NMR-$^1$H (DMSO-$d_6$) δ= 0.75 (d, 3H), 0.82-093 (m, 7H), 0.96-1.14 (m, 2H), 1.29-1.53 (m, 2H), 1.59-1.71 (m, 2H), 1.88-2.02 (m, 2H), 4.54 (ddd, 1H), 7.47 (s, 2H), 9.67 (s, 1H), 9.73 (s, 1H)

Example 16: (3,5-Dichloro-2-hydroxy-4-methyl-phenyl)-carbamic acid hexyl ester

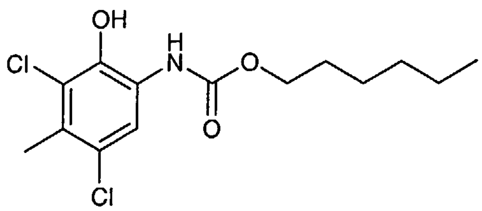

According to method G 6-amino-2,4-dichloro-3-methyl-phenol hydrochloride (35 mg, 1 eq) and hexylchloroformate (27.0 µL, 1.1 eq) gave 10.4 mg (21 %) of the title compound as a white solid.
NMR-$^1$H (DMSO-$d_6$) δ = 0.87 (t, 3H), 1.25-1.40 (m, 6H), 1.55-1.66 (m, 2H), 2.35 (s, 3H), 4.06 (t, 2H), 7.56 (s, 1H), 8.74 (s, 1H), 9.73 (s, 1H).

Example 17: (3,5-Dichloro-4-hydroxy-phenyl)-carbamic acid hexyl ester

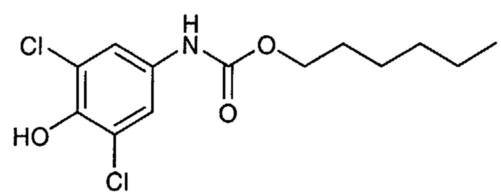

According to method G 4-amino-2,6-dichloro-phenol (40 mg, 1 eq) and hexylchloroformate (41 µL, 1.1 eq) gave 30 mg (43 %) of the title compound as a white solid.
NMR-$^1$H (DMSO-$d_6$) δ = 0.87 (t, 3H), 1.21-1.40 (m, 6H), 1.54-1.66 (m, 2H), 4.06 (t, 2H), 7.46 (s, 2H), 9.67 (s, 1H), 9.73 (s, 1H).

Example 18: 1-[3-Chloro-5-(3-cyclohexyl-ureido)-2-hydroxy-phenyl]-3-(2-trifluoromethylphenyl)-urea

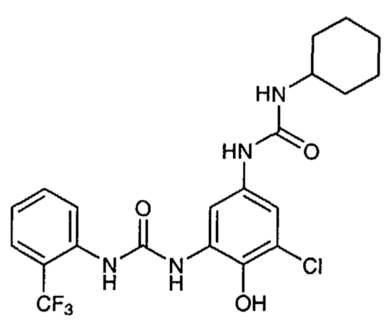

According to method J 1-(3-amino-5-chloro-4-hydroxy-phenyl)-3-cyclohexyl-urea (30 mg, 0.11 mmol) and 1-isocyanato-2-trifluoromethyl-benzene (21 mg, 0.11 mmol) gave 1-[3-chloro-5-(3-cyclohexyl-ureido)-2-hydroxy-phenyl]-3-(2-trifluoromethyl-phenyl)-urea (16 mg, 31%).
NMR-$^1$H (DMSO-$d_6$) δ 0.98-1.28 (m, 5 H), 1.39-1.76 (m, 5 H), 3.30-3.42 (m, 1 H), 5.84 (d, *J* = 7.8 Hz, 1 H), 7.24 (d, *J* = 7.8 Hz, 1 H), 7.31 (d, *J* = 2.4 Hz, 1 H), 7.52-7.67 (m, 2 H), 7.71-7.75 (m, 2 H), 8.20 (br. s, 1 H), 8.80-8.82 (m, 1 H), 8.97-9.04 (m, 1 H), 9.11 (s, 1H); MS: *m*/*z*: 471.1 [M+H$^+$].

Example 19: N-[3-Chloro-5-(3-cyclohexyl-ureido)-2-hydroxy-phenyl]-2-nitrobenzenesulfonamide

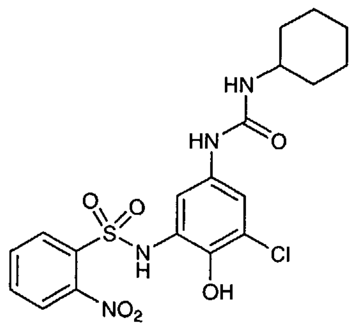

According to method J 1-(3-amino-5-chloro-4-hydroxy-phenyl)-3-cyclohexyl-urea (19 mg, 67 μmol) and 2-nitro-benzenesulfonyl chloride (15 mg, 67 μmol) gave *N*-[3-Chloro-5-(3-cyclohexyl-ureido)-2-hydroxy-phenyl]-2-nitrobenzenesulfonamide (15 mg, 48%).
NMR-$^1$H (DMSO-$d_6$) δ 1.05-1.37 (m, 5 H), 1.49-1.83 (m, 5 H), 3.42-3.47 (m, 1 H), 5.94 (d, *J* = 7.9 Hz, 1 H), 6.99 (d, *J* = 2.4 Hz, 1 H), 7.46 (d, *J* = 2.4 Hz, 1 H), 7.78-7.90 (m 2 H), 7.93-8.03 (m, 2 H), 8.27 (s, 1 H), 9.19 (br s, 1 H), 9.70 (s, 1 H); MS: *m*/*z*: 469.1 [M+H$^+$].

Example 20: 1-(3-Benzothiazol-2-yl-5-chloro-4-hydroxy-phenyl)-3-cyclohexyl-urea

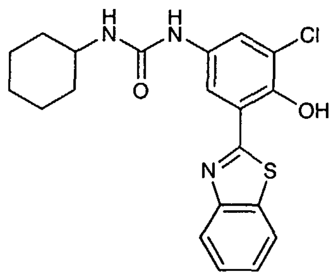

According to method K 4-amino-2-benzothiazol-2-yl-6-chloro-phenol (21 mg, 76 μmol) and isocyanato-cyclohexane (9.5 mg, 76 μmol) gave 1-(3-Benzothiazol-2-yl-5-chloro-4-hydroxyphenyl)-3-cyclohexyl-urea (3.0 mg, 10%).
NMR-$^1$H (DMSO-$d_6$) δ 1.09-1.41 (m, 5 H), 1.49-1.87 (m, 5 H), 3.41-3.47 (m, 1H), 6.12 (d, *J* = 7.8 Hz, 1 H), 7.51 (t, *J* = 7.8 Hz, 1 H), 7.60 (t, *J* = 7.1 Hz, 1 H), 7.68 (d, *J* = 2.4 Hz, 1 H), 8.06 (d, *J* = 2.4 Hz, 1 H), 8.11 (d, *J* = 8.3 Hz, 1 H), 8.21 (d, *J* = 7.3 Hz, 1 H), 8.52 (s, 1 H), 11.92 (s, 1 H); MS: *m*/*z*: 402.2 [M+H$^+$].

Example 21: 1-(5-Benzothiazol-2-yl-3-chloro-2-hydroxy-phenyl)-3-(2-trifluoromethylphenyl)-thiourea

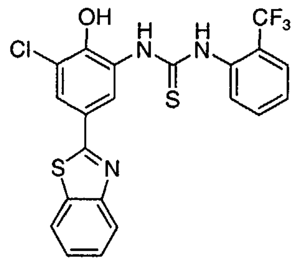

According to method K 2-Amino-4-benzothiazol-2-yl-6-chloro-phenol (20 mg, 72 μmol) and 1-isothiocyanato-2-trifluoromethyl-benzene (15 mg, 72 μmol) gave 1-(5-benzothiazol-2-yl-3-chloro-2-hydroxy-phenyl)-3-(2-trifluoromethyl-phenyl)-thiourea (12 mg, 35%).
NMR-$^1$H (DMSO-$d_6$) δ 7.40-7.56 (m, 3 H), 7.66-7.79 (m, 3 H), 7.92 (d, *J* = 2.4 Hz, 1 H), 8.03 (d, *J* = 7.8 Hz, 1 H), 8.12 (d, *J* = 8.8 Hz, 1 H), 8.68 (br s, 1 H), 9.65 (s, 1 H), 9.94 (s, 1 H), 10.65 (s, 1 H); MS: *m/z*: 480.0 [M+H$^+$].

Example 22: Specificity of inhibition of certain enzymes by compounds according to the present invention

In order to characterize the specificity of various compounds the following assays were performed. PPIase activity of hPin1, hCyp18, LpCyp18, hFKBP12 and EcParvulin was measured using the protease-coupled PPIase assay according to Fischer et al. (Fischer, G.; Bang, H.; Mech, C. Determination of enzymatic catalysis fort he cis-trans-isomerization of peptide binding in proline-containing peptides. [German] Biomed. Biochem. Acta 1984, 43, 1101-1111; Hennig et al., Selective Inactivation of Parvulin-like peptidyl-prolyl cis/trans isomerases by Juglon, Biochemistry. 1998, 37(17):5953-5960). For hPin1 measurements Ac-Ala-Ala-Ser($PO_3H_2$) -Pro-Arg-pNA was used as a substrate and trypsin (final concentration 190 μg/ml) as an isomer-specific protease. Activity measurements of other PPIases were made with the substrate peptide Suc-Ala-Phe-Pro-Phe-pNA and the protease □-chymotrypsin (final concentration 470 μg/ml). The assays were performed in a final reaction volume of 150 μl at final concentrations of 6 nM hPin1, 10 nM hCyp18, 5 nM LpCyp18, 20 nM EcParvulin and 20 nM hFKBP12, respectively, and 120 μM substrate peptide in 35 mM HEPES (pH 7.8). For inhibition experiments 100-0.01 μM of effector freshly diluted from a DMSO stock solution were added. The amount of solvent was kept constant within each experiment, usually below 0.3% (v/v). All reactions were started by addition of protease. The test was performed by observing the released 4-nitroaniline at 390 nm with a MR5000 UV/Vis spectrophotometer (Dynex) at 6°C. Data were evaluated by calculation of pseudo-first-order rate constants $k_{obs}$ in presence of PPIase and PPIase/effector, respectively, and corrected for the contribution of the non-catalyzed reaction ($k_0$). Inhibition constants $IC_{50}$ were calculated using SigmaPlot 8.0 (SPSS).

The following target enzymes which are all rotamases belonging to different classes of rotamases were used:

T-1: Protein interacting with NIMA (-kinase), hPin1
T-2: First described human Rapamycin receptor, hFKBP12
T-3: Human Cyclosporin A receptor with 18 kDa molecular weight, hCyp18
T-4: Leishmonia pneumophila virulence Cyclosporin A receptor with 18 kDa molecular weight, LpCyp18
T-5: Bacterial Juglon sensitive non proteolytic enzyme, EcParv

These rotamases are known in the art. Their production and characteristics may be taken from the following references.

**Review about all PPIase families**

Gothel, S. F.; Marahiel, M. A. TI Peptidyl-prolyl cis-trans isomerases, a superfamily of ubiquitous folding catalysts [Review]. Cell. Molec. Life Sci. 1999, 55, 423-436

**Pin1**

Lu, K. P.; Hanes, S. D.; Hunter, T. (1996) A human peptidyl-prolyl isomerase essential for regulation of mitosis.

*Nature* **1996,** *380,* 544-547

Yaffe, M. B.; Schutkowski, M.; Shen, M. H.; Zhou, X. Z.; Stukenberg, P. T.; Rahfeld, J. U.; Xu, J.; Kuang, J.; Kirschner, M. W.; Fischer, G.; Cantley, L. C.; Lu K. P. SEQUENCE-SPECIFIC AND PHOSPHORYLATION-DEPENDENT PROLINE ISOMERIZATION - A POTENTIAL MITOTIC REGULATORY MECHANISM. Science **1997**, *278*, 1957-1960

Shen, M.; Stukenberg, P. T.; Kirschner, M. W.; Lu, K. P. The essential mitotic peptidyl-prolyl isomerase Pin1 binds and regulates mitosis-specific phosphoproteins. *Genes Developm.* **1998**, *12*, 706-720.

**EcParvulin**

Rahfeld JU. Schierhom A. Mann K. Fischer G. A novel peptidyl-prolyl cis/trans isomerase from Escherichia coli. *FEBS Letters.* **1994,** *343*, 65-69

Rahfeld JU. Rucknagel KP. Schelbert B. Ludwig B. Hacker J. Mann K. Fischer G. Confirmation of the existence of a third family among peptidyl-prolyl cis/trans isomerases. Amino acid sequence and recombinant production of parvulin. *FEBS Letters.* **1994,** *352*, 180-184

**FKBPs (including FKBP12) and Cyclophilins (including Cyp18)**

For recent reviews on cyclophilins and FKBPs and their effectors, see: (a) Fischer, G. Peptidyl-prolyl *cis*/*trans* isomerases and their effectors. *Angew. Chem., Int. Ed. Engl.* **1994**, *33*, 1415-1436. (b) Galat, A.; Metcalfe, S. M. Peptidylproline *cis*/*trans* isomerases. Prog. *Biophys. Molec. Biol.* **1995**, *63*, 67-118.

**LpCyp18**

Schmidt B. Tradler T. Rahfeld JU. Ludwig B. Jain B. Mann K. Rucknagel KP. Janowski B. Schierhorn A. Kullertz G. Hacker J. Fischer G. A cyclophilin-like peptidyl-prolyl cis/trans isomerase from Legionella pneumophila--characterization, molecular cloning and overexpression. *Mol.* Microbiol. **1996**, *21*,1147-1160

In order to cluster the various rotamase inhibitors the following classes were defined with "A" indicating the most potent rotamase inhibitor.

A: IC50 < 1 μM
B: 1 μM < IC50 < 10 μM
C: 10 μM < IC50 < 50 μM
D: 50 μM < IC50 < 100 μM
E: IC50 > 100 μM

Table 2

Specificity of the inhibition with rotamases

| Compound | N° | Target | | | | |
|---|---|---|---|---|---|---|
| | | **T-1** | **T-2** | **T-3** | **T-4** | **T-5** |
| 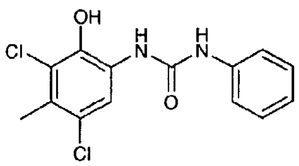 | 38 | C | D | C | E | |
| 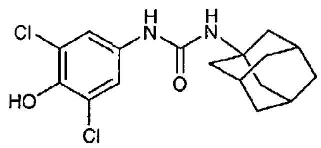 | 88 | A | E | D | C | |
| 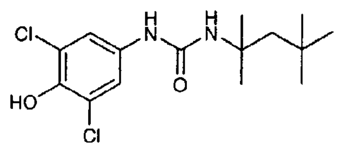 | 89 | A | E | E | E | B |
| 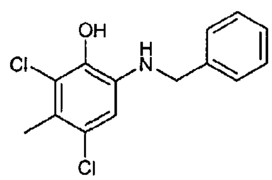 | 71 | C | E | E | E | C |
| 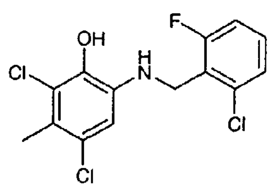 | 72 | C | E | D | E | C |
| 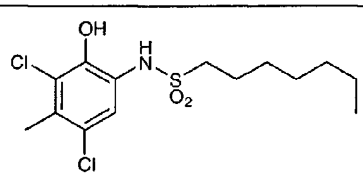 | 64 | B | E | E | E | E |
| 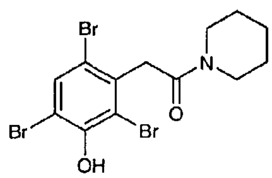 | 90 | C | E | E | E | C |

| Compound | N° | Target | | | | |
|---|---|---|---|---|---|---|
| | | T-1 | T-2 | T-3 | T-4 | T-5 |
| 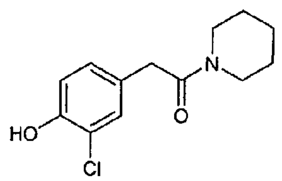 | 91 | B | E | E | E | C |
| 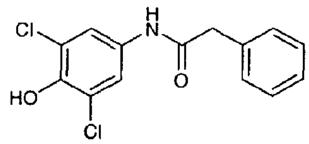 | 169 | A | E | E | E | C |
| 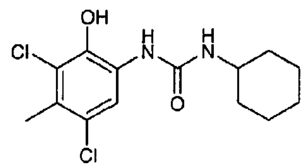 | 24 | A | E | C | E | C |
| 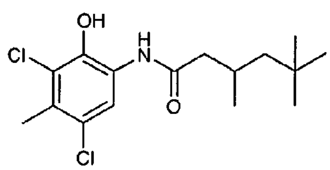 | 95 | B | E | E | D | C |
| 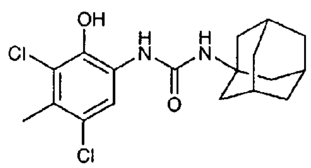 | 36 | B | D | C | C | B |
| 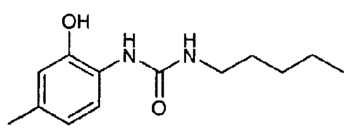 | 40 | C | E | E | E | E |
| 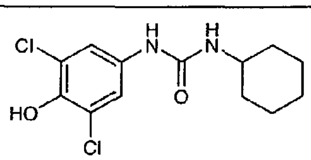 | 110 | A | E | E | E | E |
| 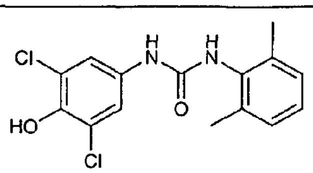 | 114 | B | E | E | E | C |
| 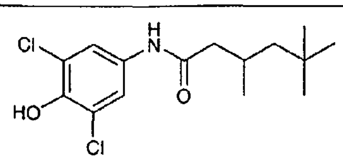 | 170 | A | E | E | E | B |

| Compound | N° | Target | | | | |
|---|---|---|---|---|---|---|
| | | T-1 | T-2 | T-3 | T-4 | T-5 |
| 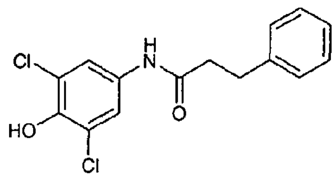 | 240 | B | E | E | E | C |
| 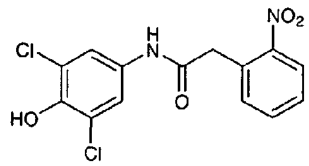 | 377 | A | E | E | E | B |
| 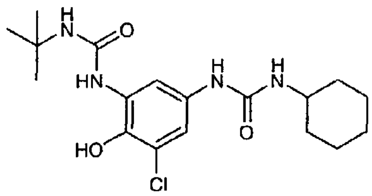 | 298 | A | E | C | E | B |
| 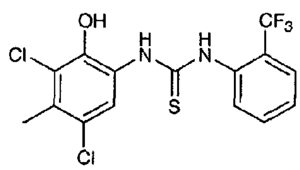 | 342 | A | E | E | E | B |
| 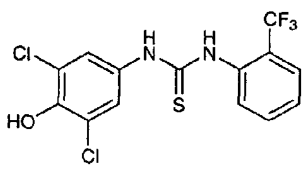 | 344 | A | E | E | E | B |
| 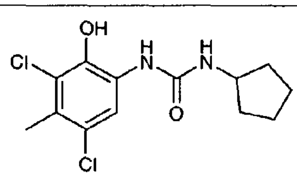 | 378 | A | E | C | E | B |
| 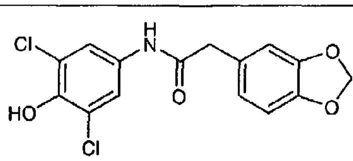 | 379 | B | E | E | E | B |
| 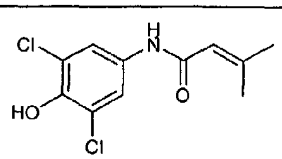 | 238 | B | E | C | E | C |

| Compound | N° | Target | | | | |
|---|---|---|---|---|---|---|
| | | T-1 | T-2 | T-3 | T-4 | T-5 |
| 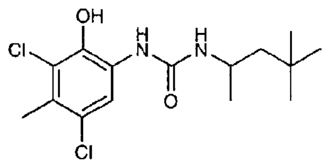 | 33 | B | E | C | C | B |

As may be taken from the above table 2 the following compounds 24, 88, 89, 110, 169, 170, 298, 342, 344, 377, 378 are of class A and are thus extremely specific for hPin1.

<u>Example 23:</u> Specificity of inhibition of proteases

In order to investigate the impact of some of the inventive compounds on the activity of key proteases the following assay was performed: Protease activities were measured spectrophotometrically at 30°C according to Schomburg and Salzmann (Schomburg, B.; Salzmann M. GBF: Enzyme Handbook. Springer Verlag, Berlin Heidelberg, 1991) and Bergmeyer et al. (Bergmeyer, H. U.; Bergmeyer, J.; Graß1, M. Methods of Enzymatic Analysis, Vol. V Enzymes 3: Peptides, Proteinases and Their Inhibitors. pp 55— 371, VCH, Weinheim, 1988). The release of 4-nitroaniline was determined at 390 nm with a Spectramax Plus UV/Vis spectrophotometer (Molecular Devices). The cathepsin B assay was performed in a reaction mixture containing 0.2 μg/ml cathepsin B, 2 mM Z-Arg-Arg-pNA in 88 mM $KH_2PO_4$, 12 mM $Na_2HPO_4$, 1.33 mM EDTA, 0.03% Brij 35 (pH 5.8). The trypsin assay was carried out in a reaction mixture containing 0.1 μg/ml trypsin and 120 μM Ac-Ala-Ala-Ser($PO_3H_2$) -Pro-Arg-pNA in 35 mM HEPES (pH 7.8) and the papain assay in a mixture consisting of 16 μg/ml papain and 2 mM Bz-DL-Arg-pNA in 10 mM $Na_2HPO_4$, 2 mM L-Cys, 5 mM EDTA (pH 6.5). In general, reactions were started by addition of peptide substrate after a 30 min incubation of 1-100 μM effector with given concentrations of enzyme.

The key proteases used were the following:

T-6: Papain
T-7: Trypsin
T-8: Cathepsin

In order to cluster the various rotamase inhibitors the following classes of activity were defined.

A: IC50 < 1 μM
B: 1 μM < IC50 < 10 μM
C: 10 μM < IC50 < 50 μM
D: 50 μM < IC50 < 100 μM
E: IC50 > 100 μM

Table 3

Specificity of the inhibition of some proteases

| Compound | N° | Target | | |
|---|---|---|---|---|
| | | T-6 | T-7 | T-8 |
| 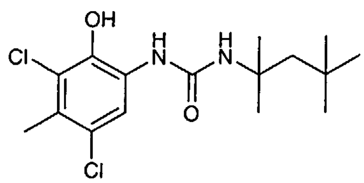 | 33 | D | C | D |
| 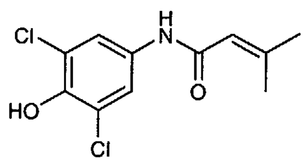 | 238 | E | E | E |
| 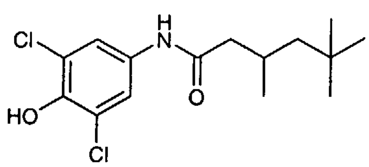 | 170 | E | E | E |
| 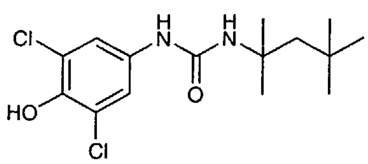 | 89 | E | D | E |
| 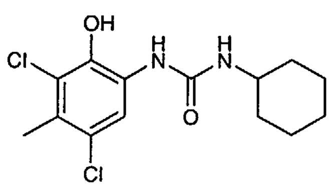 | 24 | E | | |

| Compound | N° | Target | | |
|---|---|---|---|---|
| | | T-6 | T-7 | T-8 |
| 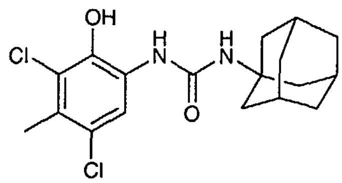 | 88 | E | | |
| 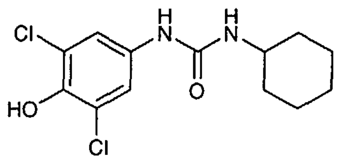 | 110 | E | | |
| 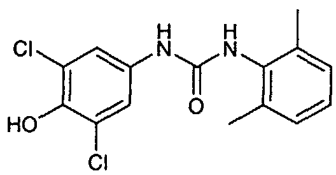 | 114 | E | | |

As may be taken from table 3 none of the tested compound is a strong inhibitor of any of the key proteases tested.

Example 24: Cytotoxic effects on tumor cell lines

In order to show that the compounds according to the present invention are actually useful in the treatment of tumors, the cytotoxic effects of some of said compounds on tumor cell lines were determined.

For this cytotoxic evaluation of the compounds the commercial available WST-1 assay (Roche) was used according to the manufacturer's instructions. The assay is based on the cleavage of the tetrazolium salt WST-1 by mitochondrial dehydrogenases found in viable cells. In general compounds were added to cells cultured in 96-well plates at 37°C. After 48 h of incubation 10 µl of WST-1 solution was added. The formazan dye was analyzed with an ELISA plate reader at (450 vs. 620) nm.

The following tumor cell lines were used in this assay:

CL-1: human acute myeloid leukemia, HL-60
CL-2: human cervix carcinoma, HeLa
CL-3: human prostate carcinoma, PC-3
CL-4: human colon adenocarcinoma, Caco-2
CL-5: human breast adenocarcinoma, MCF-7

In order to cluster the efficacy of the various compounds the following classes in terms of EC50 were defined.

A: EC50 < 10 µM
B: 10 µM < EC50 < 50 µM
C: 50 µM < EC50 < 100 µM
D: 100 µM < EC50 < 200 µM
E: EC50 > 200 µM

Table 4

Cytotoxic effects on tumor cell lines

| Compound | N° | Target | | | | |
|---|---|---|---|---|---|---|
| | | CL-1 | CL-2 | CL-3 | CL-4 | CL-5 |
| 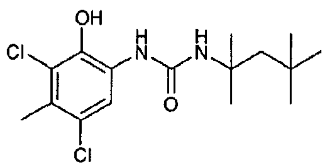 | 33 | A | A | A | A | |
| 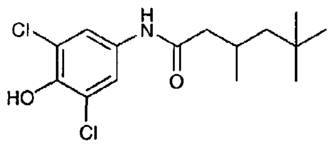 | 170 | B | C | B | B | |
| 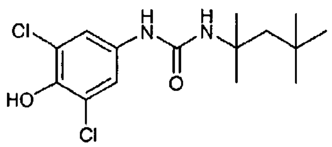 | 89 | B | B | B | B | |
| 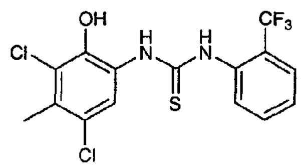 | 342 | A | B | B | B | |

As may be taken from table 4 all of the tested compounds are highly efficient in exhibiting a cytotoxic effect on at least one of the various tumor cell lines tested. Of particular relevance are compounds 33, 342.

The features of the present invention disclosed in the specification, the claims and/or the drawings may both separately and in any combination thereof be material for realizing the invention in various forms thereof.

Example 25: FACS measurements, and TUNEL assay of Compound 89

In order to show that the compounds according to the present invention are actually useful for inducing apoptosis in tumor cells, FACS measurements and TUNEL assay were performed as may be taken, e. g., from Enari M. Sakahira H. Yokoyama H. Okawa K. Iwamatsu A. Nagata S. A **caspase**-activated **DNase** that degrades DNA during apoptosis, and its inhibitor ICAD [erratum appears in Nature 1998 May 28;393(6683):396.]. *Nature. 391*:43-50, 1998, **Darzynkiewicz Z. Juan** G. Li X. Gorczyca W. Murakami T. Traganos F. Cytometry in cell necrobiology: analysis of apoptosis and accidental cell death (necrosis). Cytometry. *27:1-20*, *1997*.

Apoptotic HL-60 cells were detected by FACS analysis of FITC-dUTP-labelled DNA breaks using the Apo-Direct kit (BD-Pharmingen) according to the manufacturer's protocol.

As may be taken from Fig. 1 compound 89 induce apoptosis in tumor cells.

The features of the present invention disclosed in the specification, the claims and/or the drawing may both separately and in any combination thereof be material for realizing the invention in various forms thereof.

## Claims

1. Use of a compound as an inhibitor to a rotamase, whereby the compound has the structure:

A-X-Y **(I)**

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising
$-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $[(CR^aR^b)_n-CO-NR^c-(CR^aR^b)_m]_t-$, $-[(CR^aR^b)_n-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m)_t-$, $-L(CR^aR^b)_n-O-(CR^aR^b)_m]_t-$, $-[(CR^aR^b)_n-SR^c-(CR^aR^b)_m]_t-$;
wherein $X_2$ is selected from the group comprising
$-L(CR^aR^b)_n-CO-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-CO-NR^c-(CR^aR^b)_m]_t-$, $-[(CR^aR^b)_n-NR^c-CS-(CR^aR^b)_m]_t-$, $-[(CR^aR^b)_n-CS-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-SO-(CR^aR^b)_m]_t-$, $-[(CR^aR^b)_n-SO_2-(CR^aR^b)_m]_t-$, $-[(CR^aR^b)_n]_t-$;
wherein n and m are independently selected from each other and are any integer between 0 and 10 provided that if n is 0, m is different from 0, and if m is 0, n is different from 0;
wherein t is independently selected from n and/or m and is any integer between 0 and 10;
wherein
$R^a$, $R^b$, $R^c$, $R^d$ and $R^e$ are independently from each other selected from the group comprising H, alkyl, cycloalkyl, aryl, heteroaryl and heterocyclyl;
and wherein Y is selected from the group comprising alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide.

**2.** Use of a compound for the manufacture of a medicament for the treatment of a disease, whereby the disease involves an abnormal cell proliferation, an undesired cell proliferation, an abnormal mitosis and/or an undesired mitosis.
whereby the compound has the structure:

A-X-Y **(I)**

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising
$-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $-((CR^aR^b)_n-CO-NR^c-(CR^aR^b)_m]_t-$, $-[(CR^aR^b)_n-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m]_t-$, $-[(CR^aR^b)_n-O-(CR^aR^b)_m]_t-$, $-[(CR^aR^b)_n-SR^c-(CR^aR^b)_m]_t-$;
wherein $X_2$ is selected from the group comprising
$-[(CR^aR^b)_n-CO-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-CO-NR^c-(CR^aR^b)_m]_t-$, $-[(CR^aR^b)_n-NR^c-CS-(CR^aR^b)_m]_t-$, $-[(CR^aR^b)_n-CS-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-SO-(CR^aR^b)_m]_t$, $-[(CR^aR^b)_n-SO_2-(CR^aR^b)m]_t-$, $-[(CR^aR^b)_n]_t-$;
wherein n and m are independently selected from each other and are any integer between 0 and 10 provided that if n is 0, m is different from 0, and if m is 0, n is different from 0;
wherein t is independently selected from n and/or m and is any integer between 0 and 10;
wherein
$R^a$, $R^b$, $R^c$, $R^d$ and $R^e$ are independently from each other selected from the group comprising H, alkyl, cycloalkyl, aryl, heteroaryl and heterocyclyl;
and wherein Y is selected from the group comprising alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide.

**3.** The use according to claim 1 or 2, wherein A is

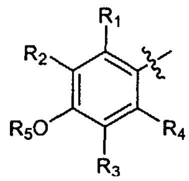

or

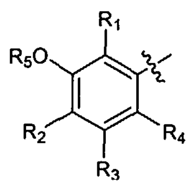

or

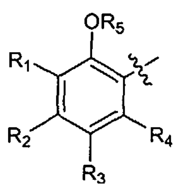

wherein $R_1$, $R_2$, $R_3$ and $R_4$ are each independently selected from the group comprising H, O, S, $NR^e$, halogen, alkyl, cycloalkyl, aryl, heterocyclyl and heteroaryl;
$R_5$ is selected from selected from the group comprising H, alkyl, cycloalkyl, aryl, heterocyclyl and heteroaryl; and
$R^e$ is selected from the group comprising H, alkyl, aryl, alkoxy, aryloxy, alkylamino and arylamino.

**4.** The use according to claims 1 to 3, wherein
$R_1$, $R_2$, $R_3$, $R_4$ and/or $R_5$ have independently from each other one or more groups of the formula $R^f$; whereby $R^f$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, alkanoylamino, aroylamino, alkylthio, arylthio, ureido and amine.

**5.** The use according to claim 4, wherein
the alkylthio group is derivatized, preferably the sulfur atom is oxidized to a sulfoxide or sulfone;
the arylthio group is derivatized, preferably the sulfur atom is oxidized to a sulfoxide or sulfone;
the ureido group is derivatized, preferably the nitrogen atom is independently mono- or di-substituted, more preferably the substitution is selected from the group comprising alkyl, aryl, heterocyclyl, heteroaryl, alkoxycarbonylamino, aryloxycarbonylamino, alkylcarbamoyloxy, arylcarbamoyloxy, alkylsulfonylamino, arylsulfonylamino, alkylaminosulfonyl, and arylaminosulfonyl; and/or
the amino group is derivatized, preferably the nitrogen atom is independently mono- or di-substituted by alkly, aryl, heterocyclyl, heteroalyl, halogen, hydroxy, oxo, carboxy, cyano, nitro, amidino and guanidino.

**6.** The use according to claim 4 or 5, whereby $R^f$ is further substituted by one ore more groups $R^g$, whereby $R^g$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

**7.** Use of a compound as inhibitor to a rotamase, whereby the compound is selected from the group comprising

3-[3-(5-Chloro-2-hydroxy-phenyl)-ureido]-propionic acid ethyl ester
1-(5-Chloro-2-hydroxy-phenyl)-3-pentyl-urea
1-(5-Chloro-2-hydroxy-phenyl)-3-(3,5-dichloro-phenyl)-urea
1-(5-Chloro-2-hydroxy-phenyl)-3-(4-chloro-phenyl)-urea
1-(5-Chloro-2-hydroxy-phenyl)-3-cyclohexyl-urea
1-(5-Chloro-2-hydroxy-phenyl)-3-(4-trifluoromethoxy-phenyl)-urea
1-(5-Chloro-2-hydroxy-phenyl)-3-(4-cyano-phenyl)-urea
1-Benzo[ 1,3]dioxol-5-yl-3-(5-chloro-2-hydroxy-phenyl)-urea
1-Benzyl-3-(5-chloro-2-hydroxy-phenyl)-urea
1-( 5-Chloro-2-hydroxy-phenyl)-3-o-tolyl-urea
1-(5-Chloro-2-hydroxy-phenyl)-3-(3-methoxy-phenyl)-urea
1-(5-Chloro-2-hydroxy-phenyl)-3-(2,6-dimethyl-phenyl)-urea
1-(5-Chloro-2-hydroxy-phenyl)-3-phenethyl-urea
1-(5-Chloro-2-hydroxy-phenyl)-3-(1,1,3,3-tetramethyl-butyl)-urea
1-(5-Chloro-2-hydroxy-phenyl)-3-(3,4,5-trimethoxy-phenyl)-urea
1-(5-Chloro-2-hydroxy-phenyl)-3-naphthalen-1-yl-urea
1-Adamantan-1-yl-3-(5-chloro-2-hydroxy-phenyl)-urea
1-(5-Chloro-2-hydroxy-phenyl)-3-(4-phenoxy-phenyl)-urea
1-(5-Chloro-2-hydroxy-phenyl)-3-phenyl-urea
3-[3-(3,5-Dichloro-2-hydroxy-4-methyl-phenyl)-ureido]-propionic acid ethyl ester
1-(3,5-Dichloro-2-hydroxy-4-methyl-phenyl)-3-pentyl-urea
1-(3,5-Dichloro-2-hydroxy-4-methyl-phenyl)-3-(3,5-dichloro-phenyl)-urea
1-(4-Chloro-phenyl)-3-(3,5-dichloro-2-hydroxy-4-methyl-phenyl)-urea
1-Cyclohexyl-3-(3,5-dichloro-2-hydroxy-4-methyl-phenyl)-urea
1-(3,5-Dichloro-2-hydroxy-4-methyl-phenyl)-3-(4-trifluoromethoxy-phenyl)-urea
1-(4-Cyano-phenyl)-3-(3,5-dichloro-2-hydroxy-4-methyl-phenyl)-urea
1-Benzo[1,3 ]dioxol-5-yl-3-(3,5-dichloro-2-hydroxy-4-methyl-phenyl)-urea
1-Benzyl-3-(3,5-dichloro-2-hydroxy-4-methyl-phenyl)-urea
1-(3,5-Dichloro-2-hydroxy-4-methyl-phenyl)-3-o-tolyl-urea
1-(3,5-Dichloro-2-hydroxy-4-methyl-phenyl)-3-(3-methoxy-phenyl)-urea
1-(3,5-Dichloro-2-hydroxy-4-methyl-phenyl)-3-(2,6-dimethyl-phenyl)-urea
1-(3,5-Dichloro-2-hydroxy-4-methyl-phenyl)-3-phenethyl-urea
1-(3,5-Dichloro-2-hydroxy-4-methyl-phenyl)-3-( 1,1,3,3-tetramethyl-butyl)-urea
1-(3,5-Dichloro-2-hydroxy-4-methyl-phenyl)-3-(3,4,5-trimethoxy-phenyl)-urea
1-(3,5-Dichloro-2-hydroxy-4-methyl-phenyl)-3-naphthalen-1-yl-urea
1-Adamantan-1-yl-3-(3,5-dichloro-2-hydroxy-4-methyl-phenyl)-urea
1-(3,5-Dichloro-2-hydroxy-4-methyl-phenyl)-3-(4-phenoxy-phenyl)-urea
1-(3,5-Dichloro-2-hydroxy-4-methyl-phenyl)-3-phenyl-urea
3-[3-(2-Hydroxy-4-methyl-phenyl)-ureido]-propionic acid ethyl ester
1-(2-Hydroxy-4-methyl-phenyl)-3-pentyl-urea
1-(3,5-Dichloro-phenyl)-3-(2-hydroxy-4-methyl-phenyl)-urea
1-(4-Chloro-phenyl)-3-(2-hydroxy-4-methyl-phenyl)-urea
1-Cyclohexyl-3-(2-hydroxy-4-methyl-phenyl)-urea
1-(2-Hydroxy-4-methyl-phenyl)-3-(4-trifluoromethoxy-phenyl)-urea
1-(4-Cyano-phenyl)-3-(2-hydroxy-4-methyl-phenyl)-urea
1-Benzo[1,3]dioxol-5-yl-3-(2-hydroxy-4-methyl-phenyl)-urea
1-Benzyl-3 -(2-hydroxy-4-methyl-phenyl)-urea
1-(2-Hydroxy-4-methyl-phenyl)-3-o-tolyl-urea
1-(2-Hydroxy-4-methyl-phenyl)-3-(3-methoxy-phenyl)-urea
1-(2,6-Dimethyl-phenyl)-3-(2-hydroxy-4-methyl-phenyl)-urea
1-(2-Hydroxy-4-methyl-phenyl)-3-phenethyl-urea
1-(2-Hydroxy-4-methyl-phenyl)-3-(1,1,3,3-tetramethyl-butyl)-urea

1-(2-Hydroxy-4-methyl-phenyl)-3-(3,4,5-trimethoxy-phenyl)-urea
1-(2-Hydroxy-4-methyl-phenyl)-3-naphthalen-1-yl-urea
1-Adamantan-1-yl-3-(2-hydroxy-4-methyl-phenyl)-urea
1-(2-Hydroxy-4-methyl-phenyl)-3-(4-phenoxy-phenyl)-urea
1-(2-Hydroxy-4-methyl-phenyl)-3-phenyl-urea
N-(2-Hydroxy-phenyl)-C-phenyl-methanesulfonamide
N-(4-Hydroxy-phenyl)-C-phenyl-methanesulfonamide
N-(5-Chloro-2-hydroxy-phenyl)-C-phenyl-methanesulfonamide
N-(2-Hydroxy-4-methyl-phenyl)-C-phenyl-methanesulfonamide
N-(3,5-Dichloro-2-hydroxy-4-methyl-phenyl)-C-phenyl-methanesulfonamide
Butane-1-sulfonic acid (3,5-dichloro-2-hydroxy-4-methyl-phenyl)-amide
Octane-1-sulfonic acid (3,5-dichloro-2-hydroxy-4-methyl-phenyl)-amide
Propane-2-sulfonic acid (3,5-dichloro-2-hydroxy-4-methyl-phenyl)-amide
1-(3,5-Dichloro-2-hydroxy-phenyl)-3-(1,1,3,3-tetramethyl-butyl)-urea
1-(3-Chloro-2-hydroxy-phenyl)-3-(1,1,3,3-tetramethyl-butyl)-urea
1-Adamantan-1-yl-3-(3,5-dichloro-2-hydroxy-phenyl)-urea
1-Adamantan-1-yl-3-(3-chloro-2-hydroxy-phenyl)-urea
6-Benzylamino-2,4-dichloro-3-methyl-phenol
2,4-Dichloro-6-(2-chloro-6-fluoro-benzylamino)-3-methyl-phenol
2,4-Dichloro-3-methyl-6-(3-methyl-benzylamino)-phenol
2,4-Dichloro-6-(3,4-dimethoxy-benzylamino)-3-methyl-phenol
N-(3-Chloro-2-hydroxy-phenyl)-2-trifluoromethyl-benzenesulfonamide
4-Acetyl-N-(3-chloro-2-hydroxy-phenyl)-benzenesulfonamide
2,4-Dichloro-6-[2-(4-chloro-phenylsulfanyl)-benzylamino]-3-methyl-phenol
6-[(Biphenyl-2-ylmethyl)-amino]-2,4-dichloro-3-methyl-phenol
4-[(3,5-Dichloro-2-hydroxy-4-methyl-phenylamino)-methyl]-benzonitrile
4-[(3,5-Dichloro-2-hydroxy-4-methyl-phenylamino)-methyl]-benzoic acid methyl ester
6-[(5-Bromo-thiophen-2-ylmethyl)-amino]-2,4-dichloro-3-methyl-phenol
2,4-Dichloro-3-methyl-6-[(3-methyl-thiophen-2-ylmethyl)-amino]-phenol
2,4-Dichloro-3-methyl-6-[(5-methyl-furan-2-ylmethyl)-amino]-phenol
1-Adamantan-1-yl-3-(2, 6-dibromo-3-chloro-4-methyl-phenyl)-urea
1-(2,6-Dibromo-3-chloro-4-methyl-phenyl)-3-(1,1,3,3-tetramethyl-butyl)-urea
1-Adamantan-1-yl-3-(3-chloro-2-cyano-phenyl)-urea
1-(3-Chloro-2-cyano-phenyl)-3-(1,1,3,3-tetramethyl-butyl)-urea
1-Adamantan-1-yl-3-(3-chloro-4-hydroxy-phenyl)-urea
1-(3-Chloro-4-hydroxy-phenyl)-3-(1,1,3,3-tetramethyl-butyl)-urea
1-Adamantan-1-yl-3-(3,5-dichloro-4-hydroxy-phenyl)-urea
1-(3,5-Dichloro-4-hydroxy-phenyl)-3-(1,1,3,3-tetramethyl-butyl)-urea
1-Piperidin-1-yl-2-(2,4,6-tribromo-3-hydroxy-phenyl)-ethanone
2-(3-Chloro-4-hydroxy-phenyl)-1-piperidin-1-yl-ethanone
1-Pyrrolidin-1-yl-2-(2,4,6-tribromo-3-hydroxy-phenyl)-ethanone
2-(3-Chloro-4-hydroxy-phenyl)-1-pyrrolidin-1-yl-ethanone
N-(3,5-Dichloro-2-hydroxy-4-methyl-phenyl)-N-methyl-2-trifluoromethyl-benzamide
3,5,5-Trimethyl-hexanoic acid (3,5-dichloro-2-hydroxy-4-methyl-phenyl)-amide
2-Chloro-6-[3-(1,1,3,3-tetramethyl-butyl)-ureido]-benzoic acid
3-Adamantan-1-yl-1-(3,5-dichloro-2-hydroxy-4-methyl-phenyl)-1-methyl-urea
1-(3,5-Dichloro-2-hydroxy-4-methyl-phenyl)-1-methyl-3-(1,1,3,3-tetramethyl-butyl)-urea
1-Adamantan-1-yl-3-(5-bromo-3-fluoro-2-hydroxy-phenyl)-urea
1-(5-Bromo-3-fluoro-2-hydroxy-phenyl)-3-(1,1,3,3-tetramethyl-butyl)-urea
1-(5-Bromo-3-fluoro-2-hydroxy-phenyl)-3-tert-butyl-urea
1-Adamantan-1-yl-3-(3,4-difluoro-2-hydroxy-phenyl)-urea
1-(3,4-Difluoro-2-hydroxy-phenyl)-3-(1,1,3,3-tetramethyl-butyl)-urea
1-tert-Butyl-3-(3,4-difluoro-2-hydroxy-phenyl)-urea
1-Adamantan-1-yl-3-(3-fluoro-4-hydroxy-phenyl)-urea
1-(3-Fluoro-4-hydroxy-phenyl)-3-( 1,1,3,3-tetramethyl-butyl)-urea
1-tert-Butyl-3-(3-fluoro-4-hydroxy-phenyl)-urea
1-tert-Butyl-3-(3,5-dichloro-4-hydroxy-phenyl)-urea
1-(3,5-Dichloro-4-hydroxy-phenyl)-3-(2-phenyl-cyclopropyl)-urea

1-Cyclohexyl-3-(3,5-dichloro-4-hydroxy-phenyl)-urea
1-Cyclopentyl-3-(3,5-dichloro-4-hydroxy-phenyl)-urea
1-(3,5-Dichloro-4-hydroxy-phenyl)-3-pentyl-urea
1-(2-Chloro-6-trifluoromethyl-phenyl)-3-(3,5-dichloro-4-hydroxy-phenyl)-urea
1-(3,5-Dichloro-4-hydroxy-phenyl)-3-(2,6-dimethyl-phenyl)-urea
1-(3,5-Dichloro-4-hydroxy-phenyl)-3-phenethyl-urea
1-(3,5-Dichloro-4-hydroxy-phenyl)-3-naphthalen-2-yl-urea
1-Biphenyl-2-yl-3-(3,5-dichloro-4-hydroxy-phenyl)-urea
1-tert-Butyl-3-(3,5-dichloro-2-hydroxy-4-methyl-phenyl)-urea
1-tert-Butyl-3-(3,5-dichloro-2-hydroxy-phenyl)-urea
1-tert-Butyl-3-(3-chloro-4-hydroxy-phenyl)-urea
1-Adamantan-1-yl-3-(3,5-dibromo-4-hydroxy-phenyl)-urea
1-(3,5-Dibromo-4-hydroxy-phenyl)-3-(1,1,3,3-tetramethyl-butyl)-urea
1-tert-Butyl-3-(3,5-dibromo-4-hydroxy-phenyl)-urea
1-(3,5-Dibromo-4-hydroxy-phenyl)-3-(2-phenyl-cyclopropyl)-urea
1-(3,5-Dibromo-4-hydroxy-phenyl)-3-pentyl-urea
1-Cyclohexyl-3-(3, 5-dibromo-4-hydroxy-phenyl)-urea
1-Adamantan-1-yl-3-(3-hydroxy-4-methoxy-phenyl)-urea 1-(3-Hydroxy-4-methoxy-phenyl)-3-(1,1,3,3-tetramethyl-butyl)-urea
1-tert-Butyl-3-(3-hydroxy-4-methoxy-phenyl)-urea
1-(3-Hydroxy-4-methoxy-phenyl)-3-(2-phenyl-cyclopropyl)-urea
1-(3-Hydroxy-4-methoxy-phenyl)-3-pentyl-urea
1-Cyclohexyl-3-(3-hydroxy-4-methoxy-phenyl)-urea
1-Adamantan-1-yl-3-(2,4-dihydroxy-phenyl)-urea
1-(2,4-Dihydroxy-phenyl)-3-(1,1,3,3-tetramethyl-butyl)-urea
1-tert-Butyl-3-(2,4-dihydroxy-phenyl)-urea
1-(2,4-Dihydroxy-phenyl)-3-(2-phenyl-cyclopropyl)-urea
1-(2,4-Dihydroxy-phenyl)-3-pentyl-urea
1-Cyclohexyl-3-(2,4-dihydroxy-phenyl)-urea
1-Adamantan-1-yl-3-(2'-hydroxy-[1,1';3',1"]terphenyl-5'-yl)-urea
1-(2'-Hydroxy-[1,1';3',1"]terphenyl-5'-yl)-3-(1,1,3,3-tetramethyl-butyl)-urea
1-tert-Butyl-3-(2'-hydroxy-[1,1';3',1"]terphenyl-5'-yl)-urea
1-(2'-Hydroxy-[1,1';3',1"]terphenyl-5'-yl)-3-(2-phenyl-cyclopropyl)-urea
1-(2'-Hydroxy-[1,1';3',1"]terphenyl-5'-yl)-3-pentyl-urea
1-Cyclohexyl-3-(2'-hydroxy-[1,1;3',1"]terphenyl-5'-yl)-urea
1-Adamantan-1-yl-3-(3-chloro-4-methoxy-phenyl)-urea
1-(3-Chloro-4-methoxy-phenyl)-3-(1,1,3,3-tetramethyl-butyl)-urea
1-tert-Butyl-3-(3-chloro-4-methoxy-phenyl)-urea
1-(3-Chloro-4-methoxy-phenyl)-3-(2-phenyl-cyclopropyl)-urea
1-(3-Chloro-4-methoxy-phenyl)-3-pentyl-urea
1-(3-Chloro-4-methoxy-phenyl)-3-cyclohexyl-urea
1-Adamantan-1-yl-3-(4-hydroxy-3-methoxy-benzyl)-urea
1-(4-Hydroxy-3-methoxy-benzyl)-3-(1,1,3,3-tetramethyl-butyl)-urea
1-tert-Butyl-3-(4-hydroxy-3-methoxy-benzyl)-urea
1-(4-Hydroxy-3-methoxy-benzyl)-3-(2-phenyl-cyclopropyl)-urea
1-(4-Hydroxy-3-methoxy-benzyl)-3-pentyl-urea
1-Cyclohexyl-3-(4-hydroxy-3-methoxy-benzyl)-urea
1-Adamantan-1-yl-3-(4-hydroxy-naphthalen-1-yl)-urea
1-(4-Hydroxy-naphthalen-1-yl)-3-(1,1,3,3-tetramethyl-butyl)-urea
1-tert-Butyl-3-(4-hydroxy-naphthalen-1-yl)-urea
1-(4-Hydroxy-naphthalen-1-yl)-3-(2-phenyl-cyclopropyl)-urea
1-(4-Hydroxy-naphthalen-1-yl)-3-pentyl-urea
1-Cyclohexyl-3-(4-hydroxy-naphthalen-1-yl)-urea
1-Adamantan-1-yl-3-[4-(2-hydroxy-ethyl)-phenyl]-urea
1-[4-(2-Hydroxy-ethyl)-phenyl]-3-(1,1,3,3-tetramethyl-butyl)-urea
1-tert-Butyl-3-[4-(2-hydroxy-ethyl)-phenyl]-urea
1-[4-(2-Hydroxy-ethyl)-phenyl]-3-(2-phenyl-cyclopropyl)-urea
1-[4-(2-Hydroxy-ethyl)-phenyl]-3-pentyl-urea

1-Cyclohexyl-3-[4-(2-hydroxy-ethyl)-phenyl]-urea
N-(3,5-Dichloro-4-hydroxy-phenyl)-2-phenyl-acetamide
3,5,5-Trimethyl-hexanoic acid (3,5-dichloro-4-hydroxy-phenyl)-amide
Octane-1-sulfonic acid (3,5-dichloro-4-hydroxy-phenyl)-amide
(3,5-Dichloro-2-hydroxy-4-methyl-phenyl)-carbamic acid 2-isopropyl-5-methyl-cyclohexy ester
(3,5-Dichloro-2-hydroxy-4-methyl-phenyl)-carbamic acid hexyl ester
(3,5-Dichloro-4-hydroxy-phenyl)-carbamic acid phenyl ester
3-[3-(5-Chloro-2-hydroxy-phenyl)-ureido]-propionic acid ethyl ester
4-Bromo-3,5-dihydroxy-N-(1,1,3,3-tetramethyl-butyl)-benzamide
N-Benzyl-4-chloro-3-hydroxy-benzamide
N-Benzyl-3,5-dihydroxy-benzamide
2,6-Dichloro-4-(2-methyl-thiazol-4-yl)-phenol
2,6-Dichloro-4-(2-phenyl-thiazol-4-yl)-phenol
2,6-Dichloro-4-piperidin-1-ylmethyl-phenol
2,6-Dichloro-4-[2-(4-chloro-phenylsulfanyl)-benzylamino]-phenol
3,5-Dichloro-4-hydroxy-N-(1,1,3,3-tetramethyl-butyl)-benzamide
2-(3-Chloro-4-hydroxy-phenyl)-N-(1,1,3,3-tetramethyl-butyl)-acetamide
4-(2-Benzyl-thiazol-4-yl)-2,6-dichloro-phenol
N-Adamantan-1-yl-2-(3,5-dichloro-4-hydroxy-phenyl)-acetamide
2-(3,5-Dichloro-4-hydroxy-phenyl)-N-phenethyl-acetamide
N-Cyclohexyl-2-(3,5-dichloro-4-hydroxy-phenyl)-acetamide
2-(3,5-Dichloro-4-hydroxy-phenyl)-N-indan-1-yl-acetamide
2-(3,5-Dichloro-4-hydroxy-phenyl)-N-(2-hydroxy-2-phenyl-ethyl)-acetamide
2-(3,5-Dichloro-4-hydroxy-phenyl)-N-(1,5-dimethyl-hexyl)-acetamide
2-(3,5 -Dichloro-4-hydroxy-phenyl)-N-(3 -methyl-butyl)-acetamide
2-(3,5-Dichloro-4-hydroxy-phenyl)-N-(3,3-diphenyl-propyl)-acetamide
1-[2-(3,5-Dichloro-4-hydroxy-phenyl)-acetyl]-3-(3-dimethylamino-propyl)-1-ethyl-urea
2-(3-Fluoro-4-hydroxy-phenyl)-N-(1,1,3,3-tetramethyl-butyl)-acetamide
2-(3-Fluoro-4-hydroxy-phenyl)-1-piperidin-1-yl-ethanone
2,6-Dichloro-4-(2-phenethyl-thiazol-4-yl)-phenol
2,6-Dichloro-4-[2-(2,2-dimethyl-propyl)-thiazol-4-yl]-phenol
2-(3,5-Dichloro-4-hydroxy-phenyl)-1-piperidin-1-yl-ethanone
2-(3,5-Dichloro-4-hydroxy-phenyl)-N-[2-(4-fluoro-phenyl)-1,1-dimethyl-ethyl]-acetamide
2-Cyano-N-cyclohexyl-2-(3,5-dichloro-4-hydroxy-phenyl)-acetamide
2,6-Dichloro-4-[2-(2,4,4-trimethyl-pentyl)-thiazol-4-yl]-phenol
2,6-Dichloro-4-[2-(1-methyl-butyl)-thiazol-4-yl]-phenol
2,6-Dichloro-4-(2-cyclopentylmethyl-thiazol-4-yl)-phenol
2,6-Dichloro-4-[2-(2-cyclopentyl-ethyl)-thiazol-4-yl]-phenol
4-(2-tert-Butyl-thiazol-4-yl)-2,6-dichloro-phenol
2,6-Dichloro-4-(2-thiophen-2-ylmethyl-thiazol-4-yl)-phenol
2,6-Dichloro-4-(2-phenoxymethyl-thiazol-4-yl)-phenol
2-Cyano-2-(3,5-dichloro-4-hydroxy-phenyl)-N-phenethyl-acetamide
1-tert-Butyl-3-[3-chloro-2-hydroxy-5-(2-oxo-2-piperidin-1-yl-ethyl)-phenyl]-urea
2-[3-(3-tert-Butyl-ureido)-5-chloro-4-hydroxy-phenyl]-N-cyclohexyl-acetamide
2-[3-(3-tert-Butyl-ureido)-5-chloro-4-hydroxy-phenyl]-N-(1,1,3,3-tetramethyl-butyl)-acetamide
2-[3-(3-tert-Butyl-ureido)-5-chloro-4-hydroxy-phenyl]-N-phenethyl-acetamide
2-(3-Acetyl-2,2-dimethyl-cyclobutyl)-N-(3,5-dichloro-4-hydroxy-phenyl)-acetamide
2-Bicyclo[2.2.1]hept-2-yl-N-(3,5-dichloro-4-hydroxy-phenyl)-acetamide
2-Propyl-pentanoic acid (3,5-dichloro-4-hydroxy-phenyl)-amide
2-Cyano-N-(3,5-dichloro-4-hydroxy-phenyl)-acetamide
N-(3,5-Dichloro-4-hydroxy-phenyl)-2-(3-hydroxy-4,7,7-trimethyl-bicyclo[2.2.1]hept-2-yl)-acetamide
2,2-Dicyclohexyl-N-(3,5-dichloro-4-hydroxy-phenyl)-acetamide
4-[2,4-Bis-(1,1-dimethyl-propyl)-phenoxy]-N-(3,5-dichloro-4-hydroxy-phenyl)-butyramide
N-(3,5-Dichloro-4-hydroxy-phenyl)-3-phenyl-acrylamide
2-Methyl-pentanoic acid (3,5-dichloro-4-hydroxy-phenyl)-amide
N-(3,5-Dichloro-4-hydroxy-phenyl)-2-phenoxy-acetamide
N-(3,5-Dichloro-4-hydroxy-phenyl)-malonamic acid ethyl ester
N-(3,5-Dichloro-4-hydroxy-phenyl)-succinamic acid ethyl ester

N-(3,5-Dichloro-4-hydroxy-phenyl)-3-(3-trifluoromethyl-phenyl)-acrylamide
3-Cyclopentyl-N-(3,5-dichloro-4-hydroxy-phenyl)-propionamide
N-(3,5-Dichloro-4-hydroxy-phenyl)-2,2-diphenyl-acetamide
Acetic acid 1-(3,5-dichloro-4-hydroxy-phenylcarbamoyl)-ethyl ester
N-(3,5-Dichloro-4-hydroxy-phenyl)-2-(2-isopropyl-5-methyl-cyclohexyloxy)-acetamide
N-(3,5-Dichloro-4-hydroxy-phenyl)-3,3-dimethyl-butyramide
3,5,5-Trimethyl-hexanoic acid (3-chloro-4-hydroxy-phenyl)-amide
3,5,5-Trimethyl-hexanoic acid (5-bromo-3-fluoro-2-hydroxy-phenyl)-amide
3,5,5-Trimethyl-hexanoic acid (3,5-dibromo-4-hydroxy-phenyl)-amide
3,5,5-Trimethyl-hexanoic acid (2,4-dihydroxy-phenyl)-amide
3,5,5-Trimethyl-hexanoic acid 4-hydroxy-3-methoxy-benzylamide
Adamantane-1-carboxylic acid 4-hydroxy-3-methoxy-benzylamide
N-(4-Hydroxy-3-methoxy-benzyl)-2-trifluoromethyl-benzamide
N-(3,5-Dichloro-4-hydroxy-phenyl)-2-thiophen-2-yl-acetamide
N-(3,5-Dichloro-4-hydroxy-phenyl)-acetamide
2-Cyclopentyl-N-(3,5-dichloro-4-hydroxy-phenyl)-acetamide
3-Methyl-but-2-enoic acid (3,5-dichloro-4-hydroxy-phenyl)-amide
N-(3,5-Dichloro-4-hydroxy-phenyl)-2-phenyl-acetamide
N-(3,5 -Dichloro-4-hydroxy-phenyl)-3 -phenyl-propionamide
3-Bromo-2-hydroxy-5-(3,5,5-trimethyl-hexanoylamino)-benzoic acid
4-Methyl-pentanoic acid (3,5-dichloro-4-hydroxy-phenyl)-amide
3,7-Dimethyl-oct-6-enoic acid (3,5-dichloro-4-hydroxy-phenyl)-amide
2-Adamantan-1-yl-N-(3,5-dichloro-4-hydroxy-phenyl)-acetamide
3-Cyclohexyl-N-(3,5-dichloro-4-hydroxy-phenyl)-propionamide
N-(3,5-Dichloro-4-hydroxy-phenyl)-2-(2-fluoro-phenyl)-acetamide
N-(3,5-Dichloro-4-hydroxy-phenyl)-3-hydroxy-3-phenyl-propionamide
N-[(3,5-Dichloro-4-hydroxy-phenylcarbamoyl)-methyl]-benzamide
3-(3,5-Dichloro-4-hydroxy-phenyl)-1,1-diethyl-urea
3-(3,5-Dichloro-4-hydroxy-phenyl)-1,1-diisopropyl-urea
1-(3,5-Dichloro-4-hydroxy-phenyl)-3-(3-hydroxy-propyl)-urea
1-(3,5-Dichloro-4-hydroxy-phenyl)-3-(2-morpholin-4-yl-ethyl)-urea
1-(3,5-Dichloro-4-hydroxy-phenyl)-3-(3-isopropoxy-propyl)-urea
1-(1-Aza-bicyclo[2.2.2]oct-3-yl)-3-(3,5-dichloro-4-hydroxy-phenyl)-urea
1-(3,5-Dichloro-4-hydroxy-phenyl)-3-(2-piperidin-1-yl-ethyl)-urea
1-Adamantan-2-yl-3-(3,5-dichloro-4-hydroxy-phenyl)-urea
1-Adamantan-1-yl-3-(4-hydroxy-3-morpholin-4-ylmethyl-phenyl)-urea
1-(4-Hydroxy-3-morpholin-4-ylmethyl-phenyl)-3-(1,1,3,3-tetramethyl-butyl)-urea
1-(4-Hydroxy-3-morpholin-4-ylmethyl-phenyl)-3-(2-trifluoromethyl-phenyl)-urea
1-(3,5-Dichloro-4-hydroxy-phenyl)-3-(2-hydroxy-2-phenyl-ethyl)-urea
1-(3,5-Dichloro-4-hydroxy-phenyl)-3-[2-hydroxy-1-hydroxymethyl-2-(4-nitro-phenyl)-ethyl]-urea
1-(3,5-Dichloro-4-hydroxy-phenyl)-3-[2-(3,4-dimethoxy-phenyl)-ethyl]-urea
2-[3-(3,5-Dichloro-4-hydroxy-phenyl)-ureido]-3-phenyl-propionamide
1-(3,5-Dichloro-4-hydroxy-phenyl)-3-[2-(4-hydroxy-phenyl)-ethyl]-urea 1-(3,5-Dichloro-4-hydroxy-phenyl)-3-indan-2-yl-urea
1-(3,5-Dichloro-4-hydroxy-phenyl)-3-(3-phenyl-propyl)-urea
1-(3,5-Dichloro-4-hydroxy-phenyl)-3-indan-1-yl-urea
1-(3,5-Dichloro-4-hydroxy-phenyl)-3-(2-pyridin-2-yl-ethyl)-urea
1-(3,5-Dichloro-4-hydroxy-phenyl)-3-(2-pyridin-3-yl-ethyl)-urea
2-[3-(3,5-Dichloro-4-hydroxy-phenyl)-ureido]-4-methyl-pentanoic acid ethyl ester
2-[3-(3,5-Dichloro-4-hydroxy-phenyl)-ureido]-3-phenyl-propionic acid ethyl ester
2-[3-(3,5-Dichloro-2-hydroxy-4-methyl-phenyl)-ureido]-4-methyl-pentanoic acid ethyl ester
2-[3-(3,5-Dichloro-2-hydroxy-4-methyl-phenyl)-ureido]-3-phenyl-propionic acid ethyl ester
1-Adamantan-1-yl-3-(3,5-difluoro-4-hydroxy-phenyl)-urea
1-(3,5-Difluoro-4-hydroxy-phenyl)-3-(1,1,3,3-tetramethyl-butyl)-urea
1-(3,5-Difluoro-4-hydroxy-phenyl)-3-(2-trifluoromethyl-phenyl)-urea
1-tert-Butyl-3-(3,5-difluoro-4-hydroxy-phenyl)-urea
1-Adamantan-1-yl-3-(2-hydroxy-phenyl)-urea
1-(2-Hydroxy-phenyl)-3-(1,1,3,3-tetramethyl-butyl)-urea

1-(2-Hydroxy-phenyl)-3-(2-trifluoromethyl-phenyl)-urea
1-Adamantan-1-yl-3-(3-hydroxy-phenyl)-urea
1-(3-Hydroxy-phenyl)-3-(1,1,3,3-tetramethyl-butyl)-urea
1-(3-Hydroxy-phenyl)-3-(2-trifluoromethyl-phenyl)-urea
1-Adamantan-1-yl-3-(3-hydroxy-phenyl)-urea
1-(3-Hydroxy-phenyl)-3-(1,1,3,3-tetramethyl-butyl)-urea
1-(3-Hydroxy-phenyl)-3-(2-trifluoromethyl-phenyl)-urea
1,3-Bis-(3,5-dichloro-4-hydroxy-phenyl)-urea
1,3-Di-adamantan-1-yl-urea
1,3-Bis-(1,1,3,3-tetramethyl-butyl)-urea
3-Adamantan-1-yl-1-(3,5-dibromo-4-hydroxy-benzyl)-1-(3,5-dichloro-2-hydroxy-4-methylphenyl)-urea
1-(3,5-Dibromo-4-hydroxy-benzyl)-1-(3,5-dichloro-2-hydroxy-4-methyl-phenyl)-3-(1,1,3,3-tetramethyl-butyl)-urea
1-(3,5-Dibromo-4-hydroxy-benzyl)-1-(3,5-dichloro-2-hydroxy-4-methyl-phenyl)-3-(2-trifluoromethyl-phenyl)-urea
3-tert-Butyl-1-(3,5-dibromo-4-hydroxy-benzyl)-1-(3,5-dichloro-2-hydroxy-4-methyl-phenyl)-urea
1-(3,5-Dichloro-4-hydroxy-phenyl)-3-(1,2,2-trimethyl-propyl)-urea
1-(3,5-Dichloro-4-hydroxy-phenyl)-3-[2-(4-fluoro-phenyl)-1,1-dimethyl-ethyl]-urea
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-[3-Chloro-5-(3-cyclohexyl-ureido)-2-hydroxy-phenyl]-3-(2-trifluoromethyl-phenyl)-urea
1-{3-Chloro-5-[2-(4-chloro-phenylsulfanyl)-benzylamino]-4-hydroxy-phenyl}-3-cyclohexyl-urea
1- {3-[(Biphenyl-2-ylmethyl)-amino]-5-chloro-4-hydroxy-phenyl}-3-cyclohexyl-urea
1-[3-Chloro-5-(2-chloro-6-fluoro-benzylamino)-4-hydroxy-phenyl]-3-cyclohexyl-urea
N-[3-Chloro-5-(3-cyclohexyl-ureido)-2-hydroxy-phenyl]-2-nitro-benzenesulfonamide
1-(3-Chloro-5-formyl-4-hydroxy-phenyl)-3-cyclohexyl-urea
1-tert-Butyl-3-[3-chloro-2-hydroxy-5-(3-phenethyl-ureido)-phenyl]-urea
1-(3-Benzothiazol-2-yl-5-chloro-4-hydroxy-phenyl)-3-phenethyl-urea
1-[3-Chloro-2-hydroxy-5-(3-phenethyl-ureido)-phenyl]-3-phenyl-urea
[3-Chloro-2-hydroxy-5-(3-phenethyl-ureido)-phenyl]-carbamic acid isobutyl ester
[3-Chloro-2-hydroxy-5-(3-phenethyl-ureido)-phenyl]-carbamic acid sec-butyl ester
Cyclopropanecarboxylic acid [3-chloro-2-hydroxy-5-(3-phenethyl-ureido)-phenyl]-amide
Cyclobutanecarboxylic acid [3-chloro-2-hydroxy-5-(3-phenethyl-ureido)-phenyl]-amide
Cyclopentanecarboxylic acid [3-chloro-2-hydroxy-5-(3-phenethyl-ureido)-phenyl]-amide
Cyclohexanecarboxylic acid [3-chloro-2-hydroxy-5-(3-phenethyl-ureido)-phenyl]-amide
1-tert-Butyl-3-{3-chloro-2-hydroxy-5-[3-(1,1,3,3-tetramethyl-butyl)-ureido]-phenyl}-urea
1-(3-Benzothiazol-2-yl-5-chloro-4-hydroxy-phenyl)-3-(1,1,3,3-tetramethyl-butyl)-urea
1-{3-Chloro-2-hydroxy-5-[3-(1,1,3,3-tetramethyl-butyl)-ureido]-phenyl} -3-phenyl-urea
{3-Chloro-2-hydroxy-5-[3-(1,1,3,3-tetramethyl-butyl)-ureido]-phenyl}-carbamic acid isobutyl ester
{3-Chloro-2-hydroxy-5-[3-(1,1,3,3-tetramethyl-butyl)-ureido]-phenyl}-carbamic acid sec-butyl ester
Cyclopropanecarboxylic acid {3-chloro-2-hydroxy-5-[3-(1,1,3,3-tetramethyl-butyl)-ureido]-phenyl}-amide
Cyclobutanecarboxylic acid {3-chloro-2-hydroxy-5-[3-(1,1,3,3-tetramethyl-butyl)-ureido]-phenyl}-amide
Cyclopentanecarboxylic acid {3-chloro-2-hydroxy-5-[3-(1,1,3,3-tetramethyl-butyl)-ureido]-phenyl}-amide
Cyclohexanecarboxylic acid {3-chloro-2-hydroxy-5-[3-(1,1,3,3-tetramethyl-butyl)-ureido]-phenyl}-amide
N-[3-(3-tert-Butyl-ureido)-5-chloro-4-hydroxy-phenyl]-3-phenyl-propionamide
N-(3-Benzothiazol-2-yl-5-chloro-4-hydroxy-phenyl)-3-phenyl-propionamide
N-[3-Chloro-4-hydroxy-5-(3-phenyl-ureido)-phenyl]-3-phenyl-propionamide
[3-Chloro-2-hydroxy-5-(3-phenyl-propionylamino)-phenyl]-carbamic acid isobutyl ester
[3-Chloro-2-hydroxy-5-(3-phenyl-propionylamino)-phenyl]-carbamic acid sec-butyl ester
Cyclopropanecarboxylic acid [3-chloro-2-hydroxy-5-(3-phenyl-propionylamino)-phenyl]-amide
Cyclobutanecarboxylic acid [3-chloro-2-hydroxy-5-(3-phenyl-propionylamino)-phenyl]-amide
Cyclopentanecarboxylic acid [3-chloro-2-hydroxy-5-(3-phenyl-propionylamino)-phenyl]-amide
Cyclohexanecarboxylic acid [3-chloro-2-hydroxy-5-(3-phenyl-propionylamino)-phenyl]-amide
1-(3,5-Dichloro-2-hydroxy-4-methyl-phenyl)-3-phenyl-thiourea
1-(3,5-Dichloro-4-hydroxy-phenyl)-3-phenyl-thiourea
1-tert-Butyl-3-(3,5-dichloro-2-hydroxy-4-methyl-phenyl)-thiourea
1-Cyclohexyl-3-(3,5-dichloro-2-hydroxy-4-methyl-phenyl)-thiourea
1-Cyclopentyl-3-(3,5-dichloro-2-hydroxy-4-methyl-phenyl)-thiourea

1-Cyclohexyl-3-(3,5-dichloro-4-hydroxy-phenyl)-thiourea
1-Cyclopentyl-3-(3,5-dichloro-4-hydroxy-phenyl)-thiourea
1-Benzyl-3-(3,5-dichloro-2-hydroxy-4-methyl-phenyl)-thiourea
1-Benzyl-3-(3,5-dichloro-4-hydroxy-phenyl)-thiourea
1-(3,5-Dichloro-2-hydroxy-4-methyl-phenyl)-3-(2-trifluoromethyl-phenyl)-thiourea
1-(3,5-Dichloro-2-hydroxy-phenyl)-3-(2-trifluoromethyl-phenyl)-thiourea
1-(3,5-Dichloro-4-hydroxy-phenyl)-3-(2-trifluoromethyl-phenyl)-thiourea
1-(3,5-Dichloro-2-hydroxy-4-methyl-phenyl)-3-(4-trifluoromethyl-phenyl)-thiourea
1-(3,5-Dichloro-4-hydroxy-phenyl)-3-(4-trifluoromethyl-phenyl)-thiourea
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-[3-Chloro-5-(3-cyclohexyl-ureido)-2-hydroxy-phenyl]-3-(2-trifluoromethyl-phenyl)-urea
1- {3-Chloro-5-[2-(4-chloro-phenylsulfanyl)-benzylamino]-4-hydroxy-phenyl}-3-cyclohexyl-urea
1- {3-[(Biphenyl-2-ylmethyl)-amino]-5-chloro-4-hydroxy-phenyl}-3-cyclohexyl-urea
1-[3-Chloro-5-(2-chloro-6-fluoro-benzylamino)-4-hydroxy-phenyl]-3-cyclohexyl-urea
N-[3-Chloro-5-(3-cyclohexyl-ureido)-2-hydroxy-phenyl]-2-nitro-benzenesulfonamide
N-[3-Chloro-5-(3-cyclohexyl-ureido)-2-hydroxy-phenyl]-2,4,6-trimethyl-benzenesulfonamide
N-[3-Chloro-5-(3-cyclohexyl-ureido)-2-hydroxy-phenyl]-2-trifluoromethylbenzenesulfonamide
Ethanesulfonic acid [3-chloro-5-(3-cyclohexyl-ureido)-2-hydroxy-phenyl]-amide
N-[3-Chloro-5-(3-cyclohexyl-ureido)-2-hydroxy-phenyl]-3,3-dimethyl-butyramide
1-(3-Benzothiazol-2-yl-5-chloro-4-hydroxy-phenyl)-3-cyclohexyl-urea
1-(3-Benzothiazol-2-yl-5-chloro-4-hydroxy-phenyl)-3-(1,1,3,3-tetramethyl-butyl)-urea
1-(3-Benzothiazol-2-yl-5-chloro-4-hydroxy-phenyl)-3-phenethyl-urea
1- {3-Chloro-4-hydroxy-5-[3-(2-trifluoromethyl-phenyl)-thioureido]-phenyl} -3-cyclohexyl-urea
1-(5-Benzothiazol-2-yl-3-chloro-2-hydroxy-phenyl)-3-tert-butyl-urea
1-(5-Benzothiazol-2-yl-3-chloro-2-hydroxy-phenyl)-3-cyclopentyl-urea
1-(5-Benzothiazol-2-yl-3-chloro-2-hydroxy-phenyl)-3-phenyl-urea
1-(5-Benzothiazol-2-yl-3-chloro-2-hydroxy-phenyl)-3-benzyl-urea
1-(5-Benzothiazol-2-yl-3-chloro-2-hydroxy-phenyl)-3-phenethyl-urea
1-(5-Benzothiazol-2-yl-3-chloro-2-hydroxy-phenyl)-3-(2-trifluoromethyl-phenyl)-urea
1-(5-Benzothiazol-2-yl-3-chloro-2-hydroxy-phenyl)-3-(4-benzyloxy-phenyl)-urea
1-(5-Benzothiazol-2-yl-3-chloro-2-hydroxy-phenyl)-3-(2-trifluoromethyl-phenyl)-thiourea
1-(5-Benzothiazol-2-yl-3-chloro-2-hydroxy-phenyl)-3-isopropyl-thiourea
1-(5-Benzothiazol-2-yl-3-chloro-2-hydroxy-phenyl)-3-tert-butyl-thiourea
1-(5-Benzothiazol-2-yl-3-chloro-2-hydroxy-phenyl)-3-cyclopentyl-thiourea
3,5,5-Trimethyl-hexanoic acid (5-benzothiazol-2-yl-3-chloro-2-hydroxy-phenyl)-amide
N-(5-Benzothiazol-2-yl-3-chloro-2-hydroxy-phenyl)-3-phenyl-propionamide
3,5,5-Trimethyl-hexanoic acid (3-benzothiazol-2-yl-5-chloro-4-hydroxy-phenyl)-amid
N-(3-Benzothiazol-2-yl-5-chloro-4-hydroxy-phenyl)-3-phenyl-propionamide
N-(3,5-Dichloro-4-hydroxy-phenyl)-2-(2-nitro-phenyl)-acetamide
1-Cyclopentyl-3-(3,5-dichloro-2-hydroxy-4-methyl-phenyl)-urea
2-Benzo[1,3]dioxol-5-yl-N-(3,5-dichloro-4-hydroxy-phenyl)-acetamide

or a pharmaceutical acceptable salt or prodrug thereof.

**8.** Use of a compound for the manufacture of a medicament for the treatment of a disease whereby the disease involves an abnormal cell proliferation, an undesired cell proliferation, an abnormal mitosis and/or an undesired mitosis, whereby the compound is selected from the group comprising

3-[3-(5-Chloro-2-hydroxy-phenyl)-ureido]-propionic acid ethyl ester
1-(5-Chloro-2-hydroxy-phenyl)-3-pentyl-urea
1-(5-Chloro-2-hydroxy-phenyl)-3-(3,5-dichloro-phenyl)-urea
1-(5-Chloro-2-hydroxy-phenyl)-3-(4-chloro-phenyl)-urea
1-(5-Chloro-2-hydroxy-phenyl)-3-cyclohexyl-urea
1-(5-Chloro-2-hydroxy-phenyl)-3-(4-trifluoromethoxy-phenyl)-urea
1-(5-Chloro-2-hydroxy-phenyl)-3-(4-cyano-phenyl)-urea
1-Benzo[1,3]dioxol-5-yl-3-(5-chloro-2-hydroxy-phenyl)-urea
1-Benzyl-3-(5-chloro-2-hydroxy-phenyl)-urea

1-(5-Chloro-2-hydroxy-phenyl)-3-o-tolyl-urea
1-(5-Chloro-2-hydroxy-phenyl)-3-(3-methoxy-phenyl)-urea
1-(5-Chloro-2-hydroxy-phenyl)-3-(2,6-dimethyl-phenyl)-urea
1-(5-Chloro-2-hydroxy-phenyl)-3-phenethyl-urea
1-(5-Chloro-2-hydroxy-phenyl)-3-(1,1,3,3-tetramethyl-butyl)-urea
1-(5-Chloro-2-hydroxy-phenyl)-3-(3,4,5-trimethoxy-phenyl)-urea
1-(5-Chloro-2-hydroxy-phenyl)-3-naphthalen-1-yl-urea
1-Adamantan-1-yl-3-(5-chloro-2-hydroxy-phenyl)-urea
1-(5-Chloro-2-hydroxy-phenyl)-3-(4-phenoxy-phenyl)-urea
1-(5-Chloro-2-hydroxy-phenyl)-3-phenyl-urea
3-[3-(3,5-Dichloro-2-hydroxy-4-methyl-phenyl)-ureido]-propionic acid ethyl ester
1-(3,5-Dichloro-2-hydroxy-4-methyl-phenyl)-3-pentyl-urea
1-(3,5-Dichloro-2-hydroxy-4-methyl-phenyl)-3-(3,5-dichloro-phenyl)-urea
1-(4-Chloro-phenyl)-3-(3,5-dichloro-2-hydroxy-4-methyl-phenyl)-urea
1-Cyclohexyl-3 -(3, 5-dichloro-2-hydroxy-4-methyl-phenyl)-urea
1-(3,5-Dichloro-2-hydroxy-4-methyl-phenyl)-3-(4-trifluoromethoxy-phenyl)-urea
1-(4-Cyano-phenyl)-3-(3,5-dichloro-2-hydroxy-4-methyl-phenyl)-urea
1-Benzo[1,3]dioxol-5-yl-3-(3,5-dichloro-2-hydroxy-4-methyl-phenyl)-urea
1-Benzyl-3-(3,5-dichloro-2-hydroxy-4-methyl-phenyl)-urea
1-(3,5-Dichloro-2-hydroxy-4-methyl-phenyl)-3-o-tolyl-urea
1-(3,5-Dichloro-2-hydroxy-4-methyl-phenyl)-3-(3-methoxy-phenyl)-urea
1-(3,5-Dichloro-2-hydroxy-4-methyl-phenyl)-3-(2,6-dimethyl-phenyl)-urea
1-(3,5-Dichloro-2-hydroxy-4-methyl-phenyl)-3-phenethyl-urea
1-(3,5-Dichloro-2-hydroxy-4-methyl-phenyl)-3-(1,1,3,3-tetramethyl-butyl)-urea
1-(3,5-Dichloro-2-hydroxy-4-methyl-phenyl)-3-(3,4,5-trimethoxy-phenyl)-urea
1-(3,5-Dichloro-2-hydroxy-4-methyl-phenyl)-3-naphthalen-1-yl-urea
1-Adamantan-1-yl-3-(3,5-dichloro-2-hydroxy-4-methyl-phenyl)-urea
1-(3,5-Dichloro-2-hydroxy-4-methyl-phenyl)-3-(4-phenoxy-phenyl)-urea
1-(3,5-Dichloro-2-hydroxy-4-methyl-phenyl)-3-phenyl-urea
3-[3-(2-Hydroxy-4-methyl-phenyl)-ureido]-propionic acid ethyl ester
1-(2-Hydroxy-4-methyl-phenyl)-3-pentyl-urea
1-(3,5-Dichloro-phenyl)-3-(2-hydroxy-4-methyl-phenyl)-urea
1-(4-Chloro-phenyl)-3-(2-hydroxy-4-methyl-phenyl)-urea
1-Cyclohexyl-3-(2-hydroxy-4-methyl-phenyl)-urea
1-(2-Hydroxy-4-methyl-phenyl)-3-(4-trifluoromethoxy-phenyl)-urea
1-(4-Cyano-phenyl)-3-(2-hydroxy-4-methyl-phenyl)-urea
1-Benzo[1,3]dioxol-5-yl-3-(2-hydroxy-4-methyl-phenyl)-urea
1-Benzyl-3-(2-hydroxy-4-methyl-phenyl)-urea
1-(2-Hydroxy-4-methyl-phenyl)-3-o-tolyl-urea
1-(2-Hydroxy-4-methyl-phenyl)-3-(3-methoxy-phenyl)-urea
1-(2,6-Dimethyl-phenyl)-3-(2-hydroxy-4-methyl-phenyl)-urea
1-(2-Hydroxy-4-methyl-phenyl)-3-phenethyl-urea
1-(2-Hydroxy-4-methyl-phenyl)-3-(1,1,3,3-tetramethyl-butyl)-urea
1-(2-Hydroxy-4-methyl-phenyl)-3-(3,4,5-trimethoxy-phenyl)-urea
1-(2-Hydroxy-4-methyl-phenyl)-3-naphthalen-1-yl-urea
1-Adamantan-1-yl-3-(2-hydroxy-4-methyl-phenyl)-urea
1-(2-Hydroxy-4-methyl-phenyl)-3-(4-phenoxy-phenyl)-urea
1-(2-Hydroxy-4-methyl-phenyl)-3-phenyl-urea
N-(2-Hydroxy-phenyl)-C-phenyl-methanesulfonamide
N-(4-Hydroxy-phenyl)-C-phenyl-methanesulfonamide
N-(5-Chloro-2-hydroxy-phenyl)-C-phenyl-methanesulfonamide
N-(2-Hydroxy-4-methyl-phenyl)-C-phenyl-methanesulfonamide
N-(3,5-Dichloro-2-hydroxy-4-methyl-phenyl)-C-phenyl-methanesulfonamide
Butane-1-sulfonic acid (3,5-dichloro-2-hydroxy-4-methyl-phenyl)-amide
Octane-1-sulfonic acid (3,5-dichloro-2-hydroxy-4-methyl-phenyl)-amide
Propane-2-sulfonic acid (3,5-dichloro-2-hydroxy-4-methyl-phenyl)-amide
1-(3,5-Dichloro-2-hydroxy-phenyl)-3-(1,1,3,3-tetramethyl-butyl)-urea
1-(3-Chloro-2-hydroxy-phenyl)-3-(1,1,3,3-tetramethyl-butyl)-urea

1-Adamantan-1-yl-3-(3,5-dichloro-2-hydroxy-phenyl)-urea
1-Adamantan-1-yl-3-(3-chloro-2-hydroxy-phenyl)-urea
6-Benzylamino-2,4-dichloro-3-methyl-phenol
2,4-Dichloro-6-(2-chloro-6-fluoro-benzylamino)-3-methyl-phenol
2,4-Dichloro-3-methyl-6-(3-methyl-benzylamino)-phenol
2,4-Dichloro-6-(3,4-dimethoxy-benzylamino)-3-methyl-phenol
N-(3-Chloro-2-hydroxy-phenyl)-2-trifluoromethyl-benzenesulfonamide
4-Acetyl-N-(3-chloro-2-hydroxy-phenyl)-benzenesulfonamide
2,4-Dichloro-6-[2-(4-chloro-phenylsulfanyl)-benzylamino]-3-methyl-phenol
6-[(Biphenyl-2-ylmethyl)-amino]-2,4-dichloro-3-methyl-phenol
4-[(3,5-Dichloro-2-hydroxy-4-methyl-phenylamino)-methyl]-benzonitrile
4-[(3,5-Dichloro-2-hydroxy-4-methyl-phenylamino)-methyl]-benzoic acid methyl ester
6-[(5-Bromo-thiophen-2-ylmethyl)-amino]-2,4-dichloro-3-methyl-phenol
2,4-Dichloro-3-methyl-6-[(3-methyl-thiophen-2-ylmethyl)-amino]-phenol
2,4-Dichloro-3-methyl-6-[(5-methyl-furan-2-ylmethyl)-amino]-phenol
1-Adamantan-1-yl-3-(2,6-dibromo-3-chloro-4-methyl-phenyl)-urea
1-(2,6-Dibromo-3-chloro-4-methyl-phenyl)-3-(1,1,3,3-tetramethyl-butyl)-urea
1-Adamantan-1-yl-3-(3-chloro-2-cyano-phenyl)-urea
1-(3-Chloro-2-cyano-phenyl)-3-(1,1,3,3-tetramethyl-butyl)-urea
1-Adamantan-1-yl-3-(3-chloro-4-hydroxy-phenyl)-urea
1-(3-Chloro-4-hydroxy-phenyl)-3-(1,1,3,3-tetramethyl-butyl)-urea
1-Adamantan-1-yl-3-(3,5-dichloro-4-hydroxy-phenyl)-urea
1-(3,5-Dichloro-4-hydroxy-phenyl)-3-(1,1,3,3-tetramethyl-butyl)-urea
1-Piperidin-1-yl-2-(2,4,6-tribromo-3-hydroxy-phenyl)-ethanone
2-(3-Chloro-4-hydroxy-phenyl)-1-piperidin-1-yl-ethanone
1-Pyrrolidin-1-yl-2-(2,4,6-tribromo-3-hydroxy-phenyl)-ethanone
2-(3-Chloro-4-hydroxy-phenyl)-1-pyrrolidin-1-yl-ethanone
N-(3,5-Dichloro-2-hydroxy-4-methyl-phenyl)-N-methyl-2-trifluoromethyl-benzamide
3,5,5-Trimethyl-hexanoic acid (3,5-dichloro-2-hydroxy-4-methyl-phenyl)-amide
2-Chloro-6-[3-(1,1,3,3-tetramethyl-butyl)-ureido]-benzoic acid
3-Adamantan-1-yl-1-(3,5-dichloro-2-hydroxy-4-methyl-phenyl)-1-methyl-urea
1-(3,5-Dichloro-2-hydroxy-4-methyl-phenyl)-1-methyl-3-(1,1,3,3-tetramethyl-butyl)-urea
1-Adamantan-1-yl-3-(5-bromo-3-fluoro-2-hydroxy-phenyl)-urea
1-(5-Bromo-3-fluoro-2-hydroxy-phenyl)-3-(1,1,3,3-tetramethyl-butyl)-urea
1-(5-Bromo-3-fluoro-2-hydroxy-phenyl)-3-tert-butyl-urea
1-Adamantan-1-yl-3-(3,4-difluoro-2-hydroxy-phenyl)-urea
1-(3,4-Difluoro-2-hydroxy-phenyl)-3-(1,1,3,3-tetramethyl-butyl)-urea
1-tert-Butyl-3-(3,4-diftuoro-2-hydroxy-phenyl)-urea
1-Adamantan-1-yl-3-(3-fluoro-4-hydroxy-phenyl)-urea
1-(3-Fluoro-4-hydroxy-phenyl)-3-(1,1,3,3-tetramethyl-butyl)-urea
1-tert-Butyl-3-(3-fluoro-4-hydroxy-phenyl)-urea
1-tert-Butyl-3-(3,5-dichloro-4-hydroxy-phenyl)-urea
1-(3,5-Dichloro-4-hydroxy-phenyl)-3-(2-phenyl-cyclopropyl)-urea
1-Cyclohexyl-3-(3,5-dichloro-4-hydroxy-phenyl)-urea
1-Cyclopentyl-3-(3,5-dichloro-4-hydroxy-phenyl)-urea
1-(3,5-Dichloro-4-hydroxy-phenyl)-3-pentyl-urea
1-(2-Chloro-6-trifluoromethyl-phenyl)-3-(3,5-dichloro-4-hydroxy-phenyl)-urea
1-(3,5-Dichloro-4-hydroxy-phenyl)-3-(2,6-dimethyl-phenyl)-urea
1-(3,5-Dichloro-4-hydroxy-phenyl)-3-phenethyl-urea
1 -(3,5 -Dichloro-4-hydroxy-phenyl)-3 -naphthalen-2-yl-urea
1-Biphenyl-2-yl-3-(3,5-dichloro-4-hydroxy-phenyl)-urea
1-tert-Butyl-3-(3,5-dichloro-2-hydroxy-4-methyl-phenyl)-urea
1-tert-Butyl-3-(3,5-dichloro-2-hydroxy-phenyl)-urea
1-tert-Butyl-3-(3-chloro-4-hydroxy-phenyl)-urea
1-Adamantan-1-yl-3-(3,5-dibromo-4-hydroxy-phenyl)-urea
1-(3,5-Dibromo-4-hydroxy-phenyl)-3-(1,1,3,3-tetramethyl-butyl)-urea
1-tert-Butyl-3-(3,5-dibromo-4-hydroxy-phenyl)-urea
1-(3,5-Dibromo-4-hydroxy-phenyl)-3-(2-phenyl-cyclopropyl)-urea

1-(3,5-Dibromo-4-hydroxy-phenyl)-3-pentyl-urea
1-Cyclohexyl-3-(3,5-dibromo-4-hydroxy-phenyl)-urea
1-Adamantan-1-yl-3-(3-hydroxy-4-methoxy-phenyl)-urea
1-(3-Hydroxy-4-methoxy-phenyl)-3-(1,1,3,3-tetramethyl-butyl)-urea
1-tert-Butyl-3-(3-hydroxy-4-methoxy-phenyl)-urea
1-(3-Hydroxy-4-methoxy-phenyl)-3-(2-phenyl-cyclopropyl)-urea
1-(3-Hydroxy-4-methoxy-phenyl)-3-pentyl-urea
1-Cyclohexyl-3-(3-hydroxy-4-methoxy-phenyl)-urea
1-Adamantan-1-yl-3-(2,4-dihydroxy-phenyl)-urea
1-(2,4-Dihydroxy-phenyl)-3-(1,1,3,3-tetramethyl-butyl)-urea
1-tert-Butyl-3-(2,4-dihydroxy-phenyl)-urea
1-(2,4-Dihydroxy-phenyl)-3-(2-phenyl-cyclopropyl)-urea
1-(2,4-Dihydroxy-phenyl)-3-pentyl-urea
1-Cyclohexyl-3-(2,4-dihydroxy-phenyl)-urea
1-Adamantan-1-yl-3-(2'-hydroxy-[1,1';3',1"]terphenyl-5'-yl)-urea
1-(2'-Hydroxy-[1,1';3',1"]terphenyl-5'-yl)-3-(1,1,3,3-tetramethyl-butyl)-urea
1-tert-Butyl-3-(2'-hydroxy-[1,1';3',1"]terphenyl-5'-yl)-urea
1-(2'-Hydroxy-[1,1';3',1"]terphenyl-5'-yl)-3-(2-phenyl-cyclopropyl)-urea
1-(2'-Hydroxy-[1,1;3',1"]terphenyl-5'-yl)-3-pentyl-urea
1-Cyclohexyl-3-(2'-hydroxy-[1,1';3',1"]terphenyl-5'-yl)-urea
1-Adamantan-1-yl-3-(3-chloro-4-methoxy-phenyl)-urea
1-(3-Chloro-4-methoxy-phenyl)-3-(1,1,3,3-tetramethyl-butyl)-urea
1-tert-Butyl-3-(3-chloro-4-methoxy-phenyl)-urea
1-(3-Chloro-4-methoxy-phenyl)-3-(2-phenyl-cyclopropyl)-urea
1-(3-Chloro-4-methoxy-phenyl)-3-pentyl-urea
1-(3-Chloro-4-methoxy-phenyl)-3-cyclohexyl-urea
1-Adamantan-1-yl-3-(4-hydroxy-3-methoxy-benzyl)-urea
1-(4-Hydroxy-3-methoxy-benzyl)-3-(1,1,3,3-tetramethyl-butyl)-urea
1-tert-Butyl-3-(4-hydroxy-3-methoxy-benzyl)-urea
1-(4-Hydroxy-3-methoxy-benzyl)-3-(2-phenyl-cyclopropyl)-urea
1-(4-Hydroxy-3-methoxy-benzyl)-3-pentyl-urea
1-Cyclohexyl-3-(4-hydroxy-3-methoxy-benzyl)-urea
1-Adamantan-1-yl-3-(4-hydroxy-naphthalen-1-yl)-urea
1-(4-Hydroxy-naphthalen-1-yl)-3-(1,1,3,3-tetramethyl-butyl)-urea
1-tert-Butyl-3-(4-hydroxy-naphthalen-1-yl)-urea
1-(4-Hydroxy-naphthalen-1-yl)-3-(2-phenyl-cyclopropyl)-urea
1-(4-Hydroxy-naphthalen-1-yl)-3-p entyl-urea
1-Cyclohexyl-3-(4-hydroxy-naphthalen-1-yl)-urea
1-Adamantan-1-yl-3-[4-(2-hydroxy-ethyl)-phenyl]-urea
1-[4-(2-Hydroxy-ethyl)-phenyl]-3-(1,1,3,3-tetramethyl-butyl)-urea
1-tert-Butyl-3-[4-(2-hydroxy-ethyl)-phenyl]-urea
1-[4-(2-Hydroxy-ethyl)-phenyl]-3-(2-phenyl-cyclopropyl)-urea
1-[4-(2-Hydroxy-ethyl)-phenyl]-3-pentyl-urea
1-Cyclohexyl-3-[4-(2-hydroxy-ethyl)-phenyl]-urea
N-(3,5-Dichloro-4-hydroxy-phenyl)-2-phenyl-acetamide
3,5,5-Trimethyl-hexanoic acid (3,5-dichloro-4-hydroxy-phenyl)-amide
Octane-1-sulfonic acid (3,5-dichloro-4-hydroxy-phenyl)-amide
(3,5-Dichloro-2-hydroxy-4-methyl-phenyl)-carbamic acid 2-isopropyl-5-methyl-cyclohexyl ester
(3,5-Dichloro-2-hydroxy-4-methyl-phenyl)-carbamic acid hexyl ester
(3,5-Dichloro-4-hydroxy-phenyl)-carbamic acid phenyl ester
3-[3-(5-Chloro-2-hydroxy-phenyl)-ureido]-propionic acid ethyl ester
4-Bromo-3,5-dihydroxy-N-(1,1,3,3-tetramethyl-butyl)-benzamide
N-Benzyl-4-chloro-3-hydroxy-benzamide
N-Benzyl-3,5-dihydroxy-benzamide
2,6-Dichloro-4-(2-methyl-thiazol-4-yl)-phenol
2,6-Dichloro-4-(2-phenyl-thiazol-4-yl)-phenol
2,6-Dichloro-4-piperidin-1-ylmethyl-phenol
2,6-Dichloro-4-[2-(4-chloro-phenylsulfanyl)-benzylamino]-phenol

3,5-Dichloro-4-hydroxy-N-( 1,1 ,3,3-tetramethyl-butyl)-benzamide
2-(3-Chloro-4-hydroxy-phenyl)-N-(1,1,3,3-tetramethyl-butyl)-acetamide
4-(2-Benzyl-thiazol-4-yl)-2,6-dichloro-phenol
N-Adamantan-1-yl-2-(3,5-dichloro-4-hydroxy-phenyl)-acetamide
2-(3,5-Dichloro-4-hydroxy-phenyl)-N-phenethyl-acetamide
N-Cyclohexyl-2-(3,5-dichloro-4-hydroxy-phenyl)-acetamide
2-(3,5-Dichloro-4-hydroxy-phenyl)-N-indan-1-yl-acetamide
2-(3,5-Dichloro-4-hydroxy-phenyl)-N-(2-hydroxy-2-phenyl-ethyl)-acetamide
2-(3,5-Dichloro-4-hydroxy-phenyl)-N-(1,5-dimethyl-hexyl)-acetamide
2-(3,5-Dichloro-4-hydroxy-phenyl)-N-(3-methyl-butyl)-acetamide
2-(3,5-Dichloro-4-hydroxy-phenyl)-N-(3,3-diphenyl-propyl)-acetamide
1-[2-(3,5-Dichloro-4-hydroxy-phenyl)-acetyl]-3-(3-dimethylamino-propyl)-1-ethyl-urea
2-(3-Fluoro-4-hydroxy-phenyl)-N-(1,1,3,3-tetramethyl-butyl)-acetamide
2-(3-Fluoro-4-hydroxy-phenyl)-1-piperidin-1-yl-ethanone
2,6-Dichloro-4-(2-phenethyl-thiazol-4-yl)-phenol
2,6-Dichloro-4-[2-(2,2-dimethyl-propyl)-thiazol-4-yl]-phenol
2-(3,5-Dichloro-4-hydroxy-phenyl)-1-piperidin-1-yl-ethanone
2-(3,5-Dichloro-4-hydroxy-phenyl)-N-[2-(4-fluoro-phenyl)-1,1-dimethyl-ethyl]-acetamide
2-Cyano-N-cyclohexyl-2-(3,5-dichloro-4-hydroxy-phenyl)-acetamide
2,6-Dichloro-4-[2-(2,4,4-trimethyl-pentyl)-thiazol-4-yl]-phenol
2,6-Dichloro-4-[2-(1-methyl-butyl)-thiazol-4-yl]-phenol
2,6-Dichloro-4-(2-cyclopentylmethyl-thiazol-4-yl)-phenol
2,6-Dichloro-4-[2-(2-cyclopentyl-ethyl)-thiazol-4-yl]-phenol
4-(2-tert-Butyl-thiazol-4-yl)-2,6-dichloro-phenol
2,6-Dichloro-4-(2-thiophen-2-ylmethyl-thiazol-4-yl)-phenol
2,6-Dichloro-4-(2-phenoxymethyl-thiazol-4-yl)-phenol
2-Cyano-2-(3,5-dichloro-4-hydroxy-phenyl)-N-phenethyl-acetamide
1-tert-Butyl-3-[3-chloro-2-hydroxy-5-(2-oxo-2-piperidin-1-yl-ethyl)-phenyl]-urea
2-[3-(3-tert-Butyl-ureido)-5-chloro-4-hydroxy-phenyl]-N-cyclohexyl-acetamide
2-[3-(3-tert-Butyl-ureido)-5-chloro-4-hydroxy-phenyl]-N-(1,1,3,3-tetramethyl-butyl)-acetamide
2-[3-(3-tert-Butyl-ureido)-5-chloro-4-hydroxy-phenyl]-N-phenethyl-acetamide
2-(3-Acetyl-2,2-dimethyl-cyclobutyl)-N-(3,5-dichloro-4-hydroxy-phenyl)-acetamide
2-Bicyclo[2.2.1]hept-2-yl-N-(3,5-dichloro-4-hydroxy-phenyl)-acetamide
2-Propyl-pentanoic acid (3,5-dichloro-4-hydroxy-phenyl)-amide
2-Cyano-N-(3,5-dichloro-4-hydroxy-phenyl)-acetamide
N-(3,5-Dichloro-4-hydroxy-phenyl)-2-(3-hydroxy-4,7,7-trimethyl-bicyclo[2.2.1]hept-2-yl)-acetamide
2,2-Dicyclohexyl-N-(3,5-dichloro-4-hydroxy-phenyl)-acetamide
4-[2,4-Bis-(1,1-dimethyl-propyl)-phenoxy]-N-(3,5-dichloro-4-hydroxy-phenyl)-butyramide
N-(3,5-Dichloro-4-hydroxy-phenyl)-3-phenyl-acrylamide
2-Methyl-pentanoic acid (3,5-dichloro-4-hydroxy-phenyl)-amide
N-(3,5-Dichloro-4-hydroxy-phenyl)-2-phenoxy-acetamide
N-(3,5-Dichloro-4-hydroxy-phenyl)-malonamic acid ethyl ester
N-(3,5-Dichloro-4-hydroxy-phenyl)-succinamic acid ethyl ester
N-(3,5-Dichloro-4-hydroxy-phenyl)-3-(3-trifluoromethyl-phenyl)-acrylamide
3-Cyclopentyl-N-(3,5-dichloro-4-hydroxy-phenyl)-propionamide
N-(3,5-Dichloro-4-hydroxy-phenyl)-2,2-diphenyl-acetamide
Acetic acid 1-(3,5-dichloro-4-hydroxy-phenylcarbamoyl)-ethyl ester
N-(3,5-Dichloro-4-hydroxy-phenyl)-2-(2-isopropyl-5-methyl-cyclohexyloxy)-acetamide
N-(3,5-Dichloro-4-hydroxy-phenyl)-3,3-dimethyl-butyramide
3,5,5-Trimethyl-hexanoic acid (3-chloro-4-hydroxy-phenyl)-amide
3,5,5-Trimethyl-hexanoic acid (5-bromo-3-fluoro-2-hydroxy-phenyl)-amide
3,5,5-Trimethyl-hexanoic acid (3,5-dibromo-4-hydroxy-phenyl)-amide
3,5,5-Trimethyl-hexanoic acid (2,4-dihydroxy-phenyl)-amide
3,5,5-Trimethyl-hexanoic acid 4-hydroxy-3-methoxy-benzylamide
Adamantane-1-carboxylic acid 4-hydroxy-3-methoxy-benzylamide
N-(4-Hydroxy-3-methoxy-benzyl)-2-trifluoromethyl-benzamide
N-(3,5-Dichloro-4-hydroxy-phenyl)-2-thiophen-2-yl-acetamide
N-(3,5-Dichloro-4-hydroxy-phenyl)-acetamide

2-Cyclopentyl-N-(3,5-dichloro-4-hydroxy-phenyl)-acetamide
3-Methyl-but-2-enoic acid (3,5-dichloro-4-hydroxy-phenyl)-amide
N-(3,5-Dichloro-4-hydroxy-phenyl)-2-phenyl-acetamide
N-(3,5-Dichloro-4-hydroxy-phenyl)-3-phenyl-propionamide
3-Bromo-2-hydroxy-5-(3,5,5-trimethyl-hexanoylamino)-benzoic acid
4-Methyl-pentanoic acid (3,5-dichloro-4-hydroxy-phenyl)-amide
3,7-Dimethyl-oct-6-enoic acid (3,5-dichloro-4-hydroxy-phenyl)-amide
2-Adamantan-1-yl-N-(3,5-dichloro-4-hydroxy-phenyl)-acetamide
3-Cyclohexyl-N-(3,5-dichloro-4-hydroxy-phenyl)-propionamide
N-(3,5-Dichloro-4-hydroxy-phenyl)-2-(2-fluoro-phenyl)-acetamide
N-(3,5-Dichloro-4-hydroxy-phenyl)-3-hydroxy-3-phenyl-propionamide
N-[(3,5-Dichloro-4-hydroxy-phenylcarbamoyl)-methyl]-benzamide
3-(3,5-Dichloro-4-hydroxy-phenyl)-1,1-diethyl-urea
3-(3,5-Dichloro-4-hydroxy-phenyl)-1,1-diisopropyl-urea
1-(3,5-Dichloro-4-hydroxy-phenyl)-3-(3-hydroxy-propyl)-urea
1-(3,5-Dichloro-4-hydroxy-phenyl)-3-(2-morpholin-4-yl-ethyl)-urea
1-(3,5-Dichloro-4-hydroxy-phenyl)-3-(3-isopropoxy-propyl)-urea
1-(1-Aza-bicyclo[2.2.2]oct-3-yl)-3-(3,5-dichloro-4-hydroxy-phenyl)-urea
1-(3,5-Dichloro-4-hydroxy-phenyl)-3-(2-piperidin-1-yl-ethyl)-urea
1-Adamantan-2-yl-3-(3,5-dichloro-4-hydroxy-phenyl)-urea
1-Adamantan-1-yl-3-(4-hydroxy-3-morpholin-4-ylmethyl-phenyl)-urea
1-(4-Hydroxy-3-morpholin-4-ylmethyl-phenyl)-3-(1,1,3,3-tetramethyl-butyl)-urea
1-(4-Hydroxy-3-morpholin-4-ylmethyl-phenyl)-3-(2-trifluoromethyl-phenyl)-urea
1-(3,5-Dichloro-4-hydroxy-phenyl)-3-(2-hydroxy-2-phenyl-ethyl)-urea
1-(3,5-Dichloro-4-hydroxy-phenyl)-3-[2-hydroxy-1-hydroxymethyl-2-(4-nitro-phenyl)-ethyl]-urea
1-(3,5-Dichloro-4-hydroxy-phenyl)-3-[2-(3,4-dimethoxy-phenyl)-ethyl]-urea
2-[3-(3,5-Dichloro-4-hydroxy-phenyl)-ureido]-3-phenyl-propionamide
1-(3,5-Dichloro-4-hydroxy-phenyl)-3-[2-(4-hydroxy-phenyl)-ethyl]-urea
1-(3,5-Dichloro-4-hydroxy-phenyl)-3-indan-2-yl-urea
1-(3,5-Dichloro-4-hydroxy-phenyl)-3-(3-phenyl-propyl)-urea
1-(3,5-Dichloro-4-hydroxy-phenyl)-3-indan-1-yl-urea
1-(3,5-Dichloro-4-hydroxy-phenyl)-3-(2-pyridin-2-yl-ethyl)-urea
1-(3,5-Dichloro-4-hydroxy-phenyl)-3-(2-pyridin-3-yl-ethyl)-urea
2-[3-(3,5-Dichloro-4-hydroxy-phenyl)-ureido]-4-methyl-pentanoic acid ethyl ester
2-[3-(3,5-Dichloro-4-hydroxy-phenyl)-ureido]-3-phenyl-propionic acid ethyl ester
2-[3-(3,5-Dichloro-2-hydroxy-4-methyl-phenyl)-ureido]-4-methyl-pentanoic acid ethyl ester
2-[3-(3,5-Dichloro-2-hydroxy-4-methyl-phenyl)-ureido]-3-phenyl-propionic acid ethyl ester
1-Adamantan-1-yl-3-(3,5-difluoro-4-hydroxy-phenyl)-urea
1-(3,5-Difluoro-4-hydroxy-phenyl)-3-(1,1,3,3-tetramethyl-butyl)-urea
1-(3,5-Difluoro-4-hydroxy-phenyl)-3-(2-trifluoromethyl-phenyl)-urea
1-tert-Butyl-3-(3,5-difluoro-4-hydroxy-phenyl)-urea
1-Adamantan-1-yl-3-(2-hydroxy-phenyl)-urea
1-(2-Hydroxy-phenyl)-3-(1,1,3,3-tetramethyl-butyl)-urea
1-(2-Hydroxy-phenyl)-3-(2-trifluoromethyl-phenyl)-urea
1-Adamantan-1-yl-3-(3-hydroxy-phenyl)-urea
1-(3-Hydroxy-phenyl)-3-(1,1,3,3-tetramethyl-butyl)-urea
1-(3-Hydroxy-phenyl)-3-(2-trifluoromethyl-phenyl)-urea
1-Adamantan-1-yl-3-(3-hydroxy-phenyl)-urea
1-(3-Hydroxy-phenyl)-3-(1,1,3,3-tetramethyl-butyl)-urea
1-(3-Hydroxy-phenyl)-3-(2-trifluoromethyl-phenyl)-urea
1,3-Bis-(3,5-dichloro-4-hydroxy-phenyl)-urea
1,3-Di-adamantan-1-yl-urea
1,3-Bis-(1,1,3,3-tetramethyl-butyl)-urea
3-Adamantan-1-yl-1-(3,5-dibromo-4-hydroxy-benzyl)-1-(3,5-dichloro-2-hydroxy-4-methyl-phenyl)-urea
1-(3,5-Dibromo-4-hydroxy-benzyl)-1-(3,5-dichloro-2-hydroxy-4-methyl-phenyl)-3-(1,1,3,3-tetramethyl-butyl)-urea
1-(3,5-Dibromo-4-hydroxy-benzyl)-1-(3,5-dichloro-2-hydroxy-4-methyl-phenyl)-3-(2-trifluoromethyl-phenyl)-urea

3-tert-Butyl-1-(3,5-dibromo-4-hydroxy-benzyl)-1-(3,5-dichloro-2-hydroxy-4-methyl-phenyl)-urea
1-(3,5-Dichloro-4-hydroxy-phenyl)-3-(1,2,2-trimethyl-propyl)-urea
1-(3,5-Dichloro-4-hydroxy-phenyl)-3-[2-(4-fluoro-phenyl)-1,1-dimethyl-ethyl]-urea
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-[3-Chloro-5-(3-cyclohexyl-ureido)-2-hydroxy-phenyl]-3-(2-trifluoromethyl-phenyl)-urea
1- {3-Chloro-5-[2-(4-chloro-phenylsulfanyl)-benzylamino]-4-hydroxy-phenyl}-3-cyclohexyl-urea
1-{3-[(Biphenyl-2-ylmethyl)-amino]-5-chloro-4-hydroxy-phenyl}-3-cyclohexyl-urea
1-[3-Chloro-5-(2-chloro-6-fluoro-benzylamino)-4-hydroxy-phenyl]-3-cyclohexyl-urea
N-[3-Chloro-5-(3-cyclohexyl-ureido)-2-hydroxy-phenyl]-2-nitro-benzenesulfonamide
1-(3-Chloro-5-formyl-4-hydroxy-phenyl)-3-cyclohexyl-urea
1-tert-Butyl-3-[3-chloro-2-hydroxy-5-(3-phenethyl-ureido)-phenyl]-urea
1-(3-Benzothiazol-2-yl-5-chloro-4-hydroxy-phenyl)-3-phenethyl-urea
1-[3-Chloro-2-hydroxy-5-(3-phenethyl-ureido)-phenyl]-3-phenyl-urea
[3-Chloro-2-hydroxy-5-(3-phenethyl-ureido)-phenyl]-carbamic acid isobutyl ester
[3-Chloro-2-hydroxy-5-(3-phenethyl-ureido)-phenyl]-carbamic acid sec-butyl ester
Cyclopropanecarboxylic acid [3-chloro-2-hydroxy-5-(3-phenethyl-ureido)-phenyl]-amide
Cyclobutanecarboxylic acid [3-chloro-2-hydroxy-5-(3-phenethyl-ureido)-phenyl]-amide
Cyclopentanecarboxylic acid [3-chloro-2-hydroxy-5-(3-phenethyl-ureido)-phenyl]-amide
Cyclohexanecarboxylic acid [3-chloro-2-hydroxy-5-(3-phenethyl-ureido)-phenyl]-amide
1-tert-Butyl-3-{3-chloro-2-hydroxy-5-[3-(1,1,3,3-tetramethyl-butyl)-ureido]-phenyl}-urea
1-(3-Benzothiazol-2-yl-5-chloro-4-hydroxy-phenyl)-3-(1,1,3,3-tetramethyl-butyl)-urea
1-{3-Chloro-2-hydroxy-5-[3-(1,1,3,3-tetramethyl-butyl)-ureidoJ-phenyl}-3-phenyl-urea
{3-Chloro-2-hydroxy-5-[3-(1,1,3,3-tetramethyl-butyl)-ureido]-phenyl}-carbamic acid isobutyl ester
{3-Chloro-2-hydroxy-5-[3-(1,1,3,3-tetramethyl-butyl)-ureido]-phenyl}-carbamic acid sec-butyl ester
Cyclopropanecarboxylic acid {3-chloro-2-hydroxy-5-[3-(1,1,3,3-tetramethyl-butyl)-ureido]-phenyl}-amide
Cyclobutanecarboxylic acid {3-chloro-2-hydroxy-5-[3-(1,1,3,3-tetramethyl-butyl)-ureido]-phenyl}-amide
Cyclopentanecarboxylic acid {3-chloro-2-hydroxy-5-[3-(1,1,3,3-tetramethyl-butyl)-ureido]-phenyl}-amide
Cyclohexanecarboxylic acid {3-chloro-2-hydroxy-5-[3-(1,1,3,3-tetramethyl-butyl)-ureido]-phenyl}-amide
N-[3-(3-tert-Butyl-ureido)-5-chloro-4-hydroxy-phenyl]-3-phenyl-propionamide
N-(3-Benzothiazol-2-yl-5-chloro-4-hydroxy-phenyl)-3-phenyl-propionamide
N-[3-Chloro-4-hydroxy-5-(3-phenyl-ureido)-phenyl]-3-phenyl-propionamide
[3-Chloro-2-hydroxy-5-(3-phenyl-propionylamino)-phenyl]-carbamic acid isobutyl ester
[3-Chloro-2-hydroxy-5-(3-phenyl-propionylamino)-phenyl]-carbamic acid sec-butyl ester
Cyclopropanecarboxylic acid [3-chloro-2-hydroxy-5-(3-phenyl-propionylamino)-phenyl]-amide
Cyclobutanecarboxylic acid [3-chloro-2-hydroxy-5-(3-phenyl-propionylamino)-phenyl]-amide
Cyclopentanecarboxylic acid [3-chloro-2-hydroxy-5-(3-phenyl-propionylamino)-phenyl]-amide
Cyclohexanecarboxylic acid [3-chloro-2-hydroxy-5-(3-phenyl-propionylamino)-phenyl]-amide
1-(3,5-Dichloro-2-hydroxy-4-methyl-phenyl)-3-phenyl-thiourea
1-(3,5-Dichloro-4-hydroxy-phenyl)-3-phenyl-thiourea
1-tert-Butyl-3-(3,5-dichloro-2-hydroxy-4-methyl-phenyl)-thiourea
1-Cyclohexyl-3-(3,5-dichloro-2-hydroxy-4-methyl-phenyl)-thiourea
1-Cyclopentyl-3-(3,5-dichloro-2-hydroxy-4-methyl-phenyl)-thiourea
1-Cyclohexyl-3-(3,5-dichloro-4-hydroxy-phenyl)-thiourea
1-Cyclopentyl-3-(3,5-dichloro-4-hydroxy-phenyl)-thiourea
1-Benzyl-3-(3,5-dichloro-2-hydroxy-4-methyl-phenyl)-thiourea
1-Benzyl-3-(3,5-dichloro-4-hydroxy-phenyl)-thiourea
1-(3,5-Dichloro-2-hydroxy-4-methyl-phenyl)-3-(2-trifluoromethyl-phenyl)-thiourea
1-(3,5-Dichloro-2-hydroxy-phenyl)-3-(2-trifluoromethyl-phenyl)-thiourea
1-(3,5-Dichloro-4-hydroxy-phenyl)-3-(2-trifluoromethyl-phenyl)-thiourea
1-(3,5-Dichloro-2-hydroxy-4-methyl-phenyl)-3-(4-trifluoromethyl-phenyl)-thiourea
1-(3,5-Dichloro-4-hydroxy-phenyl)-3-(4-trifluoromethyl-phenyl)-thiourea
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-[3-Chloro-5-(3-cyclohexyl-ureido)-2-hydroxy-phenyl]-3-(2-trifluoromethyl-phenyl)-urea
1- {3-Chloro-5-[2-(4-chloro-phenylsulfanyl)-benzylamino]-4-hydroxy-phenyl -3-cyclohexyl-urea
1- {3-[(Biphenyl-2-ylmethyl)-amino]-5-chloro-4-hydroxy-phenyl -3-cyclohexyl-urea
1-[3-Chloro-5-(2-chloro-6-fluoro-benzylamino)-4-hydroxy-phenyl]-3-cyclohexyl-urea

N-[3-Chloro-5-(3-cyclohexyl-ureido)-2-hydroxy-phenyl]-2-nitro-benzenesulfonamide
N-[3-Chloro-5-(3-cyclohexyl-ureido)-2-hydroxy-phenyl]-2,4,6-trimethyl-benzenesulfonamide
N-[3-Chloro-5-(3-cyclohexyl-ureido)-2-hydroxy-phenyl]-2-trifluoromethyl-benzenesulfonamide
Ethanesulfonic acid [3-chloro-5-(3-cyclohexyl-ureido)-2-hydroxy-phenyl]-amide
N-[3-Chloro-5-(3-cyclohexyl-ureido)-2-hydroxy-phenyl]-3,3-dimethyl-butyramide
1-(3-Benzothiazol-2-yl-5-chloro-4-hydroxy-phenyl)-3-cyclohexyl-urea
1-(3-Benzothiazol-2-yl-5-chloro-4-hydroxy-phenyl)-3-(1,1,3,3-tetramethyl-butyl)-urea
1-(3-Benzothiazol-2-yl-5-chloro-4-hydroxy-phenyl)-3-phenethyl-urea
1- {3-Chloro-4-hydroxy-5-[3-(2-triftuoromethyl-phenyl)-thioureido]-phenyl} -3-cyclohexyl-urea
1-(5-Benzothiazol-2-yl-3-chloro-2-hydroxy-phenyl)-3-tert-butyl-urea
1-(5-Benzothiazol-2-yl-3-chloro-2-hydroxy-phenyl)-3-cyclopentyl-urea
1-(5-Benzothiazol-2-yl-3-chloro-2-hydroxy-phenyl)-3-phenyl-urea
1-(5-Benzothiazol-2-yl-3-chloro-2-hydroxy-phenyl)-3-benzyl-urea
1-(5-Benzothiazol-2-yl-3-chloro-2-hydroxy-phenyl)-3-phenethyl-urea
1-(5-Benzothiazol-2-yl-3-chloro-2-hydroxy-phenyl)-3-(2-trifluoromethyl-phenyl)-urea
1-(5-Benzothiazol-2-yl-3-chloro-2-hydroxy-phenyl)-3-(4-benzyloxy-phenyl)-urea
1-(5-Benzothiazol-2-yl-3-chloro-2-hydroxy-phenyl)-3-(2-trifluoromethyl-phenyl)-thiourea
1-(5-Benzothiazol-2-yl-3-chloro-2-hydroxy-phenyl)-3-isopropyl-thiourea
1-(5-Benzothiazol-2-yl-3-chloro-2-hydroxy-phenyl)-3-tert-butyl-thiourea
1-(5-Benzothiazol-2-yl-3-chloro-2-hydroxy-phenyl)-3-cyclopentyl-thiourea
3,5,5-Trimethyl-hexanoic acid (5-benzothiazol-2-yl-3-chloro-2-hydroxy-phenyl)-amide
N-(5-Benzothiazol-2-yl-3-chloro-2-hydroxy-phenyl)-3-phenyl-propionamide
3,5,5-Trimethyl-hexanoic acid (3-benzothiazol-2-yl-5-chloro-4-hydroxy-phenyl)-amid
N-(3-Benzothiazol-2-yl-5-chloro-4-hydroxy-phenyl)-3-phenyl-propionamide
N-(3,5-Dichloro-4-hydroxy-phenyl)-2-(2-nitro-phenyl)-acetamide
1-Cyclopentyl-3-(3,5-dichloro-2-hydroxy-4-methyl-phenyl)-urea
2-Benzo[1,3]dioxol-5-yl-N-(3,5-dichloro-4-hydroxy-phenyl)-acetamide

or a pharmaceutical acceptable salt or prodrug thereof.

**9.** The use according to any of claims 1 and 3 to 7, wherein the rotamase regulates a part of the cell cycle, preferably the part of the cell cycle is mitosis.

**10.** The use according to any of claims 1 and 3 to 7 and 9, wherein the rotamase is a mammalian rotamase, preferably a human rotamase, more preferably hPin1.

**11.** The use according to any of claims 2 to 6 and 8, wherein the disease is selected from the group comprising neurodegenerative diseases, stroke, inflammatory diseases, immune based disorders, infectious diseases, heart diseases, cardiovascular diseases and cell proliferative diseases.

**12.** The use according to claim 11, wherein the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral atrophy, prion diseases and motor neuron diseases.

**13.** The use according to claim 11, wherein the infectious disease is selected from the group comprising fungal, viral, bacterial and parasite infection.

**14.** The use according to claim 13, wherein the fungal infection is selected from the group comprising gynaecological and dermatological infection.

**15.** The use according to claim 13, wherein the fungal infection is caused by Histoplasma, *Coccidioides, Cryptococcus, Blastomyces, Paracoccidioides, Aspergillus, Sporothrix, Rhizopus, Absidia, Mucor, Hormodendrum, Phialophora* Microsporum, Epidermophyton, *Rhinosporidum* or by *a* yeast, preferably *Candida or Cryptococcus.*

**16.** The use according to claim 13, wherein the fungal infection causes a disorder selected from the group comprising ringworm, candidiasis, coccidioidomycosis, blastomycosis, aspergillosis, cryptococcosis, histioplasmosis, paracoccidiomycosis, zygomycosis, sporotrichiosis, mycotic keratitis, nail hair and skin disease, lobomycosis, chromoblastomycosis, mycetoma.

**17.** The use according to claim 13, wherein the bacterial infection is selected from the group comprising infections caused by Gram-positive and by Gram-negative bacteria.

**18.** The use according to claim 17, wherein the bacterial infection is caused by *Staphylococcus, Clostridium, Streptococcus, Listeria, Salmonella, Bacillus, Escherichia, Mycobacteria, Serratia, Enterobacter, Enterococcus, Nocardia, Hemophilus, Neisseria, Proteus, Yersinia, Helicobacter or Legionella.*

**19.** The use according to claim 13, wherein the bacterial infection causes a disorder selected from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

**20.** The use according to claim 13, wherein the viral infection is selected from the group comprising infections caused by retrovirus (HIV), Papilloma virus, Polio virus, Epstein-Barr, Herpes virus, Hepatitis virus, Papova virus, Influenza virus, Rabies, JC, encephalitis causing virus or hemorrhagic fever causing virus.

**21.** The use according to claim 13, wherein the parasite infection is selected from the group comprising infections caused by *Trypanosoma, Leishmania, Trichinella, Echinococcus, Nematodes, Classes Cestoda Trematoda, Monogenea, Toxoplasma, Giardia, Balantidium, Paramecium, Plasmodium, or Entamoeba.*

**22.** The use according to claim 11, wherein the cell proliferative disorder is selected from the group comprising neoplastic and non-neoplastic disorders.

**23.** The use according to claim 22, wherein the neoplastic cell proliferative disorder is selected from the group comprising solid tumor, lymphoma and leukemia.

**24.** The use according to claim 23, wherein the solid tumor is selected from the group comprising carcinoma, sarcoma, osteoma, fibrosarcoma, and chondrosarcoma.

**25.** The use according to claim 22, wherein the neoplastic cell proliferative disorder is selected from the group comprising breast cancer, prostate cancer, colon cancer, brain cancer, lung cancer, pancreatic cancer, gastric cancer, bladder cancer and kidney cancer.

**26.** The use according to claim 22, wherein the non-neoplastic cell proliferative disorder is a fibrotic disorder, preferably the fibrotic disorder is fibrosis.

**27.** The use according to claim 22, wherein the non-neoplastic cell proliferative disorder is selected from the group comprising prostatic hypertrophy, endometriosis, psoriasis, tissue repair and wound healing.

**28.** The use according to claim 11, wherein the immune based/inflammatory disease is an autoimmune disease or disorder.

**29.** The use according to claim 11, wherein the immune based/inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease and dermatomyositis.

**30.** The use according to claim 11, wherein the immune based and/or inflammatory disease is an inflammatory condition.

**31.** The use according to claim 11, wherein the immune based and/or inflammatory disease is selected from the group comprising inflammation associated with bums, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

**32.** The use according to any of claims 2 to 6, 8 and 11 to 31 wherein the medicament is for administration via an administration route which is selected from the group comprising oral, subcutaneous, intravenous, intranasal, transdermal, intraperitoneal, intramuscular, intrapulmonar, vaginal, rectal, and intraocular administration.

**33.** The use according to any of claims 2 to 6, 8 and 11 to 32 wherein the medicament is for the administration to a mammal, preferably to a human being.

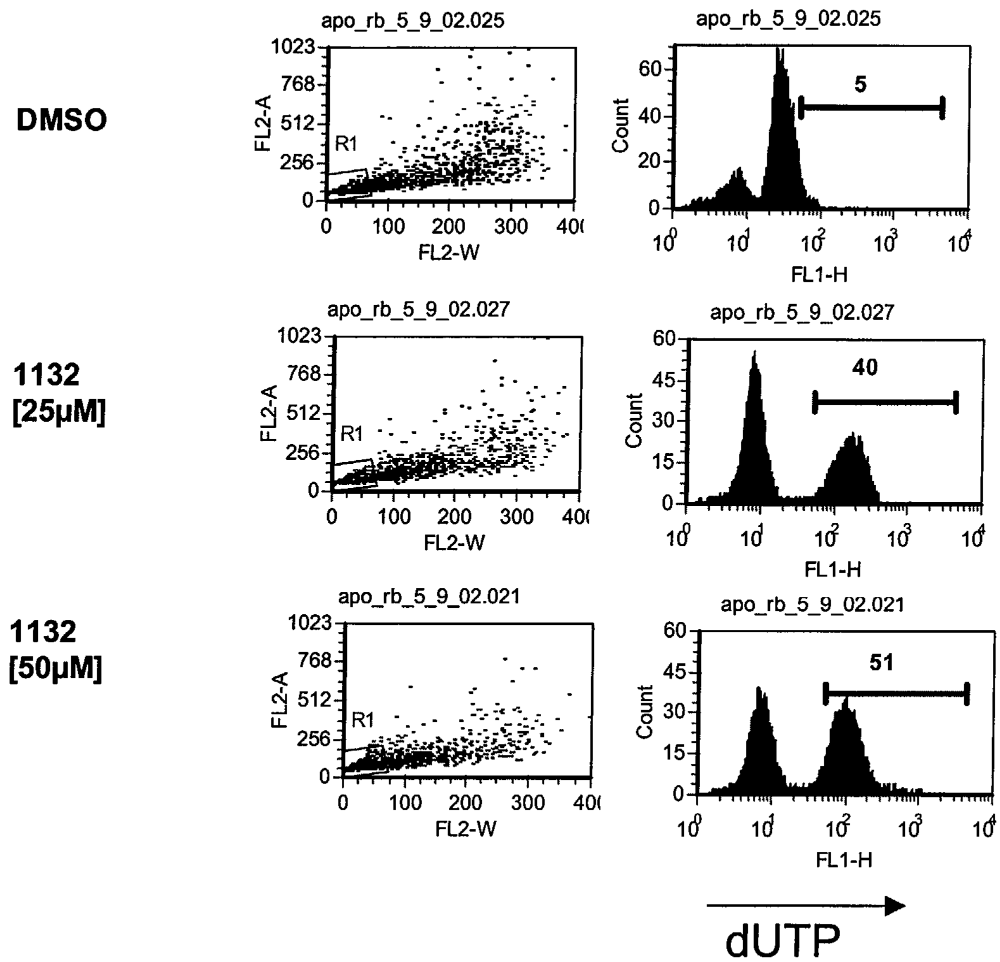
DMSO
1132
[25µM]
1132
[50µM]
apo_rb_5_9_02.025
apo_rb_5_9_02.027
apo_rb_5_9_02.021
FL2-A
FL2-W
R1
Count
FL1-H
5
40
51
dUTP

Fig. 1

European Patent Office

**PARTIAL EUROPEAN SEARCH REPORT**
which under Rule 45 of the European Patent Convention shall be considered, for the purposes of subsequent proceedings, as the European search report

Application Number EP 02 02 0987

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | WO 02 059 080 A (GUILFORD PHARMACEUTICALS INC.) 1 August 2002 (2002-08-01)<br>* page 104 - page 119; claims 1-63 *<br>--- | 1-33 | A61K31/17<br>A61K31/155<br>A61K31/18<br>A61K31/135<br>A61K31/165 |
| X | WO 02 44126 A (GUILFORD PHARMACEUTICALS INC.) 6 June 2002 (2002-06-06)<br>* page 104 - page 140; claims 1-84 *<br>--- | 1-33 | |
| X | WO 01 17953 A (GUILFORD PHARMACEUTICALS INC.) 15 March 2001 (2001-03-15)<br>* page 33 - page 51; claims 1-94 *<br>--- | 1-33 | |
| Y | WO 98 37882 A (GUILFORD PHARMACEUTICALS INC.) 3 September 1998 (1998-09-03)<br>* page 37 - page 49; claims 1-24 *<br>--- | 1-33 | |
| Y | US 6 242 468 B1 (JIA-HE LI ET AL.) 5 June 2001 (2001-06-05)<br>* column 1 - column 7, line 2 *<br>--- | 1-33 | TECHNICAL FIELDS SEARCHED (Int.Cl.7) |
| Y | US 2002 049 199 A1 ( GREGORY S. HAMILTON ET AL.) 25 April 2002 (2002-04-25)<br>* page 1, column 1 - page 3, column 1 *<br>---<br>-/-- | 1-33 | A61K |

**INCOMPLETE SEARCH**

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 26 February 2003 | Kyriakakou, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 02 02 0987

Claim(s) searched completely:
1-33

Reason for the limitation of the search:

Present claims 1-33 relate to the use of an extremely large number of possible compounds and compositions. In fact, the claims contain so many options and variables, that a lack of clarity and conciseness within the meaning of Article 84 EPC arises to such an extent as to render a meaningful search of the claims impossible. Consequently, the search has been carried out for those parts of the application which do appear to be clear (and concise), namely the use of urea and thiourea derivatives as rotamase inhibitors.

It should be noted that even with the above restriction a large number of novelty-destroying documents were found for the above claims.

European Patent Office

# PARTIAL EUROPEAN SEARCH REPORT

Application Number
EP 02 02 0987

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | US 5 744 485 A (ROBERT EDWARD ZELLE ET AL.) 28 April 1998 (1998-04-28)<br>* column 1 - column 5, line 52 *<br>----- | 1-33 | |
| | | | TECHNICAL FIELDS SEARCHED (Int.Cl.7) |

EPO FORM 1503 03.82 (P04C10)

European Patent
Office

Application Number
EP 02 02 0987

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-33 incompletely

European Patent Office

**LACK OF UNITY OF INVENTION SHEET B**

Application Number
EP 02 02 0987

The Search Division considers that the present European patent application does not comply with the requirements of unity of invention and relates to several inventions or groups of inventions, namely:

1. Claims: 1-33 incompletely

The application relates to the following 10 inventions:

Use of a compound as an inhibitor to a rotamase whereby the compound has the structure AXY and
1) X is -(CRR)-NR-CO-NR-(CRR)-, -(CRR)-NR-CS-NR-(CRR)

The first invention is searched incompletely.

2) X is -(CRR)-NR-C(N-CN)-NR-(CRR), -(CRR)-NR-C(NR)-NR-(CRR)-
3) -(CRR)-CO-NR-(CRR)-, -(CRR)-NR-CO-(CRR), (CRR)-NR-CS-(CRR), -(CRR)-CS-NR-(CRR)

4)-(CRR)-NR-(CRR)

5) -(CRR)-NRCOO-(CRR),-(CRR)-O-CONR-(CRR)

6)-(CRR)-O-(CRR), -(CRR)-SR-(CRR)-

7)- (CRR)-CO-(CRR)-, -(CRR)-CS-(CRR)

8)- (CRR)-NR-SO2-(CRR), -(CRR)-SO2-NR-(CRR)

9)-(CRR)-SO2-(CRR)-, -(CRR)-SO-(CRR)

10)-(CRR)-

It has to be stressed that depending on the nature of the A and Y groups and/ or the nature of the R substituents further inventions would be arise.

2. Claims: 1-33 incompletely

3. Claims: 1-33 incompletely

4. Claims: 1-33 incompletely

5. Claims: 1-33 incompletely

6. Claims: 1-33 incompletely

European Patent Office

## LACK OF UNITY OF INVENTION SHEET B

Application Number
EP 02 02 0987

The Search Division considers that the present European patent application does not comply with the requirements of unity of invention and relates to several inventions or groups of inventions, namely:

7. Claims: 1-33 incompletely

8. Claims: 1-33 incompletely

9. Claims: 1-33 incompletely

10. Claims: 1-33 incomletely

**ANNEX TO THE EUROPEAN SEARCH REPORT ON EUROPEAN PATENT APPLICATION NO.** EP 02 02 0987

This annex lists the patent family members relating to the patent documents cited in the above-mentioned European search report. The members are as contained in the European Patent Office EDP file on
The European Patent Office is in no way liable for these particulars which are merely given for the purpose of information.

26-02-2003

| Patent document cited in search report | Publication date | Patent family member(s) | Publication date |
|---|---|---|---|
| WO 02059080 A | 01-08-2002 | US 2002165275 A | 07-11-2002 |
| WO 0244126 A | 06-06-2002 | AU 2576702 A | 11-06-2002 |
| | | US 2002127605 A | 12-09-2002 |
| WO 0117953 A | 15-03-2001 | AU 7353300 A | 10-04-2001 |
| | | CA 2383086 A | 15-03-2001 |
| | | EP 1214293 A | 19-06-2002 |
| | | JP 2003508512 T | 04-03-2003 |
| WO 9837882 A | 03-09-1998 | AU 6181698 A | 18-09-1998 |
| | | EP 1001762 A | 24-05-2000 |
| | | JP 2001513772 T | 04-09-2001 |
| | | US 6242468 B | 05-06-2001 |
| | | ZA 9800825 A | 19-10-1998 |
| US 6242468 B | 05-06-2001 | AU 5687999 A | 14-03-2000 |
| | | BG 105340 A | 31-12-2001 |
| | | BR 9912988 A | 02-10-2001 |
| | | CA 2341530 A | 02-03-2000 |
| | | CN 1313767 T | 19-09-2001 |
| | | CZ 20010274 A | 12-09-2001 |
| | | EP 1107757 A | 20-06-2001 |
| | | HU 0103166 A | 29-05-2002 |
| | | JP 2002523367 T | 30-07-2002 |
| | | NO 20010946 A | 23-02-2001 |
| | | PL 346759 A | 25-02-2002 |
| | | SK 2562001 A | 11-09-2001 |
| | | WO 0010553 A | 02-03-2000 |
| | | AU 6181698 A | 18-09-1998 |
| | | EP 1001762 A | 24-05-2000 |
| | | JP 2001513772 T | 04-09-2001 |
| | | WO 9837882 A | 03-09-1998 |
| | | ZA 9800825 A | 19-10-1998 |
| US 2002049199 A | 25-04-2002 | US 6274607 B | 14-08-2001 |
| | | US 5958949 A | 28-09-1999 |
| | | US 5935989 A | 10-08-1999 |
| | | CA 2239781 A | 30-06-1998 |
| | | EA 2401 B | 25-04-2002 |
| | | EP 0959882 A | 01-12-1999 |
| | | JP 2002515050 T | 21-05-2002 |
| | | NO 992751 A | 04-08-1999 |
| | | NZ 336386 A | 28-09-2001 |
| | | SK 57899 A | 16-05-2000 |
| | | AU 721572 B | 06-07-2000 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 02 0987

This annex lists the patent family membersrelating to the patent documents cited in the above-mentioned European search report.
The members are as contained in the European Patent Office EDP file on
The European Patent Office is in no way liable for these particulars which are merely given for the purpose of information.

26-02-2003

| Patent document cited in search report | Publication date | Patent family member(s) | Publication date |
|---|---|---|---|
| US 2002049199 A | | AU 5724198 A | 31-07-1998 |
| | | BG 103408 A | 31-01-2000 |
| | | BR 9714092 A | 09-05-2000 |
| | | CZ 9901546 A | 17-11-1999 |
| | | HU 0002442 A | 28-12-2000 |
| | | KR 2000057488 A | 15-09-2000 |
| | | PL 334372 A | 28-02-2000 |
| | | WO 9829117 A | 09-07-1998 |
| | | US 6184243 B | 06-02-2001 |
| | | ZA 9711704 A | 23-09-1998 |
| US 5744485 A | 28-04-1998 | AT 210119 T | 15-12-2001 |
| | | AU 704475 B | 22-04-1999 |
| | | AU 2194395 A | 17-10-1995 |
| | | CA 2186380 A | 05-10-1995 |
| | | DE 69524400 D | 17-01-2002 |
| | | DE 69524400 T | 22-08-2002 |
| | | DK 754176 T | 02-04-2002 |
| | | EP 1138673 A | 04-10-2001 |
| | | EP 0754176 A | 22-01-1997 |
| | | ES 2169127 T | 01-07-2002 |
| | | JP 9510974 T | 04-11-1997 |
| | | NZ 283599 A | 26-06-1998 |
| | | PT 754176 T | 31-05-2002 |
| | | WO 9526337 A | 05-10-1995 |

EPO FORM P0459

For more details about this annex : see Official Journal of the European Patent Office, No. 12/82